# EUROPEAN PATENT APPLICATION

(11) **EP 2 365 087 A2**
(43) Date of publication of application: **14.09.2011**
(21) Application number: 10194223.3
(22) Date of filing: 20.05.2004
(51) Int. Cl.: C12N 15/82, C12N 15/29, A01H 5/00

(54) **Methods of increasing abiotic stress tolerance and/or biomass in plants and plants generated thereby**

(30) Priority: 22.05.2003 US 472433 P
(62) Divisional of application: 04734072.4
(71) Applicant: Evogene Ltd., 76121 Rechovot (IL)
(72) Inventor: Ronen, Gil, 33870 Emeq-Hefer (IL); Golan, Ezekiel, 69703 Tel Aviv (IL); Karchi, Hagai, 76834 Doar Na Emek Soreq (IL); Meissner, Rafael, 76350 Rechovot (IL)
(74) Representative: Vossius & Partner

(57) **Abstract**

Described is a method of increasing biomass and/or abiotic stress tolerance of a plant, comprising expressing within the plant an exogenous polynucleotide at least 80% homologous to the polynucleotide set forth in SEQ ID NO: 13, thereby increasing the biomass and/or the abiotic stress tolerance of the plant.

## Description

### FIELD AND BACKGROUND OF THE INVENTION

The present invention relates to methods of increasing abiotic stress tolerance and/or biomass in plants and, more particularly, to plants expressing exogenous abiotic stress-tolerance genes.

Abiotic stress (also referred to as "environmental stress") conditions such as salinity, drought, flood, suboptimal temperature and toxic chemical pollution, cause substantial damage to agricultural plants. Most plants have evolved strategies to protect themselves against these conditions. However, if the severity and duration of the stress conditions are too great, the effects on plant development, growth and yield of most crop plants are profound. Furthermore, most of the crop plants are very susceptible to abiotic stress (ABS) and thus necessitate optimal growth conditions for commercial crop yields. Continuous exposure to stress causes major alterations in the plant metabolism which ultimately lead to cell death and consequently yield losses. Thus, despite extensive research and the use of sophisticated and intensive crop-protection measures, losses due to abiotic stress conditions remain in the billions of dollars annually (1,2).

Developing stress-tolerant plants is a strategy that has the potential to solve or mediate at least some of these problems. However, traditional plant breeding strategies used to develop new lines of plants that exhibit tolerance to ABS are relatively inefficient since they are tedious, time consuming and of unpredictable outcome. Furthermore, limited germplasm resources for stress tolerance and incompatibility in crosses between distantly related plant species represent significant problems encountered in conventional breeding. Additionally, the cellular processes leading to ABS tolerance are complex in nature and involve multiple mechanisms of cellular adaptation and numerous metabolic pathways (4-7).

Genetic engineering efforts, aimed at conferring abiotic stress tolerance to transgenic crops, have been described in the prior art. Studies by Apse and Blumwald (Curr Opin Biotechnol. 13:146-150, 2002), Quesada et al. (Plant Physiol. 130:951-963, 2002), Holmström et al. (Nature 379: 683-684, 1996), Xu et al. (Plant Physiol 110: 249-257, 1996), Pilon-Smits and Ebskamp (Plant Physiol 107: 125-130, 1995) and Tarczynski et al. (Science 259: 508-510, 1993) have all attempted at generating stress tolerant plants.

In addition, several U.S. patents and patent applications also describe polynucleotides associated with stress tolerance and their use in generating stress tolerant plants. U.S. Pat. Nos. 5,296,462 and 5,356,816 describe transforming plants with polynucleotides encoding proteins involved in cold adaptation in *Arabidopsis thaliana,* to thereby promote cold tolerance in the transformed plants.

U.S. Pat. No. 6,670,528 describes transforming plants with polynucleotides encoding polypeptides binding to stress responsive elements, to thereby promote tolerance of the transformed plants to abiotic stress.

U.S. Pat. No. 6,720,477 describes transforming plants with a polynucleotide encoding a signal transduction stress-related protein, capable of increasing tolerance of the transformed plants to abiotic stress.

U.S Application Ser. Nos. 09/938842 and 10/342224 describe abiotic stress-related genes and their use to confer upon plants tolerance to abiotic stress.

U.S. Application Ser. No. 10/231035 describes overexpressing a molybdenum cofactor sulfurase in plants to thereby increase their tolerance to abiotic stress.

Although the above described studies were at least partially successful in generating stress tolerant plants, there remains a need for stress tolerant genes which can be utilized to generate plants tolerant of a wide range of abiotic stress conditions.

While reducing the present invention to practice, the present inventors have identified through bioinmformatic and laboratory studies several novel abiotic stress-tolerance genes, which can be utilized to increase tolerance to abiotic stress and/or biomass in plants.

### SUMMARY OF THE INVENTION

According to one aspect of the present invention there is provided a method of increasing tolerance of a plant to an abiotic stress. The method includes expressing within the plant an exogenous polynucleotide at least 90% homologous to a polynucleotide selected from the group consisting of SEQ ID NOs: 1-18.

According to an additional aspect of the present invention there is provided a method of increasing tolerance of a plant to an abiotic stress. The method includes expressing within the plant an exogenous polynpeptide including an amino acid sequence selected from the group consisting of SEQ ID NOs: 39-92.

According to another aspect of the present invention there is provided a method of increasing biomass of a plant. The method includes expressing within the plant an exogenous polynucleotide at least 90% homologous to a polynucleotide selected from the group consisting of SEQ ID NOs: 1-18.

According to still additional aspect of the present invention there is provided a method of increasing biomass of a plant. The method includes expressing within the plant an exogenous polypeptide including an amino acid sequence selected from the group consisting of SEQ ID NOs: 39-92.

According to yet another aspect of the present invention there is provided a plant cell comprising an exogenous polynucleotide at least 90% homologous to a polynucleotide selected from the group consisting of SEQ ID NOs: 1-18.

According to yet another aspect of the present invention there is provided a plant cell comprising an exogenous polynucleotide encoding a polypeptide including an amino acid sequence selected from the group consisting of SEQ ID NOs: 39-92.

According to still another aspect of the present invention there is provided a nucleic acid construct, including a polynucleotide at least 90% homologous to a nucleotide sequence selected from the group consisting of SEQ ID NOs: 1-18 and a promoter capable of directing transcription of the polynucleotide in a host cell.

According to another aspect of the present invention there is provided a nucleic acid construct, including a polynucleotide encoding a polypeptide comprising an amino acid sequence selected from the group consisting of SEQ ID NOs: 39-92 and a promoter capable of directing transcription of the polynucleotide in a host cell.

According to further yet another aspect of the present invention there is provided an isolated polypeptide, including an amino acid sequence at least 90% homologous to the amino acid sequence encoded by a polynucleotide selected from the group consisting of SEQ ID NOs: 1-18.

According to an additional aspect of the present invention there is provided an isolated polypeptide including an amino acid sequence selected from the group consisting of SEQ ID NOs: 39-92.

According to further features in preferred embodiments of the invention described below, the expressing is effected by (i) transforming a cell of the plant with the exogenous polynucleotide; (ii) generating a mature plant from the cell; and (iii) cultivating the mature plant under conditions suitable for expressing the exogenous polynucleotide within the mature plant.

According to still further features in the described preferred embodiments the transforming is effected by introducing to the plant cell a nucleic acid construct including the exogenous polynucleotide and at least one promoter capable of directing transcription of the exogenous polynucleotide in the plant cell.

According to still further features in the described preferred embodiments the at least one promoter is a constitutive promoter.

According to still further features in the described preferred embodiments the constitutive promoter is CaMV 35S promoter.

According to still further features in the described preferred embodiments the constitutive promoter is At6669 promoter.

According to still further features in the described preferred embodiments the at least one promoter is an inducible promoter.

According to still further features in the described preferred embodiments the inducible promoter is an abiotic stress inducible promoter.

According to still further features in the described preferred embodiments the at least one promoter is a tissue-specific promoter.

According to still further features in the described preferred embodiments the expressing is effected by infecting the plant with a virus including the exogenous polynucleotide.

According to still further features in the described preferred embodiments the virus is an avirulent virus.

According to still further features in the described preferred embodiments the abiotic stress is selected from the group consisting of salinity, water deprivation, low temperature, high temperature, heavy metal toxicity, anaerobiosis, nutrient deficiency, nutrient excess, atmospheric pollution and UV irradiation.

According to still further features in the described preferred embodiments the plant is a dicotyledonous plant.

According to still further features in the described preferred embodiments the plant is a monocotyledonous plant.

According to still further features in the described preferred embodiments the plant cell forms a part of a plant.

The present invention successfully addresses the shortcomings of the presently known configurations by providing methods of utilizing novel abiotic stress-tolerance genes to increase plants tolerance to abiotic stress and/or biomass.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

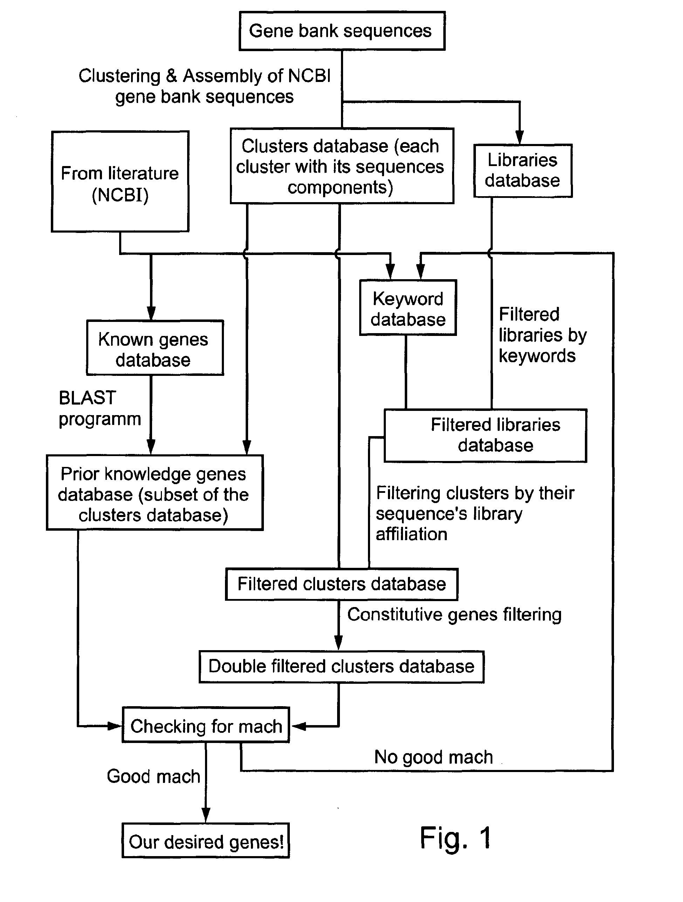

FIG. 1 is a flow chart illustrating a process of identifying putative plant stress-tolerance genes from nucleic-acid sequence databases.

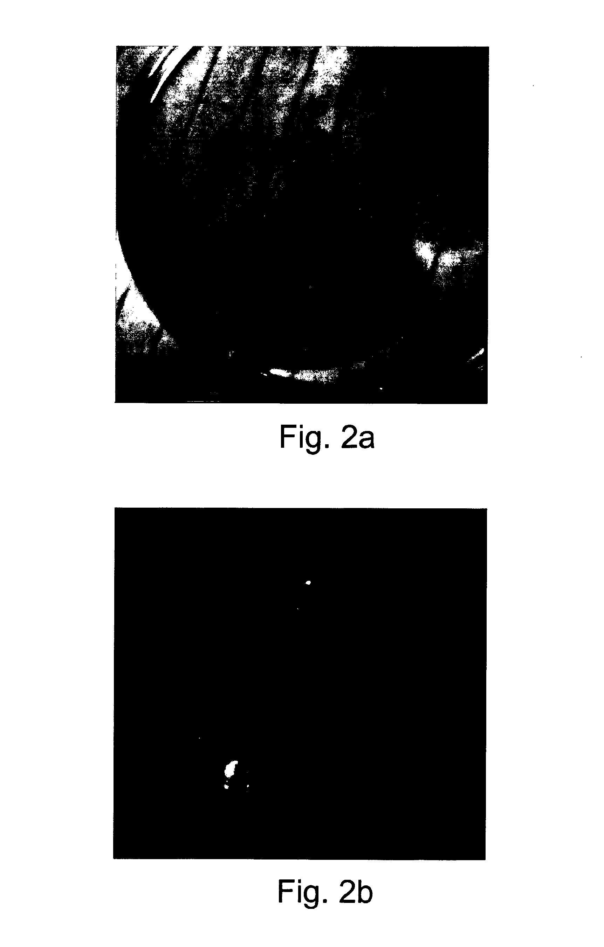

FIGs. 2A-B are photographs illustrating a T2 transgenic *Arabidopsis thaliana* mature plant at flowering stage, expressing exogenous luciferase transgene from the At6669 promoter. The same plant is shown under normal light conditions (Figure 2A) and in the dark (Figure 2B). Strong illumination indicative of luciferase expression is observed in the flower and root tissues.

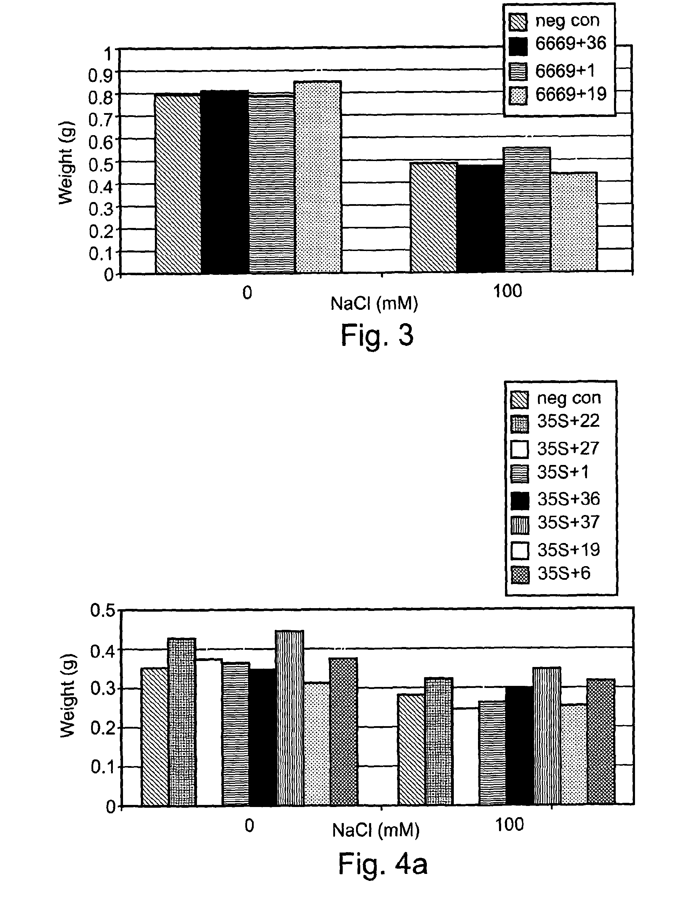

FIG. 3 illustrate the mean fresh weights of transgenic T₁ *A. thaliana* plants grown under normal or stress conditions (irrigated with 0 or 100 M NaCl solution, respectively). The plants were transformed with putative stress tolerance genes, or with luciferase reporter gene (control), positioned under the transcriptional control of the At6669 promoter. Means followed by the same letter are not significantly different according to a one way ANOVA T-Test.
FIG. 4A illustrates the mean fresh weights of T₂ *A. thaliana* plants grown under normal or stress conditions (irrigated with 0 or 100 M NaCl solution, respectively). The plants were transformed with the putative stress tolerance genes of the present invention, or with luciferase reporter gene (control), positioned under the transcriptional control of the 35S promoter. Means followed by the same letter are not significantly different according to a one way ANOVA T-Test.

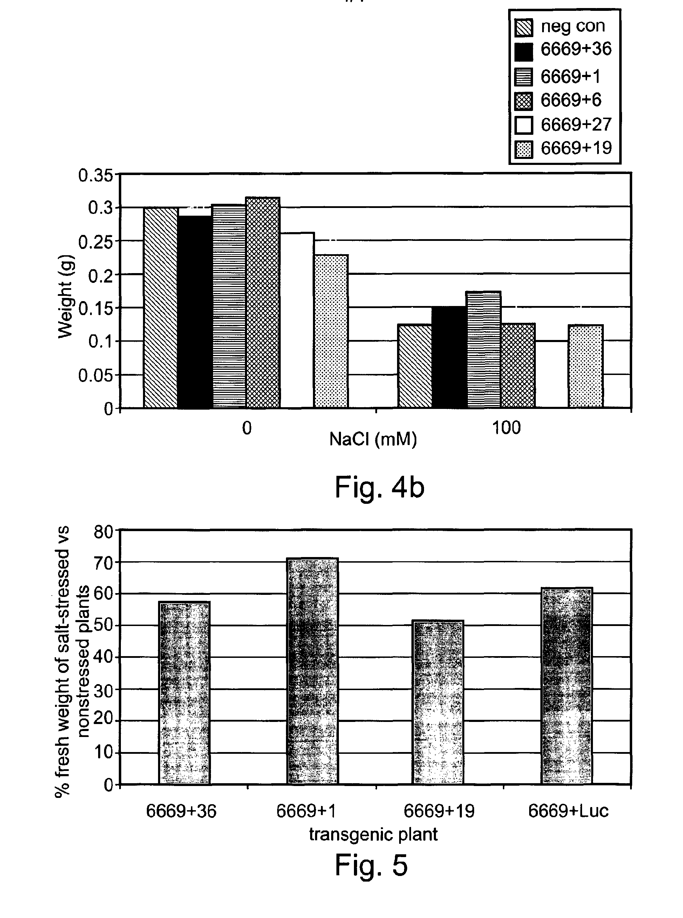

FIG. 4B illustrates the mean fresh weights of T₂ *A. thaliana* plants grown under normal or stress conditions (irrigated with 0 or 100 M NaCl solution, respectively). The plants were transformed with the putative stress tolerance genes of the present invention, or with luciferase reporter gene (control), positioned under the transcriptional control of the At6669 promoter. Means followed by the same letter are not significantly different according to a one way ANOVA T-Test.
FIG. 5 illustrates the relative (percent) fresh-weight of transgenic T₁ *A*. *thaliana* plants grown under salinity stress conditions (irrigated with 100 mM NaCl solution), as compared with similar plants grown under normal conditions (irrigated with water only). The plants were transformed with the putative stress tolerance genes of the present invention, or with luciferase reporter gene (control), positioned under the transcriptional control of the At6669 promoter.

### DESCRIPTION OF THE PREFERRED EMBODIMENTS

The present invention is of methods of increasing plants tolerance to abiotic stress and/or biomass by utilizing novel abiotic stress tolerance genes and of plants exhibiting increased tolerance to stress conditions and/or increased capacity to accumulate biomass.

The principles and operation of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

While reducing the present invention to practice, the present inventors while employing bioinformatic techniques, identified polynucleotide sequences which encode putative abiotic-stress tolerance (ABST) proteins (Example 1). Selected sequences were isolated (Example 2), cloned into expression vectors (Example 3-4) and introduced into *Arabidopsis thaliana* plants (Example 5). These plants, which were grown under salinity stress conditions, or under normal conditions, exhibited significantly higher biomass as compared with similar plants not carrying the exogenous ABST genes (Example 6).

Thus, according to one aspect of the present invention, there is provided a method of increasing tolerance of a plant to an abiotic stress and/or plant biomass. The method includes expressing within the plant an exogenous polynucleotide at least 70% homologous, preferably at least 80% homologous, more preferably at least 85% homologous, most preferably at least 90% homologous to a polynucleotide selected from the group consisting of SEQ ID NOs: 1-18. Alternatively, the exogenous polynucleotide of the present invention encodes a polypeptide having an amino acid sequence selected from the group consisting of SEQ ID NOs: 39-92.

The phrase "abiotic stress" used herein refers to any adverse effect on metabolism, growth, reproduction and/or viability of a plant. Accordingly, abiotic stress can be induced by suboptimal environmental growth conditions such as, for example, salinity, water deprivation, flooding, low or high temperature, heavy metal toxicity, anaerobiosis, nutrient deficiency, atmospheric pollution or UV irradiation.

The phrase "abiotic stress tolerance" as used herein refers to the ability of a plant to endure an abiotic stress without suffering a substantial alteration in metabolism, growth, productivity and/or viability.

A suitable plant for use with the method of the present invention can be any monocotyledonous or dicotyledonous plant including, but not limited to, maize, wheat, barely, rye, oat, rice, soybean, peanut, pea, lentil and alfalfa, cotton, rapeseed, canola, pepper, sunflower, potato, tobacco, tomato, eggplant, eucalyptus, a tree, an ornamental plant, a perennial grass and a forage crop.

As used herein, the term "exogenous polynucleotide" refers to a nucleic acid sequence which is not naturally expressed within the plant but which, when introduced into the plant either in a stable or transient manner, produces at least one polypeptide product.

Expressing the exogenous polynucleotide of the present invention within the plant can be effected by transforming one or more cells of the plant with the exogenous polynucleotide, followed by generating a mature plant from the transformed cells and cultivating the mature plant under conditions suitable for expressing the exogenous polynucleotide within the mature plant.

Preferably, the transformation is effected by introducing to the plant cell a nucleic acid construct which includes the exogenous polynucleotide of the present invention and at least one promoter capable of directing transcription of the exogenous polynucleotide in the plant cell. Further details of suitable transformation approaches are provided hereinbelow.

As used herein, the term "promoter" refers to a region of DNA which lies upstream of the transcriptional initiation site of a gene to which RNA polymerase binds to initiate transcription of RNA. The promoter controls where (e.g., which portion of a plant, which organ within an animal, etc.) and/or when (e.g., which stage or condition in the lifetime of an organism) the gene is expressed.

Any suitable promoter sequence can be used by the nucleic acid construct of the present invention. Preferably the promoter is a constitutive promoter, a tissue-specific, or an abiotic stress-inducible promoter.

Suitable constitutive promoters include, for example, CaMV 35S promoter (SEQ ID NO: 19; Odell et al., Nature 313:810-812, 1985); Arabidopsis At6669 promoter (SEQ ID NO: 20); maize Ubi 1 (Christensen et al., Plant Sol. Biol. 18:675-689, 1992); rice actin (McElroy et al., Plant Cell 2:163-171, 1990); pEMU (Last et al., Theor. Appl. Genet. 81:581-588, 1991); and Synthetic Super MAS (Ni et al., The Plant Journal 7: 661-76, 1995). Other constitutive promoters include those in U.S. Pat. Nos. 5,659,026, 5,608,149; 5.608,144; 5,604,121; 5.569,597: 5.466,785; 5,399,680; 5,268,463; and 5,608,142.

Suitable tissue-specific promoters include, but not limited to, leaf-specific promoters such as described, for example, by Yamamoto et al., Plant J. 12:255-265, 1997; Kwon et al., Plant Physiol. 105:357-67, 1994; Yamamoto et al., Plant Cell Physiol. 35:773-778, 1994; Gotor et al., Plant J. 3:509-18, 1993; Orozco et al., Plant Mol. Biol. 23:1129-1138, 1993; and Matsuoka et al., Proc. Natl. Acad. Sci. USA 90:9586-9590, 1993.

Suitable abiotic stress-inducible promoters include, but not limited to, salt-inducible promoters such as RD29A (Yamaguchi-Shinozalei et al., Mol. Gen. Genet. 236:331-340, 1993); drought-inducible promoters such as maize rab17 gene promoter (P1a et. al., Plant Mol. Biol. 21:259-266, 1993), maize rab28 gene promoter (Busk et. al., Plant J. 11:1285-1295, 1997) and maize Ivr2 gene promoter (Pelleschi et. al., Plant Mol. Biol. 39:373-380, 1999); and heat-inducible promoters such as heat tomato hsp80-promoter from tomato (U.S. Pat. No. 5,187,267).

The nucleic acid construct of the present invention preferably further includes an appropriate selectable marker and/or an origin of replication. Preferably, the nucleic acid construct utilized is a shuttle vector, which can propagate both in *E*. *coli* (wherein the construct comprises an appropriate selectable marker and origin of replication) and be compatible for propagation in cells. The construct according to the present invention can be, for example, a plasmid, a bacmid, a phagemid, a cosmid, a phage, a virus or an artificial chromosome.

The nucleic acid construct of the present invention can be utilized to stably or transiently transform plant cells. In stable transformation, the exogenous polynucleotide of the present invention is integrated into the plant genome and as such it represents a stable and inherited trait. In transient transformation, the exogenous polynucleotide is expressed by the cell transformed but it is not integrated into the genome and as such it represents a transient trait.

There are various methods of introducing foreign genes into both monocotyledonous and dicotyledonous plants (Potrykus, I., Annu. Rev. Plant. Physiol., Plant. Mol. Biol. (1991) 42:205-225; Shimamoto et al., Nature (1989) 338:274-276).

The principle methods of causing stable integration of exogenous DNA into plant genomic DNA include two main approaches:
(i) Agrobacterium-mediated gene transfer: Klee et al. (1987) Annu. Rev. Plant Physiol. 38:467-486; Klee and Rogers in Cell Culture and Somatic Cell Genetics of Plants, Vol. 6, Molecular Biology of Plant Nuclear Genes, eds. Schell, J., and Vasil, L. K., Academic Publishers, San Diego, Calif. (1989) p. 2-25; Gatenby, in Plant Biotechnology, eds. Kung, S. and Arntzen, C. J., Butterworth Publishers, Boston, Mass. (1989) p. 93-112.
(ii) Direct DNA uptake: Paszkowski et al., in Cell Culture and Somatic Cell Genetics of Plants, Vol. 6, Molecular Biology of Plant Nuclear Genes eds. Schell, J., and Vasil, L. K., Academic Publishers, San Diego, Calif. (1989) p. 52-68; including methods for direct uptake of DNA into protoplasts, Toriyama, K. et al. (1988) Bio/Technology 6:1072-1074. DNA uptake induced by brief electric shock of plant cells: Zhang et al. Plant Cell Rep. (1988) 7:379-384. Fromm et al. Nature (1986) 319:791-793. DNA injection into plant cells or tissues by particle bombardment, Klein et al. Bio/Technology (1988) 6:559-563; McCabe et al. Bio/Technology (1988) 6:923-926; Sanford, Physiol. Plant. (1990) 79:206-209; by the use of micropipette systems: Neuhaus et al., Theor. Appl. Genet. (1987) 75:30-36; Neuhaus and Spangenberg, Physiol. Plant. (1990) 79:213-217; glass fibers or silicon carbide whisker transformation of cell cultures, embryos or callus tissue, U.S. Pat. No. 5,464,765 or by the direct incubation of DNA with germinating pollen, DeWet et al. in Experimental Manipulation of Ovule Tissue, eds. Chapman, G. P. and Mantell, S. H. and Daniels, W. Longman, London, (1985) p. 197-209; and Ohta, Proc. Natl. Acad. Sci. USA (1986) 83:715-719.

The *Agrobacterium* system includes the use of plasmid vectors that contain defined DNA segments that integrate into the plant genomic DNA. Methods of inoculation of the plant tissue vary depending upon the plant species and the *Agrobacterium* delivery system. A widely used approach is the leaf disc procedure which can be performed with any tissue explant that provides a good source for initiation of whole plant differentiation. Horsch et al. in Plant Molecular Biology Manual A5, Kluwer Academic Publishers, Dordrecht (1988) p. 1-9. A supplementary approach employs the *Agrobacterium* delivery system in combination with vacuum infiltration. The *Agrobacterium* system is especially viable in the creation of transgenic dicotyledonous plants.

There are various methods of direct DNA transfer into plant cells. In electroporation, the protoplasts are briefly exposed to a strong electric field. In microinjection, the DNA is mechanically injected directly into the cells using very small micropipettes. In microparticle bombardment, the DNA is adsorbed on microprojectiles such as magnesium sulfate crystals or tungsten particles, and the microprojectiles are physically accelerated into cells or plant tissues.

Following stable transformation plant propagation is exercised. The most common method of plant propagation is by seed. Regeneration by seed propagation, however, has the deficiency that due to heterozygosity there is a lack of uniformity in the crop, since seeds are produced by plants according to the genetic variances governed by Mendelian rules. Basically, each seed is genetically different and each will grow with its own specific traits. Therefore, it is preferred that the transformed plant be produced such that the regenerated plant has the identical traits and characteristics of the parent transgenic plant. Therefore, it is preferred that the transformed plant be regenerated by micropropagation which provides a rapid, consistent reproduction of the transformed plants.

Micropropagation is a process of growing new generation plants from a single piece of tissue that has been excised from a selected parent plant or cultivar. This process permits the mass reproduction of plants having the preferred tissue expressing the fusion protein. The new generation plants which are produced are genetically identical to, and have all of the characteristics of, the original plant. Micropropagation allows mass production of quality plant material in a short period of time and offers a rapid multiplication of selected cultivars in the preservation of the characteristics of the original transgenic or transformed plant. The advantages of cloning plants are the speed of plant multiplication and the quality and uniformity of plants produced.

Micropropagation is a multi-stage procedure that requires alteration of culture medium or growth conditions between stages. Thus, the micropropagation process involves four basic stages: Stage one, initial tissue culturing; stage two, tissue culture multiplication; stage three, differentiation and plant formation; and stage four, greenhouse culturing and hardening. During stage one, initial tissue culturing, the tissue culture is established and certified contaminant-free. During stage two, the initial tissue culture is multiplied until a sufficient number of tissue samples are produced to meet production goals. During stage three, the tissue samples grown in stage two are divided and grown into individual plantlets. At stage four, the transformed plantlets are transferred to a greenhouse for hardening where the plants' tolerance to light is gradually increased so that it can be grown in the natural environment.

Preferably, mature transformed plants generated as described above are further selected for abiotic stress tolerance. Accordingly, transformed and non-transformed (wild type) plants are exposed to an abiotic stress condition, such as water depravation, suboptimal temperature, nutrient deficiency, or preferably a salt stress condition. Salt stress can be effected in many ways such as, for example, by irrigating the plants with a hyperosmotic solution, by cultivating the plants hydroponically in a hyperosmotic growth solution (e.g., Hoagland solution), or by culturing the plants in a hyperosmotic growth medium (e.g., MS medium). Since different plants vary considerably in their tolerance to salinity, the salt concentration in the irrigation water, growth solution, or growth medium is preferably adjusted according to the specific characteristics of the specific plant cultivar or variety, so as to inflict a mild or moderate effect on the physiology and/or morphology of the plants (for guidelines as to appropriate concentration please see, Bernstein and Kafkafi, Root Growth Under Salinity Stress In: Plant Roots, The Hidden Half 3rd ed. Waisel Y, Eshel A and Kafkafi U. (editors) Marcel Dekker Inc., New York, 2002, and reference therein). Following exposure to the stress condition the plants are frequently monitored until substantial physiological and/or morphological effects appear in wild type plants. Subsequently, transformed plants not exhibiting substantial physiological and/or morphological effects, or exhibiting higher biomass than wild-type plants, are identified as abiotic stress tolerant plants.

Although stable transformation is presently preferred, transient transformation of leaf cells, meristematic cells or the whole plant is also envisaged by the present invention.

Transient transformation can be effected by any of the direct DNA transfer methods described above or by viral infection using modified plant viruses.

Viruses that have been shown to be useful for the transformation of plant hosts include CaMV, TMV and BV. Transformation of plants using plant viruses is described in U.S. Pat. No. 4,855,237 (BGV), EP-A 67,553 (TMV), Japanese Published Application No. 63-14693 (TMV), EPA 194,809 (BV), EPA 278,667 (BV); and Gluzman, Y. et al., Communications in Molecular Biology: Viral Vectors, Cold Spring Harbor Laboratory, New York, pp. 172-189 (1988). Pseudovirus particles for use in expressing foreign DNA in many hosts, including plants, is described in WO 87/06261.

Preferably, the virus of the present invention is avirulent and thus is incapable of causing severe symptoms such as reduced growth rate, mosaic, ring spots, leaf roll, yellowing, streaking, pox formation, tumor formation and pitting. A suitable avirulent virus may be a naturally occurring avirulent virus or an artificially attenuated virus. Virus attenuation may be effected by using methods well known in the art including, but not limited to, sub-lethal heating, chemical treatment or by directed mutagenesis techniques such as described, for example, by Kurihara and Watanabe (Molecular Plant Pathology 4:259-269, 2003), Gal-on *et al.* (1992), Atreya *et al.* (1992) and Huet *et al.* (1994).

Suitable virus strains can be obtained from available sources such as, for example, the American Type culture Collection (ATCC) or by isolation from infected plants. Isolation of viruses from infected plant tissues can be effected by techniques well known in the art such as described, for example by Foster and Tatlor, Eds. "Plant Virology Protocols: From Virus Isolation to Transgenic Resistance (Methods in Molecular Biology (Humana Pr), Vol 81)", Humana Press, 1998. Briefly, tissues of an infected plant believed to contain a high concentration of a suitable virus, preferably young leaves and flower petals, are ground in a buffer solution (e.g., phosphate buffer solution) to produce a virus infected sap which can be used in subsequent inoculations.

Construction of plant RNA viruses for the introduction and expression of non-viral exogenous polynucleotide sequences in plants is demonstrated by the above references as well as by Dawson, W. O. et al., Virology (1989) 172:285-292; Takamatsu et al. EMBO J. (1987) 6:307-311; French et al. Science (1986) 231:1294-1297; and Takamatsu et al. FEBS Letters (1990) 269:73-76.

When the virus is a DNA virus, suitable modifications can be made to the virus itself. Alternatively, the virus can first be cloned into a bacterial plasmid for ease of constructing the desired viral vector with the foreign DNA. The virus can then be excised from the plasmid. If the virus is a DNA virus, a bacterial origin of replication can be attached to the viral DNA, which is then replicated by the bacteria. Transcription and translation of this DNA will produce the coat protein which will encapsidate the viral DNA. If the virus is an RNA virus, the virus is generally cloned as a cDNA and inserted into a plasmid. The plasmid is then used to make all of the constructions. The RNA virus is then produced by transcribing the viral sequence of the plasmid and translation of the viral genes to produce the coat protein(s) which encapsidate the viral RNA.

Construction of plant RNA viruses for the introduction and expression in plants of non-viral exogenous polynucleotide sequences such as those included in the construct of the present invention is demonstrated by the above references as well as in U.S. Pat. No. 5,316,931.

In one embodiment, a plant viral polynucleotide is provided in which the native coat protein coding sequence has been deleted from a viral polynucleotide, a non-native plant viral coat protein coding sequence and a non-native promoter, preferably the subgenomic promoter of the non-native coat protein coding sequence, capable of expression in the plant host, packaging of the recombinant plant viral polynucleotide, and ensuring a systemic infection of the host by the recombinant plant viral polynucleotide, has been inserted. Alternatively, the coat protein gene may be inactivated by insertion of the non-native polynucleotide sequence within it, such that a protein is produced. The recombinant plant viral polynucleotide may contain one or more additional non-native subgenomic promoters. Each non-native subgenomic promoter is capable of transcribing or expressing adjacent genes or polynucleotide sequences in the plant host and incapable of recombination with each other and with native subgenomic promoters. Non-native (foreign) polynucleotide sequences may be inserted adjacent the native plant viral subgenomic promoter or the native and a non-native plant viral subgenomic promoters if more than one polynucleotide sequence is included. The non-native polynucleotide sequences are transcribed or expressed in the host plant under control of the subgenomic promoter to produce the desired products.

In a second embodiment, a recombinant plant viral polynucleotide is provided as in the first embodiment except that the native coat protein coding sequence is placed adjacent one of the non-native coat protein subgenomic promoters instead of a non-native coat protein coding sequence.

In a third embodiment, a recombinant plant viral polynucleotide is provided in which the native coat protein gene is adjacent its subgenomic promoter and one or more non-native subgenomic promoters have been inserted into the viral polynucleotide. The inserted non-native subgenomic promoters are capable of transcribing or expressing adjacent genes in a plant host and are incapable of recombination with each other and with native subgenomic promoters. Non-native polynucleotide sequences may be inserted adjacent the non-native subgenomic plant viral promoters such that the sequences are transcribed or expressed in the host plant under control of the subgenomic promoters to produce the desired product.

In a fourth embodiment, a recombinant plant viral polynucleotide is provided as in the third embodiment except that the native coat protein coding sequence is replaced by a non-native coat protein coding sequence.

The viral vectors are encapsidated by the coat proteins encoded by the recombinant plant viral polynucleotide to produce a recombinant plant virus. The recombinant plant viral polynucleotide or recombinant plant virus is used to infect appropriate host plants. The recombinant plant viral polynucleotide is capable of replication in the host, systemic spread in the host, and transcription or expression of foreign gene(s) (exogenous polynucleotide) in the host to produce the desired protein.

Techniques for inoculation of viruses to plants may be found in Foster and Taylor, eds. "Plant Virology Protocols: From Virus Isolation to Transgenic Resistance (Methods in Molecular Biology (Humana Pr), Vol 81)", Humana Press, 1998; Maramorosh and Koprowski, eds. "Methods in Virology" 7 vols, Academic Press, New York 1967-1984; Hill, S.A. "Methods in Plant Virology", Blackwell, Oxford, 1984; Walkey, D.G.A. "Applied Plant Virology", Wiley, New York, 1985; and Kado and Agrawa, eds. "Principles and Techniques in Plant Virology", Van Nostrand-Reinhold, New York.

In addition to the above, the polynucleotide of the present invention can also be introduced into a chloroplast genome thereby enabling chloroplast expression.

A technique for introducing exogenous polynucleotide sequences to the genome of the chloroplasts is known. This technique involves the following procedures. First, plant cells are chemically treated so as to reduce the number of chloroplasts per cell to about one. Then, the exogenous polynucleotide is introduced via particle bombardment into the cells with the aim of introducing at least one exogenous polynucleotide molecule into the chloroplasts. The exogenous polynucleotides selected such that it is integratable into the chloroplast's genome via homologous recombination which is readily effected by enzymes inherent to the chloroplast. To this end, the exogenous polynucleotide includes, in addition to a gene of interest, at least one polynucleotide stretch which is derived from the chloroplast's genome. In addition, the exogenous polynucleotide includes a selectable marker, which serves by sequential selection procedures to ascertain that all or substantially all of the copies of the chloroplast genomes following such selection will include the exogenous polynucleotide. Further details relating to this technique are found in U.S. Pat. Nos. 4,945,050; and 5,693,507 which are incorporated herein by reference. A polypeptide can thus be produced by the protein expression system of the chloroplast and become integrated into the chloroplast's inner membrane.

Since abiotic stress tolerance in plants can involve multiple genes acting additively or in synergy (see, for example, in Quesda et al., Plant Physiol. 130:951-063, 2002), the present invention also envisages expressing a plurality of exogenous polynucleotides in a single host plant to thereby achieve superior abiotic stress tolerance.

Expressing a plurality of exogenous polynucleotides in a single host plant can be effected by co-introducing multiple nucleic acid constructs, each including a different exogenous polynucleotide, into a single plant cell. The transformed cell can than be regenerated into a mature plant using the methods described hereinabove.

Alternatively, expressing a plurality of exogenous polynucleotides in a single host plant can be effected by co-introducing into a single plant-cell a single nucleic-acid construct including a plurality of different exogenous polynucleotides. Such a construct can be designed with a single promoter sequence which can transcribe a polycistronic message including all the different exogenous polynucleotide sequences. To enable co-translation of the different polypeptides encoded by the polycistronic message, the polynucleotide sequences can be inter-linked via an internal ribosome entry site (IRES) sequence which facilitates translation of polynucleotide sequences positioned downstream of the IRES sequence. In this case, a transcribed polycistronic RNA molecule encoding the different polypeptides described above will be translated from both the capped 5' end and the two internal IRES sequences of the polycistronic RNA molecule to thereby produce in the cell all different polypeptides. Alternatively, the construct can include several promoter sequences each linked to a different exogenous polynucleotide sequence.

The plant cell transformed with the construct including a plurality of different exogenous polynucleotides, can be regenerated into a mature plant, using the methods described hereinabove.

Alternatively, expressing a plurality of exogenous polynucleotides in a single host plant can be effected by introducing different nucleic acid constructs, including different exogenous polynucleotides, into a plurality of plants. The regenerated transformed plants can then be cross-bred and resultant progeny selected for superior abiotic stress tolerance and/or biomass traits, using conventional plant breeding techniques.

Hence, the present application provides methods of utilizing novel abiotic stress-tolerance genes to increase tolerance to abiotic stress and/or biomass in a wide range of economical plants, safely and cost effectively.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

### EXAMPLE 1

### Identifying putative abiotic stress- tolerance genes

Putative abiotic stress-tolerance (ABST) genes were selected from NCBI databases of tomato expressed sequence tags (ESTs) and cDNAs. The database sequences were clustered and assembled using the LEADS^{™} software (Compugen). Clustering resulted in more than 20,000 clusters, each representing a different gene. An expression profile summary was compiled for each cluster by pooling all keywords included in the sequence records comprising the cluster. The clusters were then screened to include polynucleotides originating from libraries identified by keywords relating to ABST. The selected clusters were further filtered to exclude any cluster which included more than 100 ESTs per cluster and/or any cluster in which less than 50% of the sequences are annotated by ABST-related keywords.

Prior art ABST plant genes were identified from the publications of Quesada et al. (Plant Physiol. 130:951-963, 2002); Apse and Blumwald (Curr Opin Biotechnol. 13:146-150, 2002); Rontein et al. (Metab Eng 4:49-56, 2002); and references therein. Known plant ABST genes were aligned with the clustered tomato nucleic-acid sequences, using the BLAST program. The tomato sequences having an e-score value lower than 5 were identified as ABST orthologes. Additional prior art tomato ABST genes were identified by searching the clustered tomato sequence records using the keywords "root", "crown gall", "nutrient", "callus", "disease", "pathogen", "elicitor" and "pseudomonas".

Finally, all identified prior art ABST genes were matched (by sequence alignment using the BLAST software) with the output set of tomato gene clusters, selected as described above. Consequently, about 40% of the genes selected in the output set of clusters which matched with prior art ABST genes proved to be known ABST genes, indicating that the remaining genes of the selected clusters are potentially capable of increasing abiotic stress tolerance in plants.

The selected polynucleotide sequences (Table 1a), were analyzed for presence of ORFs using Vector NTI suite (InforMax, U.K.) version 6 (Hasting Software, Inc: www.generunner.com/). ORFs identified in each of these polynucleotide sequences were compared to Genbank database sequences, using Blast (www.ncbi.nlm.nih.gov/BLAST/); the ORF displaying the highest homology to a GenBank sequence or sequences, was mapped in order to identify an ATG start codon. The position of the ATG start codon of this ORF was then compared with that of the identified polynucleotide sequence in order to verify that each of the eighteen sequences described herein includes a full length ORF and an ATG start codon (thus qualifies as a "putative ABST gene").

**Table 1a Putative ABST genes**

| ABST No. | SEQ ID No. |
|---|---|
| 1 | 1 |
| 3 | 2 |
| 5 | 3 |
| 6 | 4 |
| 10 | 5 |
| 11 | 6 |
| 12 | 7 |
| 19 | 8 |
| 22 | 9 |
| 24 | 10 |
| 26 | 11 |
| 27 | 12 |
| 36 | 13 |
| 37 | 14 |
| 39_T0 | 15 |
| 39_T1 | 16 |
| 49_T0 | 17 |
| 49_T1 | 18 |

ABST polypeptide homologues have been identified from the NCBI databases using BLAST software (Table 1b).

**Table 1b ARST homologues**

| **ABST Putative Gene SEQ ID No.** | **ABST Polypeptide Homologue NCBI Accession No.** | **ABST Polypeptide Homologue SEQ ID No.** | **Homology (%)** |
|---|---|---|---|
| 1 | BAA96366 | 39 | 98 |
| 1 | AAS47510 | 40 | 98 |
| 1 | NP_567151 | 41 | 97 |
| 1 | NP_567104 | 42 | 96 |
| 1 | AAK55664 | 43 | 96 |
| 1 | P46298 | 44 | 97 |
| 1 | T01338 | 45 | 96 |
| 1 | T47888 | 46 | 95 |
| 1 | BAD09465 | 47 | 92 |
| 1 | Q05761 | 48 | 91 |
| 1 | BAD09464 | 49 | 88 |
| 1 | CAA79496 | 50 | 84 |
| 1 | EAJ94592 | 51 | 85 |
| 4 | NP 188036 | 52 | 76 |
| 4 | NP_035977 | 53 | 70 |
| 4 | XP_342608 | 54 | 69 |
| 4 | T09295 | 55 | 60 |
| 4 | NP_564717 | 56 | 59 |
| 4 | AAM63624 | 57 | 59 |
| 9 | P37707 | 58 | 93 |
| 9 | CAD37200 | 59 | 81 |
| 9 | CAA04664 | 60 | 78 |
| 9 | AAM64572 | 61 | 77 |
| 9 | NP 189345 | 62 | 77 |
| 9 | NP 974979 | 63 | 60 |
| 13 | AAC49992 | 64 | 88 |
| 13 | T10804 | 65 | 87 |
| 13 | AAL38357 | 66 | 87 |
| 13 | NP 188245 | 67 | 87 |
| 13 | NP 193465 | 68 | 87 |
| 13 | AAG44945 | 69 | 86 |
| 13 | T07819 | 70 | 86 |
| 13 | T12632 | 71 | 86 |
| 13 | CAC39073 | 72 | 86 |
| 13 | T01648 | 73 | 86 |
| 13 | AAF90121 | 74 | 86 |
| 13 | S48116 | 75 | 86 |
| 13 | AA086710 | 76 | 86 |
| 13 | T14002 | 77 | 85 |
| 13 | T14001 | 78 | 85 |
| 13 | T48886 | 79 | 85 |
| 13 | T14314 | 80 | 85 |
| 13 | P33560 | 81 | 85 |
| 13 | P21653 | 82 | 85 |
| 13 | T14000 | 83 | 85 |
| 13 | T48884 | 84 | 85 |
| 13 | P24422 | 85 | 85 |
| 13 | AAB53329 | 86 | 85 |
| 14 | NP 200279 | 87 | 67 |
| 14 | AAM64276 | 88 | 67 |
| 14 | AAO72577 | 89 | 66 |
| 14 | NP 175518 | 90 | 64 |
| 14 | BAC78588 | 91 | 64 |
| 14 | BAD03011 | 92 | 62 |

### EXAMPLE 2

### Isolation of putative ABS tolerance genes

RNA was extracted from 4 week-old tomato root and leaf tissues using Tri Reagent (Molecular Research Center, Inc), following the protocol provided by the manufacturer (www.mrcgene.com/tri.htm). Complementary DNA molecules were produced from the extracted mRNA using M-MuLV reverse-transcriptase (RT) enzyme (Roche) and T₁₆NN DNA primer, according to the manufacturer's instructions. The cDNA sequences set forth in SEQ ID NOs: 1,4,8-9 and 12-14, were amplified by PCR using the primers described in Table 2 below, with PFU proof reading DNA polymerase enzyme (Promega-www.promega.com/pnotes/68/381 07/7381 07.html), following the protocol provided by the manufacturer. Additional restriction endonuclease sites were added to the 5' prime end of each primer to facilitate cloning of the putative ABS tolerance genes in binary vectors.

**Table 2 PCR primers used for amplifying putative ABS tolerance (ABST) genes**

| **ABST gene SEQ ID No** | **Forward Primer SEQ ID No** | **Reverse Primer SEQ ID No** | **upstream restriction site** | **downstream restriction site** |
|---|---|---|---|---|
| 1 | 21 | 22 | BamH1 | SacI |
| 4 | 23 | 24 | BamH1 | SacI |
| 8 | 25 | 26 | BamH1 | SacI |
| 9 | 27 | 28 | XbaI | SmaI |
| 12 | 29 | 30 | BamH1 | SacI |
| 13 | 31 | 32 | BamH1 | SacI |
| 14 | 33 | 34 | BamH1 | SmaI |

### EXAMPLE 3

### Cloning the putative ABST genes

The resulting PCR blunt ended products were purified using PCR Purification Kit (Qiagen, Germany), digested with the appropriate restriction enzymes (Roche) and then inserted into the binary plasmid vector pPI. The plasmid pPI was constructed by inserting a synthetic poly-(A) signal sequence, originating from pGL3 basic plasmid vector (Promega, Acc No U47295; bp 4658-4811) into the *Hind*III restriction site of the binary vector pBI101.3 (Clontech, Acc. No. U12640).

The resulting pPI plasmid was digested with restriction enzymes (*Bam*HI and *Sac*I; MBI Fermentas) and purified using PCR Purification Kit (Qiagen, Germany). The open pPI construct was then ligated with each of the seven PCR products described hereinabove. The ligation was effected using a ligation mixture containing T4 DNA ligase enzyme (Roche) and was performed according to the manufacturer's instructions.

The pPI constructs harboring putative ABST genes were introduced to *E. coli* DH5 competent cells by electroporation, using a MicroPulser electroporator (Biorad), 0.2 cm cuvettes (Biorad) and EC-2 electroporation program (Biorad). The treated cells were cultured in LB liquid medium at 37°C for 1 hr, then plated over LB agar supplemented with kanamycin (50 mg/L; Sigma) and incubated at 37°C for 16 hrs. Colonies which developed on the selective medium were analyzed by PCR using the primers set forth in SEQ ID NOs: 35-36, which were designed to span the inserted sequence in the pPI plasmid. The resulting PCR products were separated on 1.5% agarose gels and the DNA fragment having the predicted size were isolated and sequenced using the ABI 377 sequencer (Amersham Biosciences Inc) in order to verify that the correct DNA sequences were properly introduced to the *E. coli* cells.

### EXAMPLE 4

### Generating binary vectors comprising putative ABST genes and plant promoters operably linked thereto

***Generating binary vectors comprising the Cauliflower Mosaic Virus 35S promoter:*** The Cauliflower Mosaic Virus 35S promoter sequence (set forth in SEQ ID NO: 19) was inserted upstream of the putative ABST gene in each of the pPI constructs described above. The promoter was isolated from the pBI121 plasmid (Clontech, Accession No. AF485783) using the restriction endonucleases *Hind*III and *Bam*HI (Roche). The isolated promoter was ligated into the pPI constructs digested with the same enzymes. Altogether, seven pPI constructs were generated, each comprising the CaMV 35S promoter positioned upstream of a putative ABST gene having a sequence set forth in SEQ ID NO: 1,4,8,9,12,13 or 14.

***Generating binary vectors comprising the At6669 promoter:*** The At6669 promoter sequence (set forth in SEQ ID NO: 20) was inserted upstream of the putative ABST gene in each of the pPI binary constructs described above. The promoter was isolated from *Arabidopsis thaliana* var Co10 genomic DNA by PCR amplification using the primers set forth in SEQ ID NOs: 37-38. The PCR product was purified (Qiagen, Germany) and digested with the restriction endonucleases *Hind*III and *Bam*HI (Roche). The resulting promoter sequence was introduced into the open binary constructs digested with the same enzymes. Altogether, seven pPI constructs were generated, each comprising the At6669 promoter positioned upstream of a putative ABST gene having a sequence set forth in SEQ ID NO: 1,4,8,9,12,13 or 14.

### EXAMPLE 5

### Confirming At6669 promoter activity in transgenic Arabidopsis thaliana

The capacity of At-6669 promoter to regulate transcription of genes carried by the pPI vector in plants was tested. Accordingly, the promoter At6669 was inserted into the pPI binary vector upstream of a Luciferase reporter gene. The binary vector was introduced to *Arabidopsis thaliana* plants using the procedure as described in Example 6 below. Mature transformed T₂ *Arabidopsis* plants were assayed for bio-illumination in a darkroom using an ultra-low light detection camera (Princeton Instruments Inc., USA) using the procedure described by Meissner *et al.* (Plant J. 22:265, 2000). Illumination indicating positive Luciferase activity was observed in the flower and root meristem tissues of transformed plants (Figure 2).

### EXAMPLE 6

### Transforming Agrobacterium tumefaciens cells with binary vectors harboring putative ABST genes

Each of the binary vectors described in Example 4 above were used to transform *Agrobacterium* cells. Two additional binary constructs, having the Luciferase reporter gene replacing an ABST gene (positioned downstream of the 35S or At6669 promoter), were used as negative controls.

The binary vectors were introduced to *Agrobacterium tumefaciens* GV301, or LB4404 competent cells (about 10⁹ cells/mL) by electroporation. The electroporation was effected by using a MicroPulser electroporator (Biorad), 0.2 cm cuvettes (Biorad) and EC-2 electroporation program (Biorad). The treated cells were cultured in LB liquid medium at 28°C for 3 hr, then plated over LB agar supplemented with gentamycin (50 mg/L; for *Agrobacterium* strains GV301) or streptomycin (300 mg/L; for *Agrobacterium* strain LB4404) and kanamycin (50 mg/L) at 28°C for 48 hrs. *Abrobacterium* colonies which developed on the selective media were analyzed by PCR using the primers set forth in SEQ ID NOs: 35-36, which were designed to span the inserted sequence in the pPI plasmid. The resulting PCR products were isolated and sequenced as described in Example 4 above, to verify that the correct ABST sequences were properly introduced to the *Agrobacterium* cells.

### EXAMPLE 7

### Transformation of Arabidopsis thaliana plants with putative ABSTgenes

*Arabidopsis thaliana* Columbia plants (T₀ plants) were transformed using the Floral Dip procedure described by Clough and Bent (10) and by Desfeux *et al.* (11), with minor modifications. Briefly, T₀ Plants were sown in 250 ml pots filled with wet peat-based growth mix. The pots were covered with aluminum foil and a plastic dome, kept at 4°C for 3-4 days, then uncovered and incubated in a growth chamber at 18-24°C under 16/8 hr light/dark cycles. The T₀ plants were ready for transformation six days before anthesis.

Single colonies of *Agrobacterium* carrying the binary constructs, generated as described in Example 6 above, were cultured in LB medium supplemented with kanamycin (50 mg/L) and gentamycin (50 mg/L). The cultures were incubated at 28°C for 48 hrs under vigorous shaking and then centrifuged at 4000 rpm for 5 minutes. The pellets comprising *Agrobacterium* cells were re-suspended in a transformation medium containing half-strength (2.15 g/L) Murashig-Skoog (Duchefa); 0.044 µM benzylamino purine (Sigma); 112 µg/L B5 Gambourg vitamins (Sigma); 5% sucrose; and 0.2 ml/L Silwet L-77 (OSI Specialists, CT) in double-distilled water, at pH of 5.7.

Transformation of T₀ plants was effected by inverting each plant into an *Agrobacterium* suspension, such that the above ground plant tissue was submerged for 3-5 seconds. Each inoculated T₀ plant was immediately placed in a plastic tray, then covered with clear plastic dome to maintain humidity and was kept in the dark at room temperature for 18 hrs, to facilitate infection and transformation. Transformed (transgenic) plants were then uncovered and transferred to a greenhouse for recovery and maturation. The transgenic T₀ plants were grown in the greenhouse for 3-5 weeks until siliques were brown and dry. Seeds were harvested from plants and kept at room temperature until sowing.

For generating T₁ and T₂ transgenic plants harboring the genes, seeds collected from transgenic T₀ plants were surface-sterilized by soaking in 70% ethanol for 1 minute, followed by soaking in 5% sodium hypochloride and 0.05% triton for 5 minutes. The surface-sterilized seeds were thoroughly washed in sterile distilled water then placed on culture plates containing half-strength Murashig-Skoog (Duchefa); 2% sucrose; 0.8% plant agar; 50 mM kanamycin; and 200 mM carbenicylin (Duchefa). The culture plates were incubated at 4°C for 48 hours then transferred to a growth room at 25°C for an additional week of incubation. Vital T₁ *Arabidopsis* plants were transferred to a fresh culture plates for another week of incubation. Following incubation the T₁ plants were removed from culture plates and planted in growth mix contained in 250 ml pots. The transgenic plants were allowed to grow in a greenhouse to maturity. Seeds harvested from T₁ plants were cultured and grown to maturity as T₂ plants under the same conditions as used for culturing and growing the T₁ plants.

### EXAMPLE 8

### Evaluating growth of transgenic plants cultivated under abiotic stress conditions Methods:

T₁ or T₂ transgenic plants generated as described above were individually transplanted into pots containing a growth mixture of peat and vermiculite (volume ratio 3:2, respectively). The pots were covered for 24 hr period for hardening, then placed in the greenhouse in complete random order and irrigated with tap water (provided from the pots' bottom every 3-5 days) for seven days. Thereafter, half of the plants were irrigated with a salt solution (100 mM NaCl and 5 mM CaCl₂) to induce salinity stress (stress conditions). The other half of the plants were continued to be irrigated with tap water (normal conditions). All plants were grown in the greenhouse at 100% RH for 28 days, then harvested (the above ground tissue) and weighted (immediately or following drying in oven at 50°C for 24 hr).

### Results:

No significant differences in plant fresh weights were observed between T₁ plants transformed with 3 different ABST genes and plants transformed with the Luciferase reporter gene, grown either under normal or stress conditions (Figure 3 and Table 3 below). Yet, T₁ plants transformed with SEQ DI NO: 1 positioned under the regulatory control of the At6669 promoter maintained 71 % of their fresh weight when exposed to stress conditions, while the control plants (carrying Luciferase gene positioned under the regulatory control of the AT6669 promoter) maintained only 61% of their fresh weight under similar stress conditions.

**Table 3 Fresh weight of T₁ transgenic Arabidopsis plants irrigated with water or salt solution**

| **Transgene (SEQ ID NO)** | **Promoter** | **N Rows**¹ | **Irrigation solution (mM NaCl)** | **Mean (g)** | **Std Error** |
|---|---|---|---|---|---|
| Luciferase | At6669 | 2 | 0 | 0.7925 | 0.0275 |
| Luciferase | At6669 | 2 | 100 | 0.485 | 0.045 |
| 13 | At6669 | 8 | 0 | 0.81625 | 0.020305 |
| 13 | At6669 | 8 | 100 | 0.4725 | 0.029246 |
| 1 | At6669 | 8 | 0 | 0.7875 | 0.026032 |
| 1 | At6669 | 8 | 100 | 0.55875 | 0.044699 |
| 8 | At6669 | 8 | 0 | 0.8575 | 0.023088 |
| 8 | At6669 | 8 | 100 | 0.440625 | 0.011198 |

| | | | | | |
|---|---|---|---|---|---|
| ¹N Rows represent number of independent transformation event plants measured. For each transgene, 3-5 independent transformation events with 1-3 plants per a single transformation event were used. | | | | | |

T₂ plants transformed with SEQ ID NOs: 7 or 14 positioned under the regulatory control of the 35S promoter accumulated significantly higher biomass than control plants, regardless of growth conditions. As shown in Figure 4A and Table 4 below, the mean fresh weight of plants transformed with SEQ ID NOs: 7 and 14, grown under stress conditions, were 15% and 24%, respectively, higher than the mean fresh weight control plants grown under similar stress conditions. Similarly, the mean fresh weight of plants transformed with SEQ ID NOs: 7 or 14, grown under normal conditions, were 21% and 27%, respectively, higher than the mean fresh weight control plants grown under similar normal conditions.

Similar phenomenon was observed with T₂ plants transformed with SEQ ID NO: 4 positioned under the regulatory control of the 35S promoter. Accordingly, as shown in Figure 4A and Table 4 below, the mean fresh weight of plants transformed with SEQ ID NO: 4 was 14% and 7% was higher than the mean fresh weight of control plants grown under stress and normal conditions, respectively. Similarly, T₂ plants transformed with SEQ ID NO: 4 positioned under the regulatory control of the At6669 promoter exhibited 1.3 and 5% higher biomass than control plants grown under stress and normal conditions, respectively. However, these differences were not found statistically different under the experimental conditions.

**Table 4 Fresh weight of T₂ transgeic Arabidopsis plants irrigated with water or salt solution**

| **Transgene (SEQ ID NO)** | **Promoter** | **N Rows** | **Irrigation solution (mM NaCl)** | **Mean (g)** | **Std Error** |
|---|---|---|---|---|---|
| Luciferase | CaMV-35S | 11 | 0 | 0.352727 | 0.011208 |
| Luciferase | CaMV-35S | 11 | 100 | 0.280909 | 0.010484 |
| 9 | CaMV-35S | 11 | 0 | 0.426364 | 0.019599 |
| 9 | CaMV-35S | 11 | 100 | 0.322727 | 0.027306 |
| 12 | CaMV-35S | 11 | 0 | 0.374545 | 0.015746 |
| 12 | CaMV-35S | 11 | 100 | 0.249091 | 0.020647 |
| 1 | CaMV-35S | 8 | 0 | 0.36625 | 0.034171 |
| 1 | CaMV-35S | 8 | 100 | 0.265 | 0.031225 |
| 13 | CaMV-35S | 11 | 0 | 0.349091 | 0.013515 |
| 13 | CaMV-35S | 11 | 100 | 0.293636 | 0.019921 |
| 14 | CaMV-35S | 11 | 0 | 0.446364 | 0.025558 |
| 14 | CaMV-35S | 11 | 100 | 0.348182 | 0.023772 |
| 8 | C1aMV-35S | 11 | 0 | 0.310909 | 0.015223 |
| 8 | CaMV-35S | 11 | 100 | 0.253636 | 0.01539 |
| 4 | CaMV-35S | 11 | 0 | 0.379091 | 0.010992 |
| 4 | CaMV-35S | 11 | 100 | 0.318182 | 0.013336 |

| | | | | | |
|---|---|---|---|---|---|
| ¹N Rows represent number of independent transformation event plants measured. For each transgene, 3-5 independent transformation events with 1-3 plants per a single transformation event were used. | | | | | |

T₂ plants transformed with SEQ ID NOs: 1 and 13 positioned under the regulatory control of the At6669 promoter and grown under stress conditions, exhibited significantly higher biomass than control plants grown under similar stress conditions. The mean fresh weight of T₂ plants transformed with SEQ ID NOs: 1 and 13 positioned under the regulatory control of the At6669 promoter, and grown under stress conditions, were 37% and 21%, respectively, higher than the mean fresh weight control plants grown under similar stress conditions (Figure 4B and Table 5 below). No significant increase in biomass over control was observed when these transgenic plants (carrying SEQ ID NOs: 1 and 13 regulated under At6669 promoter) where grown under normal conditions.

**Table 5 Fresh weight of T₂ transgenic Arabidopsis Plants irrigated with water or salt solution**

| **Transgene (SEQ ID NO** | **Promoter** | **N Rows** | **Irrigation solution (mM NaCl)** | **Mean (g)** | **Std Error** |
|---|---|---|---|---|---|
| Luciferase | At6669 | 6 | 0 | 0.3 | 0.010328 |
| Luciferase | At6669 | 6 | 100 | 0.125 | 0.009916 |
| 13 | At6669 | 6 | 0 | 0.286667 | 0.024449 |
| 13 | At6669 | 6 | 100 | 0.151667 | 0.007032 |
| 1 | At6669 | 6 | 0 | 0.305 | 0.03423 |
| 1 | At6669 | 6 | 100 | 0.171667 | 0.012225 |
| 4 | At6669 | 6 | 0 | 0.315 | 0.049983 |
| 4 | At6669 | 6 | 100 | 0.126667 | 0.005578 |
| 12 | At6669 | 6 | 0 | 0.263333 | 0.012824 |
| 12 | At6669 | 6 | 100 | 0.098333 | 0.007923 |
| 8 | At6669 | 6 | 0 | 0.228333 | 0.020235 |
| 8 | At6669 | 6 | 100 | 0.121667 | 0.004014 |

| | | | | | |
|---|---|---|---|---|---|
| ¹N Rows represent number of independent transformation event plants measured. For each transgene, 3-5 independent transformation events with 1-3 plants per a single transformation event were used. | | | | | |

The results illustrate that the isolated putative ABST genes, set forth in SEQ ID NOs: 1 and 13, are capable of increasing plant tolerance to abiotic stress, such as a salinity stress. In addition, the isolated putative ABST genes set forth in SEQ ID NOs: 7, 14 (and possibly also 4), are capable of substantially promoting biomass in plants grown under stress, as well as under normal conditions.

Hence, the results clearly indicate that the putative abiotic stress tolerance genes described herein can be readily isolated and utilized to substantially increase tolerance to abiotic stress and/or biomass in plants.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents, patent applications and sequences identified by their accession numbers mentioned in this specification are herein incorporated in their entirety by reference into the specification, to the same extent as if each individual publication, patent or patent application or sequence identified by their accession number was specifically and individually indicated to be incorporated herein by reference. In addition, citation or identification of any reference in this application shall not be construed as an admission that such reference is available as prior art to the present invention.

### REFERENCES CITED

### (Additional references are cited hereinabove)

1. www.fao.org/ag/ag1/ag11/spush/degrad.htm.
2. www.fao.org/ag/agl/aglw/watermanagement/introduc.stm
3. McCue KF, Hanson AD (1990).Drought and salt tolerance: towards understanding and application. Trends Biotechnol 8: 358-362.
4. Flowers TJ, Yeo Ar (1995). Breeding for salinity resistance in crop plants: where next? Aust J Plant Physiol 22:875-884.
5. Nguyen BD, Brar DS, Bui BC, Nguyen TV, Pham LN, Nguyen HT (2003). Identification and mapping of the QTL for aluminum tolerance introgressed from the new source, ORYZA RUFIPOGON Griff., into indica rice ( Oryza sativa L.). Theor Appl Genet. 106:583-93.
6. Sanchez AC, Subudhi PK, Rosenow DT, Nguyen HT (2002). Mapping QTLs associated with drought resistance in sorghum (Sorghum bicolor L. Moench). Plant Mol Biol. 48:713-26.
7. Quesada V, Garcia-Martinez S, Piqueras P, Ponce MR, Micol JL (2002). Genetic architecture of NaCl tolerance in Arabidopsis. Plant Physiol. 130:951-963.
8. Apse MP, Blumwald E (2002). Engineering salt tolerance in plants. Curr Opin Biotechnol. 13:146-150.
9. Rontein D, Basset G, Hanson AD (2002). Metabolic engineering of osmoprotectant accumulation in plants. Metab Eng 4:49-56
10. Clough SJ, Bent AF (1998). Floral dip: a simplified method for Agrobacterium-mediated transformation of Arabidopsis thaliana. Plant J 16:735-43.
11. Desfeux C, Clough SJ, Bent AF (2000). Female reproductive tissues are the primary target of Agrobacterium-mediated transformation by the Arabidopsis floral-dip method. Plant Physiol 123:895-904.

## Claims

1. A method of increasing biomass and/or abiotic stress tolerance of a plant, comprising expressing within the plant an exogenous polynucleotide at least 80% homologous to the polynucleotide set forth in SEQ ID NO: 13, thereby increasing the biomass and/or the abiotic stress tolerance of the plant.

2. A nucleic acid construct, comprising a polynucleotide at least 80% homologous to the nucleotide sequence set forth in SEQ ID NO: 13, and a plant promoter capable of directing transcription of said polynucleotide in a plant cell.

3. A plant cell comprising the nucleic acid construct of claim 2.

4. A transgenic plant comprising the nucleic acid construct of claim 2 or the plant cell of claim 3.

5. The method of claim 1, wherein said expressing is effected by:
(a) transforming a cell of said plant with said exogenous polynucleotide;
(b) generating a mature plant from said cell; and
(c) cultivating said mature plant under conditions suitable for expressing said exogenous polynucleotide within said mature plant.

6. The method of claim 5, wherein said transforming is effected by introducing to said plant cell a nucleic acid construct including said exogenous polynucleotide and at least one promoter capable of directing transcription of said exogenous polynucleotide in said plant cell.

7. The nucleic acid construct of claim 2, the plant cell of claim 3, or the transgenic plant of claim 4, wherein said promoter is a constitutive promoter.

8. The plant cell or the transgenic plant of claim 7, wherein said constitutive promoter is the At6669 promoter.

9. The nucleic acid construct of claim 2, the plant cell of claim 3, or the transgenic plant of claim 4, wherein said promoter is an inducible promoter.

10. The nucleic acid construct, the plant cell, or the transgenic plant of claim 9, wherein said inducible promoter is an abiotic stress inducible promoter.

11. The method of claim 1, wherein said expressing is effected by infecting said plant with a virus including said exogenous polynucleotide.

12. The method of claim 11, wherein said virus is an avirulent virus.

13. The method of claim 1, further comprising growing the plant under the abiotic stress.

14. The method of claim 1 or 13, wherein said abiotic stress is selected from the group consisting of salinity, water deprivation, low temperature, high temperature, heavy metal toxicity, anaerobiosis, nutrient deficiency, nutrient excess, atmospheric pollution and UV irradiation.

15. The method of claim 1, the nucleic acid construct of claim 2, the plant cell of claim 3, or the transgenic plant of claim 4, wherein said plant is a dicotyledonous plant.

16. The method of claim 1, the nucleic acid construct of claim 2, the plant cell of claim 3, or the transgenic plant of claim 4, wherein said plant is a monocotyledonous plant.


SEQUENCE LISTING

<110> Ronen, Gil
      Golan, Ezekiel
      Karchi, Hagai
      Meissner, Rafael

<120> METHODS OF INCLEASING ABIOTIC STRESS TOLERANCE AND/OR BIOMASS IN
      PLANTS

<130> 27910

<160> 92

<170> PatentIn version 3.2

<210> 1
<211> 456
<212> DNA
<213> Lycopersicon esculentum

<400> 1
atgggtcgta tgcacagtcg tggtaagggt atttcagctt ctgctctccc ttacaagaga 60

actcctccta gttggctcaa gatctctgct ccagatgttg aggacaacat ctgcaagttc 120

gctaagaaag gattgacccc ttcacagatt ggtgtgattc ttcgtgattc tcatggaatt 180

gcacaagtga agagtgttac tggtagcaag atcttgcgta tcctcaaggc acatgggctt 240

gcacctgaga ttccagagga tttgtaccac ctgattaaga aggctgttgc cattaggaag 300

catttggaga ggaacaggaa ggataaggat tctaagttcc gtttgatttt ggtggagagc 360

aggattcatc gccttgctcg ttattacaag aaaacaaaaa agctcccacc tgtctggaaa 420

tacgaatcta ccactgctag cacacttgtg gcatag 456

<210> 2
<211> 1386
<212> DNA
<213> Lycopersicon esculentum

<400> 2
atggctattc ctcttcaatt ctctagtata tccactcgca cagatctctc cttgccggag 60

actagaactt tcaggttacc gaaacctttc tccgtcatca gatgctccgc cggcgaacct 120

gttccttcct cgtcggctac tgctgagtca gagttcgatg ctaaggtttt ccggaagaat 180

ctgaccagaa gtgctaatta caatcgtaaa ggttttggac acaaagaagc tactcttgaa 240

ctcatgaatc gcgaatatac cagtgatatc atcaagaaat tgaaggagaa tgaatttgag 300

tatacatggg gaaacgtaac cgtaaaactt gcagagtcct atggtttctg ttggggggtt 360

gagcgtgcag ttcagattgc ttatgaagcg aggaaacagt ttccaacaga gaggatttgg 420

ataactaatg aaattattca caaccccact gtgaataaga ggctagagga tatggatgtt 480

aagaacattc cacttgagga agggaagaaa aactttgatg ttgttgacaa ggatgatgtt 540

gtggttttgc ctgcttttgg ggctgctgtt gatgaaatgt tggttttgag tgataaaaac 600

gtacaaattg ttgatacaac ctgcccgtgg gtgactaagg tttggaacac ggttgaaaag 660

cacaagaagg gagaatatac ctccattatc catggtaaat atgctcatga ggaaactgtt 720

gcgactgcat cctttgctgg gaaatacatc attgtgaaga acatggcaga ggcaacttat 780

gtctgtgatt atattcttgg aggtaaactt gatggttcta gctcaaccaa agaggcattt 840

atgcagaaat ttaaatatgc agtttctgaa gggtttgatc cggatgttga ccttgtaaaa 900

gctggtattg caaaccaaac aactatgttg aagggagaaa cagaagatat tgggaagttg 960

gtcgagagga ccatgatgca aaaatatggg gtggaaaatg ttaacaacca cttcgtaagt 1020

ttcaacacta tatgcgatgc cacacaagag cgtcaagatg caatgtataa gctggttgag 1080

caaaagctgg atcttatgtt agtgattggt ggctggaact caagtaacac ttcacatcta 1140

caggagattg cagaggaacg tggaattccc tcatactgga ttgacagtga acagagagta 1200

ggtcctggaa acaaaataag ttacaagtta atgcatggtg agttggttga gaaagagaac 1260

ttcttaccgg agggtcctat tacagttggg gtgacatctg gtgcatccac ccccgataag 1320

gttgttgaag atgtccttat caaggtgttt gatatcaagc gcgaggaagc cttacaattg 1380

gcctaa 1386

<210> 3
<211> 2418
<212> DNA
<213> Lycopersicon esculentum

<400> 3
atggctcaac gtgttctaac tcgtgttcac agtcttcgtg aacgtcttga tgctactttg 60

gatgctcatc gcaatgaaat tttgctcttt ctttcaagga tcgaaagcca cgggaaaggg 120

atcttgaaac ctcaccagct actggctgag tttgaatcaa ttcagaaaga agacaaagac 180

aaactgaatg atcatgcctt tgaagaagtc ctgaaatcca ctcaggaagc aattgttttg 240

cccccatggg ttgcacttgc tattcgtttg aggcccggtg tgtgggaata tgtccgtgtg 300

aatgttaatg ctcttagtgt tgaggagctg actgtgcctg agtttttgca attcaaggaa 360

gaacttgtta acggaacttc cagtgataac tttgttcttg aattggattt tgagcccttc 420

actgcatcat ttccaaaacc aaccctcacg aaatcaattg gaaatggagt tgaattcctc 480

aacaggcacc tctctgctaa aatgttccat gacaaggaaa gcatgacccc tcttctcgag 540

tttcttcgag ttcaccacta caatggaaag tcaatgatgc tgaatgatag aattcagaat 600

ttgtatactc tccaaaaagt cctgaggaag gccgaggaat acctcaccac cctttcgcca 660

gaaacttcat actcctcatt tgagcacaag ttccaagaaa ttggcttgga gagaggttgg 720

ggtgacaccg cagagcgtgt tctagagatg atctgcatgc tcctggatct ccttgaggct 780

cctgactcat gtactcttga gaagttcctt agtagaattc ctatggtttt caatgtagtt 840

atactttcac ctcatggata tttcgcccag gaaaatgtct tgggttaccc cgacactggt 900

ggtcaggttg tctatatttt ggatcaagtt cctgccttgg agcgtgagat gctcaagcgc 960

ataaaggagc aaggacttga tatcaaaccg cgtattctta ttgttactcg gcttctccct 1020

gatgcagttg gtaccacttg tggtcagcga ctcgagaagg tatttggaac tgagcattca 1080

catattctta gggtcccctt taggactgaa aagggcattg ttcgcaaatg gatctctcgt 1140

tttgaagtct ggccatacat ggagactttc attgaggatg tggggaaaga aataaccgca 1200

gaactgcaag ctaagccaga tcttattatt ggaaactata gtgagggaaa ccttgcagcc 1260

tccttgttgg ctcacaagtt aggtgtaaca cagtgcacca ttgctcatgc attggagaaa 1320

accaaatatc ctgattctga catttacttg aacaaatttg acgagaaata ccacttctca 1380

gctcagttca cagctgatct tatagcaatg aatcatactg atttcattat caccagcacc 1440

ttccaggaga tagcaggaag caaggacact gttggacagt atgagagcca catggccttc 1500

acaatgcctg gattgtatag agttgttcat ggcattgatg tgttcgaccc caaattcaac 1560

attgtgtcac caggagctga tgtgaatctc tatttcccat actccgaaaa ggaaaagaga 1620

ttgacaactt ttcaccctga aattgaagac ttgctgttta gcgatgttga gaacgaagaa 1680

cacctgtgtg tgttgaagga caggaataag cccatcatat tcaccatggc aagattggac 1740

cgagtgaaga acttaactgg acttgtcgag tggtatgcta agaatccacg actaagggag 1800

ttggttaacc ttgtagtggt tggtggagac cgaagaaagg aatccaaaga cttggaagag 1860

caggcagaga tgaagaagat gtatgaactt ataaagactc acaatttgaa tggccagttc 1920

cgatggattt cttcccagat gaaccgcgtg aggaatgggg aactctacag gtacattgct 1980

gacacaaggg gagctttcgt gcagcctgca ttctacgagg ctttcggtct gactgttgtt 2040

gaggccatga gctgcggttt gcctacattt gcaactaatc aaggtggtcc agctgagatc 2100

atcgttcatg gaaagtctgg tttccaaatt gatccatacc atggcgagca ggctgctgat 2160

ctcctcgctg agttcttcga gaaatgtaag gtagaccctt cacattggga agccatttcc 2220

aagggtggcc ttaagcgtat acaggagaag tacacatggc aaatctactc cgaccggctg 2280

ttgacactag ctgctgttta cgggttctgg aagcacgttt ccaagcttga tcgtcttgaa 2340

attcgtcgtt atcttgagat gttttacgct ctcaaattcc gcaagctggc tgaacttgtc 2400

ccattggctg ttgagtaa 2418

<210> 4
<211> 522
<212> DNA
<213> Lycopersicon esculentum

<400> 4
atggaagaca aaagcaatga ttattatgca gttttggggt tgaagaagga atgcactgac 60

acagaactta ggaatgccta taagaagctt gcactgaaat ggcacccaga tcgctgttca 120

gcatcgggga atttgaagtt tgtagatgaa gcaaagaagc aatttcaggc aattcaagaa 180

gcatattctg tgttatcgga tgcaaacaaa aagtttttgt acgatgtagg agtttatgac 240

tctggtgatg atgacgacga aaatggcatg ggtgatttcc tgaatgaaat ggcagctatg 300

atgagccaaa ataagtccaa tgaaaatcag ggagaagaaa cctttgagga attgcaggat 360

atgtttaatg aaatgttcaa cagtgataat ggaacgtttt cttcttcttc ttcttcttct 420

tcttcttctt ggactggaac tccttcaatg tgctctacta catcatctac atcttcaagt 480

gagacttttt taacctttcc ccaacaagag aagttcaggt ga 522

<210> 5
<211> 1434
<212> DNA
<213> Lycopersicon esculentum

<400> 5
atggctactg ctactactct ctctcctgct gatgctgaaa agctcaacaa cctcaaatct 60

gccgtcgccg gtctaaatca aatcagtgaa aatgagaaat ctggatttat taaccttgtc 120

ggtcgctatc taagtggtga agcacaacac attgactgga gtaagatcca gacgccaact 180

gatgaagttg tggtgcctta tgacaagtta gcacctcttt ctgaagatcc cgcggaaact 240

aagaagcttt tggacaaact tgttgtcctg aagctcaatg gaggcttggg aacaacaatg 300

ggatgcacgg gtcccaaatc agttattgaa gttcgtaatg gtttgacatt ccttgacttg 360

attgtcaagc aaattgaggc actcaatgcc aagttcggat gcagtgttcc cctgcttttg 420

atgaattcgt tcaacaccca cgatgataca ctgaagattg ttgaaaaata tgcaaactca 480

aacattgata ttcatacatt caatcagagc cagtaccctc gcctggttac tgaagacttt 540

gccccacttc catgcaaagg caattccgga aaagatggat ggtaccctcc aggtcatggt 600

gatgttttcc cttctttgat gaatagtgga aagcttgatg cactactagc aaagggcaag 660

gaatatgtct ttgttgcaaa ctctgataat ttgggcgcca ttgttgattt gaaaatctta 720

aatcatttga tcctaaacaa aaatgagtac tgcatggagg ttactcccaa aactttagct 780

gatgtcaaag gtggcacctt aatctcatat gaaggaaaag tacagctatt ggaaatagca 840

caagtccctg atgaacatgt caatgaattc aagtcaattg aaaaattcaa aattttcaac 900

accaacaact tgtgggtgag tcttagtgct attaaaagac ttgtagaagc agatgcactc 960

aagatggaga ttattcccaa cccaaaggaa gtagacggag ttaaagttct tcaacttgaa 1020

actgctgccg gtgctgcgat taagtttttc gaccgggcaa ttggtgctaa tgttcctcga 1080

tctcgtttcc ttcccgtgaa agcaacttca gatttgctcc ttgttcagtc tgatctttac 1140

accttgactg atgagggcta tgtcatccga aacccggcca ggtctaatcc gtccaaccca 1200

tccatcgagt taggacctga attcaagaag gtggccaact tcttaggccg tttcaagtcc 1260

attcccagca tcattgatct aggtagcttg aaggtgaccg gtgatgtatg gtttggatct 1320

agcgttaccc taaaggggaa agtgactgtt gcagccaaat ccggagtgaa gctagaaatt 1380

ccagatggtg ctgtgattgc aaacaaggac atcaatggac ctgaggatat atag 1434

<210> 6
<211> 1485
<212> DNA
<213> Lycopersicon esculentum

<400> 6
atggcaaaga gtggcatttt ggtaattgtt tcagctcttg ttgttcttgc agtttgtggt 60

gtttttgctg aggagaacga atatgtgttg actttggacc attctaacct cactgagact 120

gttgctaagc acaacttcat tgttgttgaa ttctatgcac cttggtgtgg acactgtaag 180

agtcttgctc ctgagtatga aaaagctgcc tcagagctga gtagtcatga ccctccaatt 240

gttctagcta agtatgatgc aaatgatgaa gccaatagag aactttcaaa acagtacgag 300

atccagggtt tcccaactat taagatattg agagatggag gaaagaaagt tcaagactat 360

aacggtcctc gtgaagcagc tggtattgta tcctacttga agaaacaagt gggtcctgca 420

tctgctgaaa tcaagtcgaa ggaagatgcc acaaacctta ttgatgagaa aagtatcttt 480

gttgttggta tatttccaga cccctccgga gagaaattcg agaactattt aacgctagct 540

gaaaaactgc gaggcgagtt cgattttgct cacactgttg atgctaaaca cctccctcgg 600

ggtggaccag tcaacaagcc cactcttcgt cttctaaagc catttgatga actctttgtt 660

gattttgagg actttgatgt cgatgcaatg gagaagttca tctcagaatc tagtattcct 720

gttgttacta tttttgacaa tgacccaaac aaccatcctt atgttaacaa gttcttcgaa 780

ggcaccaacg ccaaggcatt gctatttgtg aactttagct ctgaatttga tgcttttaag 840

tccaagtaca acgatgttgc tgtgatttac aaaggggatg gggtgagctt tctcttgggt 900

gatgttgagg ctggtcaagg tgcttttgag tacttcggac tgaagccgga acaggcacct 960

gtgatcatca taatggacgc tgatgaacaa aagtatatta aggaccatgt ggaacctgat 1020

gccattgctg cttacttgaa ggattacaag gaaggaaaac tgaagccaca tgtgaagtca 1080

gagcccatcc ctgaagtcaa tgacgaacct gttaaggtgg ttgttaggga taccctccag 1140

gatatggttt acaaatcggg aaaaaatgtg ctgttagagt tctatgcacc ttggtgtggc 1200

cactgcaaga gtctggctcc aattttggat gaagtggctg tatcatttga aagcgatcct 1260

gatgttctca ttgcaaaact ggacgcaacc gcaaatgatc tcccgaaagg tgactttgat 1320

gttcagggat tccctactat gtacttcaga tccgcctctg gtaacttgtc acagtacaat 1380

ggtgagagaa caaaagaggc tatcatcgaa ttcatcgaga agaatcgtgg caagcctgct 1440

cagtcagact ctgccaaagt cgattcagca aaggatgaac tttag 1485

<210> 7
<211> 250
<212> DNA
<213> Lycopersicon esculentum

<400> 7
   atgccgggtg cgctcgcatg gcaggtcaca aaacctaata aagatgcgat ttttgtgttc 60
   ggaggggaga tggtacgggg ttttttgccc gactctcctc tctgtgtctg tctttggcct 120
   cctttctttt ctagaactgt ctgcaagatc tattcagata aacgtctcat caaatttggc 180
   gttaatgtcc ccgaacgatg gtttcgatat ccgcaaagtt atagcagcgc tctacctttt 240
   caatggatag 250

<210> 8
<211> 717
<212> DNA
<213> Lycopersicon esculentum

<400> 8
atgaattctc agatttgcag atctgctaca agagcagcta agtcactcct ttctgcttca 60

tctaagcaga cttctcgtgc tttttcagga ggacgagcag cagctgcagc agccacagtt 120

tcattgagag gagtggtgcc ttctctagcc tcatatggca ggaatgaatc tggaaatgca 180

tctagagctt ggatttctgg tgtgcttgcc cttcctgcag cagcttacat gctccaggag 240

caagaagcac atgctgccga gatggagcgc acctttattg ccatcaagcc agatggagta 300

cagagaggcc tgatttcaga aatcgtatca cggtttgagc gcaagggctt caagctggtt 360

gcaatcaaag ttgtgattcc ttccaaggaa tttgcaaaga agcactatca tgacttgagt 420

gagcgaccat tctttaacgg cttgtgcgac ttccttagct ctggccctgt cttagcaatg 480

gtttgggaag gtgaaggtgt aatcagatat ggaaggaagc ttatcggagc caccgatcca 540

cagaaatctg aacctggaac catcagaggc gatttagctg ttgtagtagg aaggaacatc 600

atccatggca gcgatggccc cgagaccgca aaggatgaga tcaacctatg gtttaaacca 660

gaggagttgg ttaattacac cagcaactct gagaagtggc tatatggtga taactaa 717

<210> 9
<211> 897
<212> DNA
<213> Lycopersicon esculentum

<400> 9
atggagaata tgcagagcta ttggcaattt ggcgacgagc ttcgaggaca atcaaaagcc 60

tcagaggatc ataaatggtc aacagctgct ataaaattat ctgaacagat gaagtacaaa 120

ggtgaacgta ggaataacct tgacctttca aagagctctg ctgaaattag gcccaggggt 180

aatcatatgt ttcaggaaga taacaagtgg gaaagcctta acttcaatat gttaaatttg 240

gaaagcaaga tgactgaaaa tatgagcaag aatcgcatta tggatagcat tttcaatgca 300

aatccagttt atcttaagcc caattttaac agcttgggaa attcatcttt aagcaagttc 360

aatgctagca actataccaa ggaacctagc aagaataaca ataacaacgt tgagagcaca 420

aatggaaata actccgttga caaaaggttt aagactctgc ctgctgctga aacactgccg 480

aagaatgagg ttcttggtgg atatatattt gtttgtaaca atgacacaat gcaggaagac 540

ctaaagcgcc tgctctttgg ccttcctcct agatacagag attccgtgag ggcaataaca 600

ccagggttgc ccttgttcct atataattac actactcacc agttgcatgg tatctttgag 660

gcatcgagtt ttggaggttc caacattgat ccaactgcct gggaggataa aaagtgtaaa 720

ggagagtcaa ggttccctgc tcaggtgagg atccgtgtcc ggaaagtctg taatcctttg 780

gaggaagatg ctttcagacc agttttacat cattatgatg gccccaagtt ccgtctggag 840

ctctccattc ctgagacttt ggacttacta gatctctgtg aaaaagccgg tgtgtag 897

<210> 10
<211> 489
<212> DNA
<213> Lycopersicon esculentum

<400> 10
atgtcacacc ccaccgcagc tccgctatca ggaccacgcc tctcacaacc ctcgtcgtcg 60

gcgatgtctc ccttgtacaa acagaaatct tggtcaccgg acacgtttcg cgacgaggcg 120

tggcagcggc ggaagggtac ccacggaagc tgcctcaaac ggcggagcaa gagcgttacc 180

gatgaggact ttgatgagat taaggcctgt atcgaattag ggtttggatt tgattcgcca 240

gaaatggatc agcgattgtc tgatactttt ccggcgtatg acctgtttta cgccgtgaat 300

aaacaataca ccgacactct ttcaaagact tcctctgtat catcggtcat ctccaattgc 360

gagtcaaccc ttcctcccgt cagtccccac accattgtct ttccaggaga taatccacag 420

gcagtgaaga caaggttgcg gcaatgggca caggtggttg cgtgtgtggt gcgtcaatct 480

tcgtattaa 489

<210> 11
<211> 489
<212> DNA
<213> Lycopersicon esculentum

<400> 11
atgtcacacc ccaccgcagc tccgctatca ggaccacgcc tctcacaacc ctcgtcgtcg 60

gcgatgtctc ccttgtacaa acagaaatct tggtcaccgg acacgtttcg cgacgaggcg 120

tggcagcggc ggaagggtac ccacggaagc tgcctcaaac ggcggagcaa gagcgttacc 180

gatgaggact ttgatgagat taaggcctgt atcgaattag ggtttggatt tgattcgcca 240

gaaatggatc agcgattgtc tgatactttt ccggcgtatg acctgtttta cgccgtgaat 300

aaacaataca ccgacactct ttcaaagact tcctctgtat catcggtcat ctccaattgc 360

gagtcaaccc ttcctcccgt cagtccccac accattgtct ttccaggaga taatccacag 420

gcagtgaaga caaggttgcg gcaatgggca caggtggttg cgtgtgtggt gcgtcaatct    480

tcgtattaa                                                            489

<210>  12
<211>  606
<212>  DNA
<213>  Lycopersicon esculentum

<400>  12
atgggatact ggaaagcaaa ggttcttcca aagatcaagc agatctttga taaaaatgga     60

cccaagaaaa ctgctgctgc tgaggcatgc aagacttttg atcaagctaa ggaggaatat    120

agcaaggagt ttgaagagaa gaagactgag cttcaaccca aagttgttga aatttatgaa    180

gctgctgcag ttgagatcaa gagcttagtg aaggaaccaa agggtgcagg gctgaagaaa    240

aactcagatg gggttcagaa attccttgat gaccttgtca agattgaatt tccgggatca    300

aaagctgtta gcgaagcaag ttcaaacttt gggccttcct atgtatcggg cccaattatt    360

tttgtgttcg aaaaagtttc cactttcata gtcacagaag ataagaagga agaggaaccc    420

gcggctgctg atgacgtgca tgcaccagcc gccacgtcaa ctgaagaggt ggaggtgaag    480

gtgaaggaga aggagaagga gatagttatt gagtctggag aaccaaacaa ggaagaagca    540

cctgctactg ttgttgctga cgtggcacct gcaactaagg tggaagaagc accaaaggtg    600

gtttaa                                                               606

<210>  13
<211>  750
<212>  DNA
<213>  Lycopersicon esculentum

<400>  13
atggctggcg gcgtagctat tggaagtttt agtgattcat tcagcgttgt ctctcttaag     60

tcctatcttg ccgaattcat ctccacactc atctttgtct tcgccggagt tggttccgcc    120

attgcttacg gcaagttgac aacaaatgct gcacttgatc cggctgggct tgtagctatt    180

gcagtttgcc atggatttgc tctattcgta gccgtttcga tttccgctaa catctccggt    240

ggtcatgtta accctgcggt cacctgtgga ttaaccttcg gcggacatat tacctttatc    300

actggctcct tctacatgct tgctcaactt accggcgccg ctgtagcttg cttcctcctc    360

aaattcgtca ccggaggatg tgctattcca acccatggag tgggagctgg tgtgagcata    420

ctagaaggac tcgtgatgga aataataatc acatttggtt tagtttatac tgtgttcgca    480

accgccgctg acccgaagaa gggttcattg ggcacaattg caccgattgc aattggtctc    540

attgttggag ctaatatttt ggctgccgga ccattctccg gtggatcaat gaacccagct    600

cgttcatttg gacctgcaat ggttagtggt aactttgagg gtttctggat ctactggatt    660

ggtccattag ttggtggtag tttggctggt cttatttaca caaatgtgtt catgacacaa    720

gaacatgctc ctttatccaa tgagttctaa                                     750

<210>  14
<211>  735
<212>  DNA
<213>  Lycopersicon esculentum

<400>  14
atggaggtcg attctagtgg gaatcctaat tggttatttg attatgagtt gatgacggat     60

attacttctg ctgcatctgt taccgtcgct gagtttcagt ctccggctac tattgatttc 120

agctggcctg ctcaaacgat ctatgcttct tctaatctca ttactgaaac agattacaca 180

tttgcggatt cagaagttag caaggaggca agctcacgaa agcggttaaa aagtgaatgt 240

tgcagctctc cgagatctaa ggcatgcaga gagaaattgc ggagggacag actgaatgag 300

aggttcctcg cattgagctc tgtccttgat cctggaaggc caccaaaaac tgagaaagtt 360

gcaattctaa gtgatgctca aaggatgctg attgagctgc gaactgaaac ccagaagctg 420

aaggagtcaa atgaggagct gcaagagaag ataaaagaac ttaaggcaga gaagaatgag 480

ctccgagatg aaaagcaaag gctaaaggaa gaaaaggata atttggagca gcaggttaaa 540

agcttagctt ctaaagcagg atttctctcc catccttctg ccatgggagc tgcatttact 600

gcacaaggac aagttgctgc aggcaacaaa ttgatgcctt tcattggtta tcccagygty 660

gcgatgtggc rattcatgca acctgctgtt gttgacacat ctcaagatca tgtgctccgt 720

cctccagttg cttaa 735

<210> 15
<211> 645
<212> DNA
<213> Lycopersicon esculentum

<400> 15
cccttcaaac ctaaaaaaaa gagaaaatca acaaaaaaaa tgggagcttg tgcaacgaag 60

ccaaaggatt tgaaaggaga tgcaccggaa accgcaccgg aaaatgttcc ggcgactgaa 120

atcgccacca aggatgcggc ggaagtagcc gtcgccgcca aggatgtggt ggttgtggcg 180

gaagtagaag tgaaaaagga aattgaggcc gatgctgctg ctgcagatga cgacgatgct 240

gaaaaacgcc gatctctatc taacttgttc aaagagaacg aagaatgtaa ggggtcagag 300

caagtaaacg aggaggcatc caagatcaca ccatcagaag ctaagccaga agaagttgag 360

aaggttgttg atgctcctgt aacttcagag atagaaaaag cactagaagt ggcctcaatt 420

actgctgagg ctcccaaggt ggagccttcc gaggagaaga agatagatga agtgaaatca 480

gaacctaaga ctcctgctga gaagaaagta gaggaagcaa aaccggcagt agagactcct 540

gcagagaaga aaatagaaga ggcaccagtt gctccaactc acgtggagac gaaagccgaa 600

gatgctccaa aggtaactgt agtagaagaa aagaagtcga gctag 645

<210> 16
<211> 630
<212> DNA
<213> Lycopersicon esculentum

<400> 16
cccttcaaac ctaaaaaaaa gagaaaatca acaaaaaaaa tgggagcttg tgcaacgaag 60

ccaaaggatt tgaaaggaga tgcaccggaa accgcaccgg aaaatgttcc ggcgactgaa 120

atcgccacca aggatgcggc ggaagtagcc gtcgccgcca aggatgtggt ggttgtggcg 180

gaagtagaag tgaaaaagga aattgaggcc gatgctgctg ctgcagatga cgacgatgct 240

gaaaaacgcc gatctctatc taacttgttc aaagagaacg aagaatgtaa ggggtcagag 300

caagtaaacg aggaggcatc caagatcaca ccatcagaag ctaagccaga agaagttgag 360

aaggttgttg atgctcctgt aacttcagag atagaaaaag cactagaagt ggcctcaatt 420

actgctgagg ctcccaaggt ggagccttcc gaggagaaga agatagatga agtgaaatca 480

gaacctaaga ctcctgctga gaagaaagta gaggaagcaa aaccggcagt agagactcct 540

gcagagaaga aaatagaaga ggcaccagtt gctccaactc acgtggagac gaaagccgaa 600

gattgcatgg tgaaaaacgc gtggaaatga 630

<210> 17
<211> 216
<212> DNA
<213> Lycopersicon esculentum

<400> 17
atggagataa catgtgcact tgtctgtgag atcttactag caatcttgct tcctcctctt 60

ggtgtttgcc ttcgcaatgg ttgctgcact gtggagttct tgatttgctt ggtattgacc 120

atattgggct atgttcctgg aattatctat gctctctatg caatcctctt tgttgaacgt 180

gaaccaagaa gggattatta tgacactctt gcttga 216

<210> 18
<211> 216
<212> DNA
<213> Lycopersicon esculentum

<400> 18
atggactcaa gatgtgcaat tctgtgtgaa gttttactag caatcttgct tcctcctctt 60

ggtgtttgcc ttcgcaatgg ttgctgcact gtggagttct tgatttgctt ggtattgacc 120

atattgggct atgttcctgg aattatctat gctctttatg caatcctctg tattcaaagt 180

gaaccacact accatcatta ccattctctt gcttga 216

<210> 19
<211> 877
<212> DNA
<213> Cauliflower mosaic virus

<400> 19
aagcttgcat gcctgcaggt ccccagatta gccttttcaa tttcagaaag aatgctaacc 60

cacagatggt tagagaggct tacgcagcag gtctcatcaa gacgatctac ccgagcaata 120

atctccagga aatcaaatac cttcccaaga aggttaaaga tgcagtcaaa agattcagga 180

ctaactgcat caagaacaca gagaaagata tatttctcaa gatcagaagt actattccag 240

tatggacgat tcaaggcttg cttcacaaac caaggcaagt aatagagatt ggagtctcta 300

aaaaggtagt tcccactgaa tcaaaggcca tggagtcaaa gattcaaata gaggacctaa 360

cagaactcgc cgtaaagact ggcgaacagt tcatacagag tctcttacga ctcaatgaca 420

agaagaaaat cttcgtcaac atggtggagc acgacacact tgtctactcc aaaaatatca 480

aagatacagt ctcagaagac caaagggcaa ttgagacttt tcaacaaagg gtaatatccg 540

gaaacctcct cggattccat tgcccagcta tctgtcactt tattgtgaag atagtggaaa 600

aggaaggtgg ctcctacaaa tgccatcatt gcgataaagg aaaggccatc gttgaagatg 660

cctctgccga cagtggtccc aaagatggac ccccacccac gaggagcatc gtggaaaaag 720

aagacgttcc aaccacgtct tcaaagcaag tggattgatg tgatatctcc actgacgtaa 780

gggatgacgc acaatcccac tatccttcgc aagacccttc ctctatataa ggaagttcat 840

ttcatttgga gagaacacgg gggactctag aggatcc 877

<210> 20
<211> 2322
<212> DNA
<213> Arabidopsis thaliana

<400> 20
aagctttaag ctccaagccc acatctatgc acttcaacat atctttttct agatgagttg 60

gtaaaagtag aaaaagatat gatgatttta aatttgtttc tatttatatg tgttcatcga 120

aacttcattt tttttagttt taatagagag tttatatgac ttttaaaaat tgatttaaaa 180

ctgtgtcaaa aattaaaagg acaataaaaa atttgcatac aaccgaaaat acttatattt 240

agacaagaaa aaataatact tgtgatgctg attttatttt attatatatc atgaatcatg 300

atcatccaat tttccggata agccaaagtc aaaatgatgg gttcccccta atcttttatg 360

ctgagaaata gatgtatatt cttagatagt aatataaaat tgggttaaag aatgatgatt 420

cgattatagc ctcaactaga agatacgtgt agtgcaggtg tgtagttaac tggtggtagt 480

ggcagacaac cagattagga gttaaataaa gcctttagat ttgagagatt gaaatattcg 540

attggaacct ttctagattt ttacagccat ctaaaattag atgcagatca cctactacca 600

ttcaaaaatg aacaaaataa tttcatttac attttcctag cataagatat aataataaaa 660

tagtgctcat tttaattact ttttctaaat attttcgtta ttttaaattt tgcttgtcta 720

tactctacag ctcatttaat aacggaaaca aaaataattg cagggatacg gatgggtagc 780

tttcaaaact tacatcatct tctgtttctt gagatcaact atttttggag ctttgtctca 840

atcgtaccaa aggataatgg tcctacctcc ttttgcattc ttaactttat cttctctact 900

tatttctttt ttgggatttt tgggggtatt attttatctt ttgtagatat acacattgat 960

ttactacaaa cgtatactac tatccatctt caactcttcg gaatatgatt tcgaaaaaac 1020

tatgaagatt aacgggtatc ttaaacatgt taagatacac cggacaattt tcatttagaa 1080

gaattgatat gcaattaaca ataaatagtt gatgatcttt tagttttgaa gatgtgcgtt 1140

aagacttaag cgtgtggtaa caaggtggga ctcgggcaac gcaaagcctt gtagagtcca 1200

cttgctcaac ttgtctttct tttatctctt ttccaagtct caagattcaa tgaactccgt 1260

gtaacacaaa cacgcccata gatgagctca tttttggtat ttccaatatt gccactccat 1320

gataatatca tctagggatg gggttcattt attttgaaat ctcaacaaat ctcgtcgatt 1380

ctaacacaca tgattgattt gtttacttac ttgaaagttg gcaactatct gggattaaaa 1440

tttatctttt tctactgcta gctagaagca tctatatatg ttagcctaat acgtggaaga 1500

tgtcattgct aataatggct aaagatgtgt attaattttt cttctttttt ccttgaattt 1560

ttgttctttg acataaacta tgctgtcaaa atgtgtagaa tctttttaca taaatcattc 1620

cctgttacac actaaaaggt tcacaacgga cgattgtatt ggacttccag atcataaacc 1680

atgcaaaact gaaaaccaca agaataatta gttctaactt tagaacgttc gtacgtgttt 1740

catgttcaaa aagcgtcaat tataaaagtt gggaaattac ttttgagttt tgacatttct 1800

aaggacagtc aaatatgaca acattgggat gcaacttacc ttgtattaac ttattttgtt 1860

ataaaaccat atattacata ttttaaaggg ttgataaata atcaaatata ccaaaacata 1920

gcttttcaat atatttgtaa aacacgtttg gtctactagc taattatgag aacatttgtt 1980

caatgcatga ttatctagta tctactagtg gattatgaaa attagatatt ttcattgcat 2040

gattatcttc catatatagt gataacatca aaagaatcta caccaattat tgcatttttt 2100

cattatataa taagcactaa actgtaaaat tatattcagc cacccaaacc atgacaaatc    2160

accttaaagg cttaaacaca taacagccat tacgagtcac aggtaagggt ataatagtaa    2220

agaatcaatc tatataatat acgacccacc ctttctcatt ctttctggag agtaacatcg    2280

agacaaagaa gaaaaactaa aaaagagaac cccaaaggat cc                       2322


<210>  21
<211>  31
<212>  DNA
<213>  Artificial sequence

<220>
<223>  Single strand DNA oligonucleotide

<400>  21
gggaaggatc catgggtcgt atgcacagtc g                                     31


<210>  22
<211>  32
<212>  DNA
<213>  Artificial sequence

<220>
<223>  Single strand DNA oligonucleotide

<400>  22
cgacggagct cctatgccac aagtgtgcta gc                                    32


<210>  23
<211>  35
<212>  DNA
<213>  Artificial sequence

<220>
<223>  Single strand DNA oligonucleotide

<400>  23
gggaaggatc cggtggtatt gaagttatgg aagac                                 35


<210>  24
<211>  30
<212>  DNA
<213>  Artificial sequence

<220>
<223>  Single strand DNA oligonucleotide

<400>  24
gctgcgagct cctaatgctt ccgtccactc                                       30


<210>  25
<211>  34
<212>  DNA
<213>  Artificial sequence

<220>
<223>  Single strand DNA oligonucleotide

<400>  25
gggaaggatc catgaattct cagatttgca gatc                                  34


<210>  26
<211>  36
<212>  DNA
<213>  Artificial sequence

<220>
<223>  Single strand DNA oligonucleotide

<400>  26
gaggagagct cttagttatc accatatagc cacttc                                36


<210>  27
<211>  33
<212>  DNA
<213>  Artificial sequence

<220>
<223>  Single strand DNA oligonucleotide

<400>  27
gggaatctag acaacatgga gaatatgcag agc                                   33


<210>  28
<211>  31
<212>  DNA
<213>  Artificial sequence

<220>
<223>  Single strand DNA oligonucleotide

<400>  28
agaagcccgg gcacatagca cctacacacc g                                     31


<210>  29
<211>  33
<212>  DNA
<213>  Artificial sequence

<220>
<223>  Single strand DNA oligonucleotide

<400>  29
gggaaggatc cgatgggata ctggaaagca aag                                   33


<210>  30
<211>  32
<212>  DNA
<213>  Artificial sequence

<220>
<223>  Single strand DNA oligonucleotide

<400>  30
agccggagct cttaaaccac ctttggtgct tc                                    32


<210>  31
<211>  29
<212>  DNA
<213>  Artificial sequence

<220>
<223>  Single strand DNA oligonucleotide

<400>  31
gggaaggatc caaatggctg gcggcgtag                                        29


<210>  32
<211>  34
<212>  DNA
<213>  Artificial sequence

<220>
<223>  Single strand DNA oligonucleotide

<400>  32
agaaggagct ctagaactca ttggataaag gagc                                34

<210>  33
<211>  32
<212>  DNA
<213>  Artificial sequence

<220>
<223>  Single strand DNA oligonucleotide

<400>  33
gggaaggatc catggaggtc gattctagtg gg                                  32

<210>  34
<211>  32
<212>  DNA
<213>  Artificial sequence

<220>
<223>  Single strand DNA oligonucleotide

<400>  34
agaagcccgg gttaagcaac tggaggacgg ag                                  32

<210>  35
<211>  22
<212>  DNA
<213>  Artificial sequence

<220>
<223>  Single strand DNA oligonucleotide

<400>  35
ggtggctcct acaaatgcca tc                                             22

<210>  36
<211>  20
<212>  DNA
<213>  Artificial sequence

<220>
<223>  Single strand DNA oligonucleotide

<400>  36
aagttgggta acgccagggt                                                20

<210>  37
<211>  22
<212>  DNA
<213>  Artificial sequence

<220>
<223>  Single strand DNA oligonucleotide

<400>  37
tagtttggtc agatgggaaa cg                                             22

<210>  38
<211>  25
<212>  DNA
<213>  Artificial sequence

<220>
<223>  Single strand DNA oligonucleotide

<400>  38
aaatattgga tcctttgggg ttctc                                          25

<210>  39
<211>  151
<212>  PRT
<213>  Panax ginseng

<400>  39

Met Gly Arg Met His Ser Arg Gly Lys Gly Ile Ser Ala Ser Ala Leu
1               5                   10                  15

Pro Tyr Lys Arg Thr Pro Pro Ser Trp Leu Lys Ile Thr Pro Gln Asp
            20                  25                  30

Val Asp Asp Asn Ile Cys Lys Phe Ala Lys Lys Gly Leu Thr Pro Ser
        35                  40                  45

Gln Ile Gly Val Ile Leu Arg Asp Ser His Gly Ile Ala Gln Val Lys
    50                  55                  60

Ser Val Thr Gly Ser Lys Ile Leu Arg Ile Leu Lys Ala His Gly Leu
65                  70                  75                  80

Ala Pro Glu Ile Pro Glu Asp Leu Tyr His Leu Ile Lys Lys Ala Val
                85                  90                  95

Ala Ile Arg Lys His Leu Glu Arg Asn Arg Lys Asp Lys Asp Ser Lys
            100                 105                 110

Phe Arg Leu Ile Leu Val Glu Ser Arg Ile His Arg Leu Ala Arg Tyr
        115                 120                 125

Tyr Lys Lys Thr Lys Lys Leu Pro Pro Val Trp Lys Tyr Glu Ser Thr
    130                 135                 140

Thr Ala Ser Thr Leu Val Ala
145                 150

<210>  40
<211>  151
<212>  PRT
<213>  Glycine max

<400>  40

Met Gly Arg Met His Ser Arg Gly Lys Gly Ile Ser Ser Ser Ala Leu
1               5                   10                  15

Pro Tyr Lys Arg Thr Pro Pro Ser Trp Leu Lys Ile Ser Ser Gln Asp
            20                  25                  30

Val Glu Glu Asn Ile Cys Lys Phe Ala Lys Lys Gly Leu Thr Pro Ser
        35                  40                  45

Gln Ile Gly Val Ile Leu Arg Asp Ser His Gly Ile Ala Gln Val Asn
    50                  55                  60

Ser Val Thr Gly Ser Lys Ile Leu Arg Ile Leu Lys Ala His Gly Leu
65                  70                  75                  80

Ala Pro Glu Ile Pro Glu Asp Leu Tyr His Leu Ile Lys Lys Ala Val
                85                  90                  95

Ser Ile Arg Lys His Leu Glu Arg Asn Arg Lys Asp Lys Asp Ser Lys
            100                 105                 110

Phe Arg Leu Ile Leu Val Glu Ser Arg Ile His Arg Leu Ala Arg Tyr
        115                 120                 125

Tyr Lys Lys Thr Lys Lys Leu Pro Pro Val Trp Lys Tyr Glu Ser Thr
    130                 135                 140

Thr Ala Ser Thr Leu Val Ala
145                 150

<210>  41
<211>  151
<212>  PRT
<213>  Arabidopsis thaliana

<400>  41

<210>  42
<211>  151
<212>  PRT
<213>  Arabidopsis thaliana

<400>  42

Met Gly Arg Met His Ser Arg Gly Lys Gly Ile Ser Ala Ser Ala Leu

1               5                   10                  15

Pro Tyr Lys Arg Ser Ser Pro Ser Trp Leu Lys Thr Thr Pro Gln Asp
            20                  25                  30

Val Asp Glu Ser Ile Cys Lys Phe Ala Lys Lys Gly Leu Thr Pro Ser
        35                  40                  45

Gln Ile Gly Val Ile Leu Arg Asp Ser His Gly Ile Pro Gln Val Lys
    50                  55                  60

Ser Val Thr Gly Ser Lys Ile Leu Arg Ile Leu Lys Ala His Gly Leu
65                  70                  75                  80

Ala Pro Glu Ile Pro Glu Asp Leu Tyr His Leu Ile Lys Lys Ala Val
                85                  90                  95

Ala Ile Arg Lys His Leu Glu Arg Asn Arg Lys Asp Lys Asp Ser Lys
            100                 105                 110

Phe Arg Leu Ile Leu Val Glu Ser Arg Ile His Arg Leu Ala Arg Tyr
        115                 120                 125

Tyr Lys Lys Thr Lys Lys Leu Pro Pro Val Trp Lys Tyr Glu Ser Thr
    130                 135                 140

Thr Ala Ser Thr Leu Val Ala
145                 150

<210>  43
<211>  151
<212>  PRT
<213>  Arabidopsis thaliana

<400>  43

Met Gly Arg Met His Ser Arg Gly Lys Gly Ile Ser Ala Ser Ala Leu
1               5                   10                  15

Pro His Lys Arg Ser Ser Pro Ser Trp Leu Lys Thr Thr Pro Gln Asp
            20                  25                  30

Val Asp Glu Ser Ile Cys Lys Phe Ala Lys Lys Gly Leu Thr Pro Ser
        35                  40                  45

Gln Ile Gly Val Ile Leu Arg Asp Ser His Gly Ile Pro Gln Val Lys
    50                  55                  60

Ser Val Thr Gly Ser Lys Ile Leu Arg Ile Leu Lys Ala His Gly Leu
65                  70                  75                  80

Ala Pro Glu Ile Pro Glu Asp Leu Tyr His Leu Ile Lys Lys Ala Val
                85                  90                  95

Ala Ile Arg Lys His Leu Glu Arg Asn Arg Lys Asp Lys Asp Ser Lys
            100                 105                 110

Phe Arg Leu Ile Leu Val Glu Ser Arg Ile His Arg Leu Ala Arg Tyr

115                 120                 125

Tyr Lys Lys Thr Lys Lys Leu Pro Pro Val Trp Lys Tyr Glu Ser Thr
    130                 135                 140

Thr Ala Ser Thr Leu Val Ala
145                 150

<210>  44
<211>  151
<212>  PRT
<213>  Pisum sativum

<400>  44

Met Gly Arg Leu His Ser Lys Gly Lys Gly Ile Ser Ser Ser Ala Leu
1               5                   10                  15

Pro Tyr Arg Arg Thr Ala Pro Ser Trp Leu Lys Ile Ser Ser Gln Asp
            20                  25                  30

Val Asp Glu Thr Ile Cys Lys Phe Ala Lys Lys Gly Leu Thr Pro Ser
        35                  40                  45

Gln Ile Gly Val Ile Leu Arg Asp Ser His Gly Ile Ala Gln Val Lys
    50                  55                  60

Ser Val Thr Gly Ser Lys Ile Leu Arg Ile Leu Lys Ala His Gly Leu
65                  70                  75                  80

Ala Pro Glu Ile Pro Glu Asp Leu Tyr His Leu Ile Lys Lys Ala Val
                85                  90                  95

Ser Ile Arg Lys His Leu Glu Arg Phe Arg Lys Asp Lys Asp Ser Lys
            100                 105                 110

Phe Arg Leu Ile Leu Val Glu Ser Arg Ile His Arg Leu Ala Arg Tyr
        115                 120                 125

Tyr Lys Lys Thr Lys Lys Leu Pro Pro Val Trp Lys Tyr Glu Ser Thr
    130                 135                 140

Thr Ala Ser Thr Leu Val Ala
145                 150

<210>  45
<211>  150
<212>  PRT
<213>  Arabidopsis thaliana

<400>  45

Met Gly Arg Met His Ser Arg Gly Lys Gly Ile Ser Ala Ser Ala Leu
1               5                   10                  15

Pro Tyr Lys Arg Ser Ser Pro Ser Trp Leu Lys Thr Thr Ser Gln Asp
            20                  25                  30

Val Asp Glu Ser Ile Cys Lys Phe Ala Lys Lys Gly Leu Thr Pro Ser
        35                  40                  45

Gln Ile Gly Val Ile Leu Arg Asp Ser His Gly Ile Pro Gln Val Lys
    50                  55                  60

Ser Val Thr Gly Ser Lys Ile Leu Arg Ile Leu Lys Ala His Gly Leu
65                  70                  75                  80

Ala Pro Glu Ile Pro Glu Asp Leu Tyr His Leu Ile Lys Lys Ala Val
                85                  90                  95

Ala Ile Arg Lys His Leu Glu Arg Asn Arg Lys Asp Lys Asp Ser Lys
            100                 105                 110

Phe Arg Leu Ile Leu Val Glu Ser Arg Ile His Arg Leu Ala Arg Tyr
        115                 120                 125

Tyr Lys Lys Thr Lys Lys Leu Pro Pro Val Trp Asn Glu Ser Thr Thr
    130                 135                 140

Ala Ser Thr Leu Val Ala
145                 150

<210>  46
<211>  150
<212>  PRT
<213>  Arabidopsis thaliana

<400>  46

Met Gly Arg Met His Ser Arg Gly Lys Gly Ile Ser Ala Ser Ala Leu
1               5                   10                  15

Pro Tyr Lys Arg Ser Ser Pro Ser Trp Leu Lys Thr Thr Pro Gln Asp
            20                  25                  30

Val Asp Glu Ser Ile Cys Lys Phe Ala Lys Lys Gly Leu Thr Pro Ser
        35                  40                  45

Gln Ile Gly Val Ile Leu Arg Asp Ser His Gly Ile Pro Gln Val Lys
    50                  55                  60

Ser Val Thr Gly Ser Lys Ile Leu Arg Ile Leu Lys Ala His Gly Leu
65                  70                  75                  80

Ala Pro Glu Ile Pro Glu Asp Leu Tyr His Leu Ile Lys Lys Ala Val
                85                  90                  95

Ala Ile Arg Lys His Leu Glu Arg Asn Arg Lys Asp Lys Asp Ser Lys
            100                 105                 110

Phe Arg Leu Ile Leu Val Glu Ser Arg Ile His Arg Leu Ala Arg Tyr
        115                 120                 125

Tyr Lys Lys Thr Lys Lys Leu Pro Pro Val Trp Asn Glu Ser Thr Thr
    130                 135                 140

Ala Ser Thr Leu Val Ala
145                 150

<210>  47
<211>  151
<212>  PRT
<213>  Oryza sativa

<400>  47

Met Gly Arg Met His Ser Arg Gly Lys Gly Ile Ser Ser Ser Ala Leu
1               5                   10                  15

Pro Tyr Lys Arg Thr Pro Pro Ser Trp Leu Lys Thr Ala Ala Ser Asp
            20                  25                  30

Val Glu Glu Met Ile Met Lys Ala Ala Lys Lys Gly Gln Met Pro Ser
        35                  40                  45

Gln Ile Gly Val Val Leu Arg Asp Gln His Gly Ile Pro Leu Val Lys
    50                  55                  60

Ser Val Thr Gly Ser Lys Ile Leu Arg Ile Leu Lys Ala His Gly Leu
65                  70                  75                  80

Ala Pro Glu Ile Pro Glu Asp Leu Tyr Phe Leu Ile Lys Lys Ala Val
                85                  90                  95

Ala Ile Arg Lys His Leu Glu Arg Asn Arg Lys Asp Lys Asp Ser Lys
            100                 105                 110

Phe Arg Leu Ile Leu Val Glu Ser Arg Ile His Arg Leu Ala Arg Tyr
        115                 120                 125

Tyr Lys Arg Thr Lys Lys Leu Pro Pro Thr Trp Lys Tyr Glu Ser Thr
    130                 135                 140

Thr Ala Ser Thr Leu Val Ala
145                 150


<210>  48
<211>  151
<212>  PRT
<213>  Zea mays

<400>  48

Met Gly Ala Met His Ser Arg Gly Lys Gly Ile Ser Ser Ser Ala Leu
1               5                   10                  15

Pro Tyr Lys Arg Thr Pro Pro Thr Trp Leu Lys Thr Ala Ala Ser Asp
            20                  25                  30

Val Glu Glu Met Ile Thr Lys Ala Ala Lys Lys Gly Gln Met Pro Ser
        35                  40                  45

Gln Ile Gly Val Leu Leu Arg Asp Gln His Gly Ile Pro Leu Val Lys
    50                  55                  60

Ser Val Thr Gly Ser Lys Ile Leu Arg Ile Leu Lys Ala His Gly Leu
65                  70                  75                  80

Ala Pro Glu Ile Pro Glu Asp Leu Tyr Phe Leu Ile Lys Lys Ala Val
                85                  90                  95

Ala Ile Arg Lys His Leu Glu Arg Asn Arg Lys Asp Lys Asp Ser Lys
            100                 105                 110

Phe Arg Leu Ile Leu Val Glu Ser Arg Ile His Arg Leu Ala Arg Tyr
        115                 120                 125

Tyr Lys Arg Thr Lys Lys Leu Pro Pro Thr Trp Lys Tyr Glu Ser Thr
    130                 135                 140

Thr Ala Ser Thr Leu Val Ala
145                 150

<210>  49
<211>  156
<212>  PRT
<213>  Oryza sativa

<400>  49

Met Gly Arg Met His Ser Arg Gly Lys Gly Ile Ser Ser Ser Ala Ile
1               5                   10                  15

Pro Tyr Lys Arg Thr Pro Pro Ser Trp Val Lys Thr Ala Ala Ala Asp
            20                  25                  30

Val Glu Glu Met Ile Met Lys Ala Ala Lys Lys Gly Gln Met Pro Ser
        35                  40                  45

Gln Ile Gly Val Val Leu Arg Asp Gln His Gly Ile Pro Leu Val Lys
    50                  55                  60

Ser Val Thr Gly Ser Lys Ile Leu Arg Ile Leu Lys Ala His Gly Leu
65                  70                  75                  80

Ala Pro Glu Ile Pro Glu Asp Leu Tyr Phe Leu Ile Lys Lys Ala Val
                85                  90                  95

Ala Ile Arg Lys His Leu Glu Arg Asn Arg Lys Asp Lys Asp Ser Lys
            100                 105                 110

Phe Arg Leu Ile Leu Val Glu Ser Arg Ile His Arg Leu Ala Arg Tyr
        115                 120                 125

Tyr Lys Arg Thr Lys Lys Leu Pro Pro Thr Trp Lys Thr Thr Tyr Gln
    130                 135                 140

Leu Ser Ala Val Cys Leu Asn Tyr Phe Gln Ala Pro
145                 150                 155

<210>  50
<211>  151
<212>  PRT
<213>  Drosophila melanogaster

<400>  50

Met Gly Arg Met His Ala Pro Gly Lys Gly Ile Ser Gln Ser Ala Leu
1               5                   10                  15

Pro Tyr Arg Arg Thr Val Pro Ser Trp Leu Lys Leu Asn Ala Asp Asp
            20                  25                  30

Val Lys Glu Gln Ile Lys Lys Ala Gly Gln Glu Gly Ser Asp Ser Leu
        35                  40                  45

Gln Ile Gly Ile Ile Leu Arg Asp Ser His Gly Val Ala Gln Val Arg
    50                  55                  60

Phe Val Asn Gly Asn Lys Ile Leu Arg Ile Met Lys Ser Val Gly Leu
65                  70                  75                  80

Lys Pro Asp Ile Pro Glu Asp Leu Tyr His Met Ile Lys Lys Ala Val
                85                  90                  95

Ala Ile Arg Lys His Leu Glu Arg Asn Arg Lys Asp Lys Asp Gly Lys
            100                 105                 110

Phe Arg Leu Ile Leu Val Glu Ser Arg Ile His Arg Leu Ala Arg Tyr
        115                 120                 125

Tyr Lys Thr Lys Ser Val Leu Pro Pro Asn Trp Lys Tyr Glu Ser Ser
    130                 135                 140

Thr Ala Ser Ala Leu Val Ala
145                 150

<210>  51
<211>  155
<212>  PRT
<213>  Unknown

<220>
<223>  environmental sequence

<400>  51

Met Gly Arg Met His Thr Pro Gly Lys Gly Met Ser Gly Ser Ala Leu
1               5                   10                  15

Pro Tyr Lys Arg Ser Ala Pro Ser Trp Leu Lys Ile Thr Pro Thr Glu
            20                  25                  30

Val Thr Glu Met Ile Val Lys Met Ala Lys Lys Gly Met Thr Pro Ser
        35                  40                  45

Gln Ile Gly Val Met Leu Arg Asp Asn Gln Gly Ile Ala Gln Val Ser
    50                  55                  60

Thr Val Thr Asn Ser Lys Ile Leu Arg Ile Leu Arg Gly Gln Gly Leu
65                  70                  75                  80

Ala Pro Ser Leu Pro Glu Asp Leu Tyr Cys Leu Ile Lys Lys Ala Val
                85                  90                  95

Ser Val Arg Lys His Leu Glu Arg Asn Arg Lys Asp Met Asp Ser Lys

100                 105                 110

Phe Arg Leu Ile Leu Ile Glu Ser Arg Ile His Arg Leu Ala Arg Tyr
        115                 120                 125

Tyr Lys Leu Ala Lys Lys Leu Glu Pro Thr Phe Lys Tyr Asp Ser Ala
    130                 135                 140

Thr Ala Ser Thr Leu Leu Thr Ala Ala Gly Lys
145                 150                 155

<210>  52
<211>  230
<212>  PRT
<213>  Arabidopsis thaliana

<400>  52

Met Ala Ser Ser Asn Ser Glu Lys Ile Asn Glu Asn Leu Tyr Ala Val
1               5                   10                  15

Leu Gly Leu Lys Lys Glu Cys Ser Lys Thr Glu Leu Arg Ser Ala Tyr
            20                  25                  30

Lys Lys Leu Ala Leu Arg Trp His Pro Asp Arg Cys Ser Ser Met Glu
        35                  40                  45

Phe Val Glu Glu Ala Lys Lys Lys Phe Gln Ala Ile Gln Glu Ala Tyr
    50                  55                  60

Ser Val Leu Ser Asp Ser Asn Lys Arg Phe Leu Tyr Asp Val Gly Ala
65                  70                  75                  80

Tyr Asn Thr Asp Asp Asp Asp Asp Gln Asn Gly Met Gly Asp Phe Leu
                85                  90                  95

Asn Glu Met Ala Thr Met Met Asn Gln Ser Lys Pro Ser Asp Asn Asn
            100                 105                 110

Thr Gly Asp Ser Phe Glu Gln Leu Gln Asp Leu Phe Asn Glu Met Phe
        115                 120                 125

Gln Gly Asp Ala Ala Ala Phe Pro Ser Ser Ser Ser Cys Ser Thr Ser
    130                 135                 140

Asn Phe Thr Ser Ser Arg Ser Phe Val Phe Asp Thr Asn Ser Gln Arg
145                 150                 155                 160

Ser Ser Ser Phe Ala Thr Ser Ser Met Gly Met Asn Asn Asp Pro Phe
                165                 170                 175

Gly Tyr Asp Pro Arg Ala His Ser Phe Ser Leu Gly Val Asp His Gln
            180                 185                 190

Gln Glu Phe Lys Lys Gly Lys Asn Asn Gly Gly Arg Arg Asn Arg Arg
        195                 200                 205

Lys Asn Asn Val Pro Ser Ala Gly His Glu Thr Ser Ser Ser Asn Asn

210                 215                 220

Tyr Gly Val Pro Thr Ser
225                 230

<210>  53
<211>  242
<212>  PRT
<213>  Mus musculus

<400>  53

Met Val Asp Tyr Tyr Glu Val Leu Gly Val Gln Arg His Ala Ser Pro
1               5                   10                  15

Glu Asp Ile Lys Lys Ala Tyr Arg Lys Gln Ala Leu Lys Trp His Pro
            20                  25                  30

Asp Lys Asn Pro Glu Asn Lys Glu Glu Ala Glu Arg Lys Phe Lys Gln
        35                  40                  45

Val Ala Glu Ala Tyr Glu Val Leu Ser Asp Ala Lys Lys Arg Asp Ile
    50                  55                  60

Tyr Asp Lys Tyr Gly Lys Glu Gly Leu Asn Gly Gly Gly Gly Gly Gly
65                  70                  75                  80

Gly Ile His Phe Asp Ser Pro Phe Glu Phe Gly Phe Thr Phe Arg Asn
                85                  90                  95

Pro Asp Asp Val Phe Arg Glu Phe Phe Gly Gly Arg Asp Pro Phe Ser
            100                 105                 110

Phe Asp Phe Phe Glu Asp Pro Phe Asp Asp Phe Phe Gly Asn Arg Arg
        115                 120                 125

Gly Pro Arg Gly Asn Arg Ser Arg Gly Ala Ala Pro Phe Phe Ser Thr
    130                 135                 140

Phe Ser Gly Phe Pro Ser Phe Gly Ser Gly Phe Pro Ala Phe Asp Thr
145                 150                 155                 160

Gly Phe Thr Pro Phe Gly Ser Leu Gly His Gly Gly Leu Thr Ser Phe
                165                 170                 175

Ser Ser Thr Ser Phe Gly Gly Ser Gly Met Gly Asn Phe Lys Ser Ile
            180                 185                 190

Ser Thr Ser Thr Lys Ile Val Asn Gly Lys Lys Ile Thr Thr Lys Arg
        195                 200                 205

Ile Val Glu Asn Gly Gln Glu Arg Val Glu Val Glu Glu Asp Gly Gln
    210                 215                 220

Leu Lys Pro Leu Thr Ile Asn Gly Lys Glu His Leu Leu Arg Leu Asp
225                 230                 235                 240

Asn Lys

<210>  54
<211>  242
<212>  PRT
<213>  Rattus norvegicus

<400>  54

Met Val Asp Tyr Tyr Glu Val Leu Gly Val Gln Arg His Ala Ser Pro
1               5                   10                  15

Glu Asp Ile Lys Lys Ala Tyr Arg Lys Gln Ala Leu Lys Trp His Pro
            20                  25                  30

Asp Lys Asn Pro Glu Asn Lys Glu Glu Ala Glu Arg Lys Phe Lys Gln
        35                  40                  45

Val Ala Glu Ala Tyr Glu Val Leu Ser Asp Ala Lys Lys Arg Asp Ile
    50                  55                  60

Tyr Asp Lys Tyr Gly Lys Glu Gly Leu Asn Gly Gly Gly Gly Gly Gly
65                  70                  75                  80

Gly Ser His Phe Asp Ser Pro Phe Glu Phe Gly Phe Thr Phe Arg Asn
                85                  90                  95

Pro Asp Asp Val Phe Arg Glu Phe Phe Gly Gly Arg Asp Pro Phe Ser
            100                 105                 110

Phe Asp Phe Phe Glu Asp Pro Phe Asp Asp Phe Phe Gly Asn Arg Arg
        115                 120                 125

Gly Pro Arg Gly Ser Arg Ser Arg Gly Ala Gly Ser Phe Phe Ser Ala
    130                 135                 140

Phe Ser Gly Phe Pro Ser Phe Gly Ser Gly Phe Pro Ala Phe Asp Thr
145                 150                 155                 160

Gly Phe Thr Pro Phe Gly Ser Leu Gly His Gly Gly Leu Thr Ser Phe
                165                 170                 175

Ser Ser Ala Ser Phe Gly Gly Ser Gly Met Gly Asn Phe Lys Ser Ile
            180                 185                 190

Ser Thr Ser Thr Lys Ile Val Asn Gly Lys Lys Ile Thr Thr Lys Arg
        195                 200                 205

Ile Val Glu Asn Gly Gln Glu Arg Val Glu Val Glu Glu Asp Gly Gln
    210                 215                 220

Leu Lys Ser Leu Thr Ile Asn Gly Lys Glu His Leu Leu Arg Leu Asp
225                 230                 235                 240

Asn Lys


<210>  55

<211>  158
<212>  PRT
<213>  Picea glauca

<400>  55

Met Glu Thr Thr Phe Tyr Ser Ile Leu Gly Val Asn Lys Asp Ser Ser
1               5                   10                  15

Ser Ala Glu Ile Arg Ser Ala Tyr Arg Lys Leu Ala Met Lys Trp His
            20                  25                  30

Pro Asp Lys Trp Ser Thr Asp Pro Ser Ser Ser Glu Thr Ala Lys Leu
        35                  40                  45

Arg Phe Gln Gln Ile Gln Glu Ala Tyr Ser Val Leu Ser Asp Asp Thr
    50                  55                  60

Lys Arg Ala Leu Tyr Asp Ala Gly Met Tyr Glu Pro Ser Glu Asp Met
65                  70                  75                  80

Asp Ala Phe Cys Asp Phe Leu Asp Glu Leu Ser Ser Leu Ile Ala Thr
                85                  90                  95

Val Lys Val Gln Ser Asn Lys Asp Asp Glu Leu Leu Gln Leu Gln Glu
            100                 105                 110

Met Phe Thr Lys Met Leu Glu Glu Asp Trp Phe Ser Thr Asp Asn Phe
        115                 120                 125

Glu Ala Phe Lys Glu Ile Ser Ser Gln His Ser Asp Asp Lys Pro Glu
    130                 135                 140

Asn Gly Gln Asp His Glu Pro Tyr Gly Ser Val Asp Asp Leu
145                 150                 155

<210>  56
<211>  156
<212>  PRT
<213>  Arabidopsis thaliana

<400>  56

Met Ala Cys Glu Gly Gly Gly Ser Asn Val Arg Ser Ser Tyr Tyr Thr
1               5                   10                  15

Ile Leu Gly Ile Arg Lys Asp Ala Ser Val Ser Asp Ile Arg Thr Ala
            20                  25                  30

Tyr Arg Lys Leu Ala Met Lys Trp His Pro Asp Arg Tyr Ala Arg Asn
        35                  40                  45

Pro Gly Val Ala Gly Glu Ala Lys Arg Arg Phe Gln Gln Ile Gln Glu
    50                  55                  60

Ala Tyr Ser Val Leu Asn Asp Glu Asn Lys Arg Ser Met Tyr Asp Val
65                  70                  75                  80

Gly Leu Tyr Asp Pro His Glu Asp Asp Asp Asp Asp Phe Cys Asp Phe
                85                  90                  95

Met Gln Glu Met Ile Ser Met Met Asn Asn Val Lys Asp Ala Gly Glu
            100                 105                 110

Ser Leu Glu Asp Leu Gln Arg Met Phe Thr Asp Met Val Gly Gly Asp
        115                 120                 125

Gly Val Ser Tyr Asp Cys Asn Asn Asn Pro Lys Gly Asn Lys Arg Pro
    130                 135                 140

Arg Val Asn Ile Ser Arg Ser Ser Ala Ala Met Arg
145                 150                 155

<210>  57
<211>  156
<212>  PRT
<213>  Arabidopsis thaliana

<400>  57

Met Ala Cys Glu Gly Gly Gly Ser Asn Val Arg Ser Ser Tyr Tyr Thr
1               5                   10                  15

Val Leu Gly Ile Arg Lys Asp Ala Ser Val Ser Asp Ile Arg Thr Ala
            20                  25                  30

Tyr Arg Lys Leu Ala Met Lys Trp His Pro Asp Arg Tyr Ala Arg Asn
        35                  40                  45

Pro Gly Val Ala Gly Glu Ala Lys Arg Arg Phe Gln Gln Ile Gln Glu
    50                  55                  60

Ala Tyr Ser Val Leu Asn Asp Glu Asn Lys Arg Ser Met Tyr Asp Val
65                  70                  75                  80

Gly Leu Tyr Asp Pro His Glu Asp Asp Asp Asp Asp Phe Cys Asp Phe
                85                  90                  95

Met Gln Glu Met Ile Ser Met Met Asn Asn Val Lys Asp Glu Gly Glu
            100                 105                 110

Ser Leu Glu Asp Leu Gln Arg Met Phe Thr Asp Met Val Gly Gly Asp
        115                 120                 125

Gly Val Ser Tyr Asp Cys Asn Asn Asn Pro Lys Gly Ser Lys Arg Pro
    130                 135                 140

Arg Val Asn Val Ser Arg Ser Ser Ala Ala Met Arg
145                 150                 155

<210>  58
<211>  207
<212>  PRT
<213>  Daucus carota

<400>  58

Met Ile Asp Gln Glu Glu Ser Asn Phe Asn Phe Asn Phe Asn Gln Pro
1               5                   10                  15

Gln Gln Pro Gln Gln Gln Gln Phe His Gly Lys Ser Val Lys Lys Asn
            20                  25                  30

Lys Asn Lys Asn Asn Asn Asn Asn Ser Glu Ser Gly Asn Lys Asn Gly
        35                  40                  45

Gly Glu Asn Lys Asn Gly Val Glu Lys Arg Phe Lys Thr Leu Pro Pro
    50                  55                  60

Ala Glu Ser Leu Pro Arg Asn Glu Thr Val Gly Gly Tyr Ile Phe Val
65                  70                  75                  80

Cys Asn Asn Asp Thr Met Gln Glu Asn Leu Lys Arg Gln Leu Phe Gly
                85                  90                  95

Leu Pro Pro Arg Tyr Arg Asp Ser Val Arg Ala Ile Thr Pro Gly Leu
            100                 105                 110

Pro Leu Phe Leu Tyr Asn Tyr Ser Thr His Gln Leu His Gly Val Phe
        115                 120                 125

Glu Ala Ala Ser Phe Gly Gly Thr Asn Ile Asp Pro Thr Ala Trp Glu
    130                 135                 140

Asp Lys Lys Asn Gln Gly Glu Ser Arg Phe Pro Ala Gln Val Arg Val
145                 150                 155                 160

Met Thr Arg Lys Ile Cys Glu Pro Leu Glu Glu Asp Ser Phe Arg Pro
                165                 170                 175

Ile Leu His His Tyr Asp Gly Pro Lys Phe Arg Leu Glu Leu Asn Ile
            180                 185                 190

Pro Glu Ala Ile Ser Leu Leu Asp Ile Phe Glu Glu Thr Lys Ala
        195                 200                 205

<210>  59
<211>  213
<212>  PRT
<213>  Pisum sativum

<400>  59

Met Asn Lys Asn Ser Leu Arg Asn Gly Val Tyr Asn Met Asn Ala Val
1               5                   10                  15

Tyr Gln Lys Ser Asn Ala Asn Phe Val Gly Asn Met Asn Ser Asn Lys
            20                  25                  30

Tyr Ser Gly Asn Val Gln Leu Asn Lys Asp Pro His Ser Asn Asn Asn
        35                  40                  45

Asn Asn Asn Asn Glu Asn Asn Thr Asn Ala Thr Asp Lys Arg Phe Lys
    50                  55                  60

Thr Leu Pro Ala Ala Glu Thr Leu Pro Arg Asn Glu Val Leu Gly Gly
65                  70                  75                  80

Tyr Ile Phe Val Cys Asn Asn Asp Thr Met Gln Glu Asp Leu Lys Arg
                85                  90                  95

Gln Leu Phe Gly Leu Pro Pro Arg Tyr Arg Asp Ser Val Arg Ala Ile
            100                 105                 110

Thr Pro Gly Leu Pro Leu Phe Leu Tyr Asn Tyr Thr Thr His Gln Leu
        115                 120                 125

His Gly Ile Phe Glu Ala Thr Cys Phe Gly Gly Ser Asn Ile Asp Pro
    130                 135                 140

Thr Ala Trp Glu Asp Lys Lys Cys Lys Gly Glu Ser Arg Phe Pro Ala
145                 150                 155                 160

Gln Val Arg Ile Arg Val Arg Lys Ile Cys Lys Ala Leu Glu Glu Asp
                165                 170                 175

Ser Phe Arg Pro Val Leu His His Tyr Asp Gly Pro Lys Phe Arg Leu
            180                 185                 190

Glu Leu Ser Val Pro Glu Thr Leu Asp Leu Met Asp Leu Cys Glu Gln
        195                 200                 205

Ala Gly Ser Ala Ala
    210

<210>  60
<211>  305
<212>  PRT
<213>  Citrus X paradisi

<400>  60

Met Asp Asn Met His Ser Phe Trp Gln Leu Gly Asp Glu Leu Arg Gly
1               5                   10                  15

Gln Ser Arg Thr Ser Glu Asp Gln Ser Trp Leu Arg Ala Ala Ser Arg
            20                  25                  30

Leu Ala Glu Gln Thr Arg Phe Lys Gly Glu Arg Met Asn Asn Leu Asp
        35                  40                  45

Leu Ser Lys Gly Met Thr Glu Ile Arg Pro Arg Asp Lys Ile Met Tyr
    50                  55                  60

His Glu Asp Asn Asn Phe Glu Ser Phe Asn Phe Asn Phe Asn Met Met
65                  70                  75                  80

Asn Leu Asp Asn Lys Val Val Glu Asn Val Thr Lys Ser Ser Leu Arg
                85                  90                  95

Asn Gly Ile Tyr Asn Met Asn Ala Val Tyr Gln Lys Asn Ser Gly His
            100                 105                 110

Asn Met Gly Asn Leu Met Val Asn Lys Tyr Gly Gly Asn Asn Leu Ser
        115                 120                 125

Val Lys Glu Ala Glu Asn Asn Asn Asn Asn Asn Asn Asn Asn Asn Asp
    130                 135                 140

Ser Asn Ala Asn Ser Ala Leu Asp Lys Arg Phe Lys Thr Leu Pro Ala
145                 150                 155                 160

Thr Glu Thr Leu Pro Arg Asn Glu Val Leu Gly Gly Tyr Ile Phe Val
                165                 170                 175

Cys Asn Asn Asp Thr Met Gln Glu Asp Leu Lys Arg Gln Leu Phe Gly
            180                 185                 190

Leu Pro Pro Arg Tyr Arg Asp Ser Val Arg Ala Ile Thr Pro Gly Leu
        195                 200                 205

Pro Leu Phe Leu Tyr Asn Tyr Thr Thr His Gln Leu His Gly Ile Phe
    210                 215                 220

Glu Ala Thr Gly Phe Gly Gly Ser Asn Ile Asp Pro Thr Ala Trp Glu
225                 230                 235                 240

Asp Lys Lys Cys Lys Gly Glu Ser Arg Phe Pro Ala Gln Val Arg Ile
                245                 250                 255

Arg Val Arg Lys Leu Cys Lys Ala Leu Glu Glu Asp Ala Phe Arg Pro
            260                 265                 270

Val Leu His His Tyr Asp Gly Pro Lys Phe Arg Leu Glu Leu Ser Val
        275                 280                 285

Pro Glu Thr Leu Asp Leu Met Asp Leu Cys Glu Gln Ala Gly Ser Ala
    290                 295                 300

Ala
305

<210>  61
<211>  296
<212>  PRT
<213>  Arabidopsis thaliana

<400>  61

Met Asp Ser Phe Trp Gln Leu Gly Asp Glu Leu Arg Gly Gln Thr Arg
1               5                   10                  15

Ala Ser Glu Asp His Lys Trp Ser Thr Val Ala Thr Lys Leu Ala Glu
            20                  25                  30

Gln Thr Arg Met Lys Gly Glu Arg Met Asn Asn Leu Asp Leu Ser Lys
        35                  40                  45

Gly Tyr Thr Glu Phe Arg Pro Ser Glu Lys Phe Ser Phe Gln Glu Asn
    50                  55                  60

Asn Leu Asn Phe Asn Met Leu Asn Leu Asp Gly Lys Phe Gly Glu Ser
65                  70                  75                  80

Ile Met Gly Lys Thr Ser Met Gln Ser Asn Val Tyr Asn Met Asn Thr
                85                  90                  95

Val Phe Gln Lys Asn Asp Phe Lys Ser Gly Gly Asn Met Lys Val Asn
            100                 105                 110

Lys Tyr Asn Gly Asn Ile Val Ala Asn Lys Glu Met Ser Asn Asn Lys
        115                 120                 125

His Asn Asn Asn Cys Asn Asp Asn Gly Asn Met Asn Leu Ala Val Asp
    130                 135                 140

Lys Arg Phe Lys Thr Leu Pro Ala Ser Glu Thr Leu Pro Arg Asn Glu
145                 150                 155                 160

Val Leu Gly Gly Tyr Ile Phe Val Cys Asn Asn Asp Thr Met Gln Glu
                165                 170                 175

Asp Met Lys Arg His Leu Phe Gly Leu Pro Pro Arg Tyr Arg Asp Ser
            180                 185                 190

Val Arg Ala Ile Thr Pro Gly Leu Pro Leu Phe Leu Tyr Asn Tyr Thr
        195                 200                 205

Thr His Gln Leu His Gly Ile Phe Glu Ala Thr Thr Phe Gly Gly Thr
    210                 215                 220

Asn Ile Asp Ala Thr Ala Trp Glu Asp Lys Lys Cys Lys Gly Glu Ser
225                 230                 235                 240

Arg Phe Pro Ala Gln Val Arg Ile Arg Val Arg Lys Ile Cys Lys Ala
                245                 250                 255

Leu Glu Glu Asp Ser Phe Arg Pro Val Leu His His Tyr Asp Gly Pro
            260                 265                 270

Lys Phe Arg Leu Glu Leu Ser Val Pro Glu Thr Leu Asp Leu Leu Asp
        275                 280                 285

Leu Cys Glu Gln Ala Gly Ser Ala
    290                 295

<210>  62
<211>  296
<212>  PRT
<213>  Arabidopsis thaliana

<400>  62

Met Asp Ser Phe Trp Gln Leu Gly Asp Glu Leu Arg Gly Gln Thr Arg
1               5                   10                  15

Ala Ser Glu Asp His Lys Trp Ser Thr Val Ala Thr Lys Leu Ala Glu
            20                  25                  30

Gln Thr Arg Met Lys Gly Glu Arg Met Asn Asn Leu Asp Leu Ser Lys
        35                  40                  45

Gly Tyr Thr Glu Phe Arg Pro Ser Glu Lys Phe Ser Phe Gln Glu Asn
    50                  55                  60

Asn Leu Asn Phe Asn Met Leu Asn Leu Asp Gly Lys Phe Gly Glu Ser
65                  70                  75                  80

Ile Met Gly Lys Thr Ser Met Gln Ser Asn Val Tyr Asn Met Asn Thr
                85                  90                  95

Val Phe Gln Lys Asn Asp Phe Lys Ser Gly Gly Asn Met Lys Val Asn
            100                 105                 110

Lys Tyr Asn Gly Asn Val Val Ala Asn Lys Glu Met Ser Asn Asn Lys
        115                 120                 125

His Asn Asn Asn Cys Asn Asp Asn Gly Asn Met Asn Leu Ala Val Asp
    130                 135                 140

Lys Arg Phe Lys Thr Leu Pro Ala Ser Glu Thr Leu Pro Arg Asn Glu
145                 150                 155                 160

Val Leu Gly Gly Tyr Ile Phe Val Cys Asn Asn Asp Thr Met Gln Glu
                165                 170                 175

Asp Met Lys Arg His Leu Phe Gly Leu Pro Pro Arg Tyr Arg Asp Ser
            180                 185                 190

Val Arg Ala Ile Thr Pro Gly Leu Pro Leu Phe Leu Tyr Asn Tyr Thr
        195                 200                 205

Thr His Gln Leu His Gly Ile Phe Glu Ala Thr Thr Phe Gly Gly Thr
    210                 215                 220

Asn Ile Asp Ala Thr Ala Trp Glu Asp Lys Lys Cys Lys Gly Glu Ser
225                 230                 235                 240

Arg Phe Pro Ala Gln Val Arg Ile Arg Val Arg Lys Ile Cys Lys Ala
                245                 250                 255

Leu Glu Glu Asp Ser Phe Arg Pro Val Leu His His Tyr Asp Gly Pro
            260                 265                 270

Lys Phe Arg Leu Glu Leu Ser Val Pro Glu Thr Leu Asp Leu Leu Asp
        275                 280                 285

Leu Cys Glu Gln Ala Gly Ser Ala
    290                 295

<210>  63
<211>  742
<212>  PRT
<213>  Arabidopsis thaliana

<400>  63

Met Ala Glu Ala Met Glu Thr Glu Met Asp Phe Ser Asp Gly Glu Gln
1               5                   10                  15

Thr Asn Gly Asn Ser His Val Thr Ala Ser Gln Tyr Phe Ala Pro Pro
            20                  25                  30

Gly Tyr Asn Arg Ser Leu Val Ala Ala Tyr Gly Asn Gly Asn Thr Thr
        35                  40                  45

Ile Gly Leu Glu Lys Gly Ile Glu Arg Arg Leu Asp His His Glu Gln
    50                  55                  60

Leu Pro Gly Tyr Ile Phe Met Cys Asn Gly Arg Thr Lys Thr Asp Cys
65                  70                  75                  80

Tyr Arg Tyr Arg Val Phe Gly Ile Pro Arg Gly Gly Lys Asp Val Val
                85                  90                  95

Glu Ser Ile Lys Pro Gly Met Lys Leu Phe Leu Tyr Asp Phe Glu Lys
            100                 105                 110

Arg Leu Leu Tyr Gly Val Tyr Glu Ala Thr Val Gly Gly Arg Leu Asp
        115                 120                 125

Ile Glu Pro Glu Ala Phe Glu Gly Lys Tyr Pro Ala Gln Val Gly Phe
    130                 135                 140

Arg Ile Val Met Asn Cys Leu Pro Leu Thr Glu Asn Thr Phe Lys Ser
145                 150                 155                 160

Ala Ile Tyr Glu Asn Tyr Lys Gly Ser Lys Phe Lys Gln Glu Leu Ser
                165                 170                 175

Pro His Gln Val Met Ser Leu Leu Ser Leu Phe Arg Ser Phe Thr Ser
            180                 185                 190

Pro Glu Leu Asp Leu Leu Pro His Arg Leu Ala Ser Arg Ala Ser Ala
        195                 200                 205

Pro Arg Thr Leu Ser Phe Glu Glu Arg Phe Ile Ala Ala Thr His Leu
    210                 215                 220

Arg Asn Ala Ser Ser Val Leu Asp Pro Leu Ser Ala Arg His Val Glu
225                 230                 235                 240

Pro Arg Leu Gly Ser Val Met Ala His Gln Pro Val Pro Arg Thr Ser
                245                 250                 255

Leu Leu Gln His Ser Tyr Phe Arg Gln Asp Asp Tyr Thr Thr Pro Pro
            260                 265                 270

Arg Glu Ser Leu Ser Asn Leu Asn Gln Pro Tyr Tyr Pro Thr Glu Ala
        275                 280                 285

Arg Gln Leu Arg Leu Leu Gly Asp Pro Ser Arg Ser Asp Ser Pro Arg
    290                 295                 300

Ser Glu Pro Pro Arg Ser Ser Ile Gln Asp Pro Gln Leu Lys Tyr Leu
305                 310                 315                 320

Thr Ile Leu Ser Asn Ile Arg Arg Tyr Gly Ser Ala Ser Asp Arg Leu
                325                 330                 335

Ala Ser Glu Asn Glu Tyr His Pro Ala Thr Pro Ser Glu Lys Asp Gln
            340                 345                 350

Phe Ala Val Pro Tyr Ser Asp Asn Lys Asn Tyr Pro Ser Thr Leu Ser
        355                 360                 365

Gly Ser Glu His Pro Ser Ala Ser Ala Ala Asn Gly Ser Val Tyr Arg
    370                 375                 380

Ser Glu Phe Tyr Asn Ser Ala Ser Gln Lys Glu Gly Glu Ala Ser Gln
385                 390                 395                 400

Gln His Glu Ile Pro Ala Gly Thr Tyr His His Pro Glu Ala Ser Thr
                405                 410                 415

Val Ser Asn Thr Thr Lys Ser Met Gln Pro Asp Met Gln Ala Val Ser
            420                 425                 430

Val Ala Gln Ser His Thr Glu Thr Ala Gly Tyr Pro Thr Pro Ala His
        435                 440                 445

Gly Glu Ala Ser Gln Pro Pro Ala Gly Ala Ile Gly Tyr Thr His Gln
    450                 455                 460

Pro Gln Ser Val Ala Gly Asn Tyr Ser Thr His Ser Gln Pro Gly Asn
465                 470                 475                 480

Val Glu Glu Ser Thr Gln Ser Tyr Ala Gly Thr Asp Ser Tyr Ser Gln
                485                 490                 495

Gln Gln Tyr Tyr Ala Ala Met Gly Pro Thr Thr Gln Leu His Ala Gly
            500                 505                 510

Gly Tyr Ile Gln Lys Pro His Glu Ile Gly Tyr Ser Gln Gln Pro His
        515                 520                 525

Asp Ala Ala Thr Gly Tyr Ser Gln Gln Pro His Asp Ala Ala Thr Gly
    530                 535                 540

Tyr Ser Gln Gln Pro His Asp Ala Ala Thr Gly Tyr Ser Gln Gln Pro
545                 550                 555                 560

His Ala Ala Ser Thr Gly Tyr Ser Gln Gln Thr Tyr Ala Ala Ala Thr
                565                 570                 575

Gly Tyr Thr Gln Gln Pro His Ala Ala Ala Ala Gly Tyr Thr Gln Gln
            580                 585                 590

Pro His Ala Ala Ala Thr Gly Tyr Ser Gln Gln Pro His Ala Ala Ala
        595                 600                 605

Thr Ala His Ala Gln Gln Pro Tyr Ala Ala Ala Thr Ala His Ala Gln

610                 615                 620

Gln Leu His Ala Val Ala Thr Gly Tyr Ala Leu Gln Leu His Ala Ala
625                 630                 635                 640

Ala Thr Gly Tyr Ala Gln Gln Pro His Ala Ala Ala Thr Gly Tyr Ala
                645                 650                 655

Leu Gln Pro His Ala Gln Ala Val Glu Tyr Thr Met Gln Pro His Ala
            660                 665                 670

Gln Ala Val Gly Tyr Met Pro Gln Tyr His Ala His Ala Val Val Tyr
        675                 680                 685

Ser Gln Gln Gly Val Thr Gln Gly Ser Val Pro Arg Ala Pro Gly Thr
    690                 695                 700

Thr Asp Cys Asn Ala Ala Asn Gln Ala Tyr Ser Ala Thr Gly Asp Trp
705                 710                 715                 720

Asn Ala Val His Gln Ser Tyr Tyr Pro Gln Thr Ala Asp Ala Thr Thr
                725                 730                 735

Thr Tyr Tyr Gln Thr Ser
            740

<210>  64
<211>  195
<212>  PRT
<213>  Arabidopsis thaliana

<400>  64

Met Ala Gly Val Ala Phe Gly Ser Phe Asp Asp Ser Phe Ser Leu Ala
1               5                   10                  15

Ser Leu Arg Ala Tyr Leu Ala Glu Phe Ile Ser Thr Leu Leu Phe Val
            20                  25                  30

Phe Ala Gly Val Gly Ser Ala Ile Ala Tyr Ala Lys Leu Thr Ser Asp
        35                  40                  45

Ala Ala Leu Asp Thr Pro Gly Leu Val Ala Ile Ala Val Cys His Gly
    50                  55                  60

Phe Ala Leu Phe Val Ala Val Ala Ile Gly Ala Asn Ile Ser Gly Gly
65                  70                  75                  80

His Val Asn Pro Ala Val Thr Phe Gly Leu Ala Val Gly Gly Gln Ile
                85                  90                  95

Thr Val Ile Thr Gly Val Phe Tyr Trp Ile Ala Gln Leu Leu Gly Ser
            100                 105                 110

Thr Ala Ala Cys Phe Leu Leu Lys Tyr Val Thr Gly Gly Leu Ala Val
        115                 120                 125

Pro Thr His Ser Val Ala Ala Gly Leu Gly Ser Ile Glu Gly Val Val

130                 135                 140

Met Glu Ile Ile Ile Thr Phe Ala Leu Val Tyr Thr Val Tyr Ala Thr
145                 150                 155                 160

Ala Ala Asp Pro Lys Lys Gly Ser Leu Gly Thr Ile Ala Pro Leu Ala
                165                 170                 175

Ile Gly Leu Ile Val Gly Ala Asn Ile Leu Ala Ala Gly Pro Phe Ser
            180                 185                 190

Gly Gly Ser
        195

<210>  65
<211>  248
<212>  PRT
<213>  Gossypium hirsutum

<400>  65

Met Ala Gly Ile Ala Phe Gly Arg Phe Asp Asp Ser Phe Ser Leu Gly
1               5                   10                  15

Thr Val Lys Ala Tyr Leu Ala Glu Phe Ile Ser Thr Leu Val Phe Val
            20                  25                  30

Phe Ala Gly Val Gly Ser Ala Ile Ala Tyr Asn Lys Leu Thr Thr Asp
        35                  40                  45

Ala Ala Leu Asp Pro Asp Gly Leu Val Ala Ile Ala Val Cys His Gly
    50                  55                  60

Phe Ala Leu Phe Val Ala Val Ala Ile Gly Ala Asn Ile Ser Gly Gly
65                  70                  75                  80

His Val Asn Pro Ala Val Thr Phe Gly Leu Ala Leu Gly Gly Gln Ile
                85                  90                  95

Thr Ile Leu Thr Gly Ile Phe Tyr Trp Ile Ala Gln Leu Leu Gly Ser
            100                 105                 110

Ile Val Ala Cys Phe Leu Leu Lys Ala Val Thr Gly Gly Leu Thr Val
        115                 120                 125

Pro Ile His Gly Leu Gly Ala Gly Val Gly Ala Ile Gln Gly Val Val
    130                 135                 140

Met Glu Ile Ile Ile Thr Phe Ala Leu Val Tyr Thr Val Tyr Ala Thr
145                 150                 155                 160

Ala Ala Asp Pro Lys Lys Gly Ser Leu Gly Thr Ile Ala Pro Ile Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Ala Val Ala Ser

195                 200                 205

Gly Asp Phe Asn Gly Ile Trp Ile Tyr Trp Val Gly Pro Leu Ile Gly
    210                 215                 220

Gly Gly Leu Ser Gly Leu Ile Tyr Gly Asn Val Phe Met Asn Ser Asp
225                 230                 235                 240

His Ala Pro Leu Ser Asn Asp Phe
                245

<210>  66
<211>  250
<212>  PRT
<213>  Arabidopsis thaliana

<400>  66

Met Ala Gly Val Ala Phe Gly Ser Phe Asp Asp Ser Phe Ser Leu Ala
1               5                   10                  15

Ser Leu Arg Ala Tyr Leu Ala Glu Phe Ile Ser Thr Leu Leu Phe Val
            20                  25                  30

Phe Ala Gly Val Gly Ser Ala Ile Ala Tyr Ala Lys Leu Thr Ser Asp
        35                  40                  45

Ala Ala Leu Asp Thr Pro Gly Leu Val Ala Ile Ala Val Cys His Gly
    50                  55                  60

Phe Ala Leu Phe Val Ala Val Ala Ile Gly Ala Asn Ile Ser Gly Gly
65                  70                  75                  80

His Val Asn Pro Ala Val Thr Phe Gly Leu Ala Val Gly Gly Gln Ile
                85                  90                  95

Thr Val Ile Thr Gly Val Phe Tyr Trp Ile Ala Gln Leu Leu Gly Ser
            100                 105                 110

Thr Ala Ala Cys Phe Leu Leu Lys Tyr Val Thr Gly Gly Leu Ala Val
        115                 120                 125

Pro Thr His Ser Val Ala Ala Gly Leu Gly Ser Ile Glu Gly Val Val
    130                 135                 140

Met Glu Ile Ile Ile Thr Phe Ala Leu Val Tyr Thr Val Tyr Ala Thr
145                 150                 155                 160

Ala Ala Asp Pro Lys Lys Gly Ser Leu Gly Thr Ile Ala Pro Leu Ala
                165                 170                 175

Ile Gly Leu Ile Val Gly Ala Asn Ile Leu Ala Ala Gly Pro Phe Ser
            180                 185                 190

Gly Gly Ser Met Asn Pro Ala Arg Ser Phe Gly Pro Ala Val Ala Ala
        195                 200                 205

Gly Asp Phe Ser Gly His Trp Val Tyr Trp Val Gly Pro Leu Ile Gly

210                 215                 220

Gly Glu Leu Ala Gly Leu Ile Tyr Gly Asn Val Phe Met Gly Ser Ser
225                 230                 235                 240

Glu His Val Pro Leu Ala Ser Ala Asp Phe
                245                 250

<210>  67
<211>  250
<212>  PRT
<213>  Arabidopsis thaliana

<400>  67

Met Ala Gly Val Ala Phe Gly Ser Phe Asp Asp Ser Phe Ser Leu Ala
1               5                   10                  15

Ser Leu Arg Ala Tyr Leu Ala Glu Phe Ile Ser Thr Leu Leu Phe Val
            20                  25                  30

Phe Ala Gly Val Gly Ser Ala Ile Ala Tyr Ala Lys Leu Thr Ser Asp
        35                  40                  45

Ala Ala Leu Asp Thr Pro Gly Leu Val Ala Ile Ala Val Cys His Gly
    50                  55                  60

Phe Ala Leu Phe Val Ala Val Ala Ile Gly Ala Asn Ile Ser Gly Gly
65                  70                  75                  80

His Val Asn Pro Ala Val Thr Phe Gly Leu Ala Val Gly Gly Gln Ile
                85                  90                  95

Thr Val Ile Thr Gly Val Phe Tyr Trp Ile Ala Gln Leu Leu Gly Ser
            100                 105                 110

Thr Ala Ala Cys Phe Leu Leu Lys Tyr Val Thr Gly Gly Leu Ala Val
        115                 120                 125

Pro Thr His Ser Val Ala Ala Gly Leu Gly Ser Ile Glu Gly Val Val
    130                 135                 140

Met Glu Ile Ile Ile Thr Phe Ala Leu Val Tyr Thr Val Tyr Ala Thr
145                 150                 155                 160

Ala Ala Asp Pro Lys Lys Gly Ser Leu Gly Thr Ile Ala Pro Leu Ala
                165                 170                 175

Ile Gly Leu Ile Val Gly Ala Asn Ile Leu Ala Ala Gly Pro Phe Ser
            180                 185                 190

Gly Gly Ser Met Asn Pro Ala Arg Ser Phe Gly Pro Ala Val Ala Ala
        195                 200                 205

Gly Asp Phe Ser Gly His Trp Val Tyr Trp Val Gly Pro Leu Ile Gly
    210                 215                 220

Gly Gly Leu Ala Gly Leu Ile Tyr Gly Asn Val Phe Met Gly Ser Ser

225                 230                 235                 240

Glu His Val Pro Leu Ala Ser Ala Asp Phe
                245                 250

<210>  68
<211>  250
<212>  PRT
<213>  Arabidopsis thaliana

<400>  68

Met Val Lys Ile Glu Ile Gly Ser Val Gly Asp Ser Phe Ser Val Ala
1               5                   10                  15

Ser Leu Lys Ala Tyr Leu Ser Glu Phe Ile Ala Thr Leu Leu Phe Val
            20                  25                  30

Phe Ala Gly Val Gly Ser Ala Leu Ala Phe Ala Lys Leu Thr Ser Asp
        35                  40                  45

Ala Ala Leu Asp Pro Ala Gly Leu Val Ala Val Ala Val Ala His Ala
    50                  55                  60

Phe Ala Leu Phe Val Gly Val Ser Ile Ala Ala Asn Ile Ser Gly Gly
65                  70                  75                  80

His Leu Asn Pro Ala Val Thr Leu Gly Leu Ala Val Gly Gly Asn Ile
                85                  90                  95

Thr Val Ile Thr Gly Phe Phe Tyr Trp Ile Ala Gln Cys Leu Gly Ser
            100                 105                 110

Ile Val Ala Cys Leu Leu Leu Val Phe Val Thr Asn Gly Glu Ser Val
        115                 120                 125

Pro Thr His Gly Val Ala Ala Gly Leu Gly Ala Ile Glu Gly Val Val
    130                 135                 140

Met Glu Ile Val Val Thr Phe Ala Leu Val Tyr Thr Val Tyr Ala Thr
145                 150                 155                 160

Ala Ala Asp Pro Lys Lys Gly Ser Leu Gly Thr Ile Ala Pro Ile Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Ala Val Val Ser
        195                 200                 205

Gly Asp Phe Ser Gln Ile Trp Ile Tyr Trp Val Gly Pro Leu Val Gly
    210                 215                 220

Gly Ala Leu Ala Gly Leu Ile Tyr Gly Asp Val Phe Ile Gly Ser Tyr
225                 230                 235                 240

Ala Pro Ala Pro Thr Thr Glu Ser Tyr Pro

245                 250

<210>  69
<211>  248
<212>  PRT
<213>  Nicotiana glauca

<400>  69

Met Pro Gly Ile Ala Phe Gly Arg Ile Asp Asp Ser Phe Ser Val Gly
1               5                   10                  15

Ser Leu Lys Ala Tyr Leu Ala Glu Phe Ile Ser Thr Leu Leu Phe Val
            20                  25                  30

Phe Ala Gly Val Gly Ser Ala Ile Ala Tyr Asn Lys Leu Thr Ala Asn
        35                  40                  45

Ala Ala Leu Asp Pro Ala Gly Leu Val Ala Val Ala Val Cys His Gly
    50                  55                  60

Phe Ala Leu Phe Val Ala Val Ala Val Gly Ala Asn Ile Ser Gly Gly
65                  70                  75                  80

His Val Asn Pro Ala Val Thr Phe Gly Leu Ala Leu Gly Gly Gln Ile
                85                  90                  95

Thr Leu Leu Thr Gly Leu Phe Tyr Ile Ile Ala Gln Leu Leu Gly Ser
            100                 105                 110

Ile Val Ala Cys Leu Leu Leu Lys Val Val Thr Gly Gly Leu Ala Val
        115                 120                 125

Pro Thr His Asn Val Ala Ala Gly Val Gly Ala Leu Glu Gly Val Val
    130                 135                 140

Met Glu Ile Ile Ile Thr Phe Ala Leu Val Tyr Thr Val Tyr Ala Thr
145                 150                 155                 160

Ala Ala Asp Pro Lys Lys Gly Ser Leu Gly Thr Ile Ala Pro Ile Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Ala Val Ala Ser
        195                 200                 205

Gly Asp Phe Thr Asn Asn Trp Ile Tyr Trp Ala Gly Pro Leu Val Gly
    210                 215                 220

Gly Gly Leu Ala Gly Leu Thr Tyr Ser Asn Val Phe Met Gln His Glu
225                 230                 235                 240

His Ala Pro Leu Ser Ser Asp Phe
                245

<210>  70

<211>  248
<212>  PRT
<213>  Raphanus sativus

<400>  70

Met Ala Gly Val Ala Phe Gly Ser Phe Asp Asp Ser Phe Ser Leu Ala
1               5                   10                  15

Ser Leu Arg Ala Tyr Leu Ala Glu Phe Ile Ser Thr Leu Leu Phe Val
            20                  25                  30

Phe Ala Gly Val Gly Ser Ala Ile Ala Tyr Ala Lys Leu Thr Ser Asp
        35                  40                  45

Ala Ala Leu Asp Thr Pro Gly Leu Val Ala Ile Ala Val Cys His Gly
    50                  55                  60

Phe Ala Leu Phe Val Ala Val Ala Ile Gly Ala Asn Ile Ser Gly Gly
65                  70                  75                  80

His Val Asn Pro Ala Val Thr Phe Gly Leu Ala Val Gly Gly Gln Ile
                85                  90                  95

Thr Leu Ile Thr Gly Val Phe Tyr Trp Val Ala Gln Leu Leu Gly Ser
            100                 105                 110

Thr Ala Ala Cys Phe Leu Leu Lys Tyr Val Thr Gly Gly Leu Ala Val
        115                 120                 125

Pro Thr His Ser Val Ala Ala Gly Val Gly Ala Ile Glu Gly Val Val
    130                 135                 140

Met Glu Ile Ile Ile Thr Phe Ser Leu Val Tyr Thr Val Tyr Pro Thr
145                 150                 155                 160

Ala Ala Asp Pro Lys Lys Gly Ser Leu Gly Thr Ile Ala Pro Leu Ala
                165                 170                 175

Ile Gly Leu Ile Val Gly Ala Asn Ile Leu Ala Ala Gly Pro Phe Ser
            180                 185                 190

Gly Gly Ser Met Asn Pro Ala Arg Ser Phe Gly Pro Ala Val Ala Ala
        195                 200                 205

Gly Asp Phe Ser Gly His Trp Val Tyr Trp Val Gly Pro Leu Ile Gly
    210                 215                 220

Gly Gly Leu Ala Gly Val Thr Tyr Gly Asn Val Phe Met Thr Ser Glu
225                 230                 235                 240

His Val Pro Leu Ala Ser Glu Phe
                245


<210>  71
<211>  248
<212>  PRT
<213>  Helianthus annuus

<400>  71

Met Pro Gly Ile Ala Phe Gly Ser Phe Asp Asp Ser Phe Ser Leu Ser
1               5                   10                  15

Ser Ile Lys Ser Tyr Val Ala Glu Phe Ile Ser Thr Leu Leu Phe Val
            20                  25                  30

Phe Ala Gly Val Gly Ser Ala Ile Ala Phe Ala Lys Leu Thr Ala Asp
        35                  40                  45

Ala Asp Leu Asp Pro Ala Gly Leu Val Ala Ile Ala Val Cys His Gly
    50                  55                  60

Leu Ala Leu Phe Val Ala Val Ser Ile Ala Ala Asn Ile Ser Gly Gly
65                  70                  75                  80

His Val Asn Pro Ala Val Thr Phe Gly Leu Ala Val Gly Gly Gln Ile
                85                  90                  95

Thr Ile Leu Thr Gly Ile Phe Tyr Trp Ile Ala Gln Cys Ile Gly Ser
            100                 105                 110

Ile Ala Ala Cys Tyr Leu Leu Ser Phe Val Thr Gly Gly Leu Ala Val
        115                 120                 125

Pro Thr His Ala Val Ala Ala Gly Val Gly Ala Ile Gln Gly Val Val
    130                 135                 140

Met Glu Ile Ile Ile Thr Phe Ala Leu Val Tyr Asn Val Tyr Ala Thr
145                 150                 155                 160

Ala Val Asp Pro Lys Lys Gly Asp Leu Gly Thr Ile Ala Pro Leu Ala
                165                 170                 175

Ile Gly Leu Ile Val Gly Ala Asn Ile Leu Ala Ala Gly Pro Phe Ser
            180                 185                 190

Gly Gly Ser Met Asn Pro Ala Arg Ser Phe Gly Pro Ala Val Ala Ala
        195                 200                 205

Gly Asp Phe Ser Gly His Trp Ile Tyr Trp Val Gly Pro Leu Val Gly
    210                 215                 220

Gly Gly Leu Ala Gly Ala Ile Tyr Ser Asn Val Phe Ile Ser Asn Glu
225                 230                 235                 240

His Ala Pro Leu Ser Ser Glu Phe
                245

<210>  72
<211>  248
<212>  PRT
<213>  Oryza sativa

<400>  72

Met Val Lys Leu Ala Phe Gly Ser Leu Gly Asp Ser Phe Ser Ala Thr
1               5                   10                  15

Ser Val Lys Ala Tyr Val Ala Glu Phe Ile Ala Thr Leu Leu Phe Val
            20                  25                  30

Phe Ala Gly Val Gly Ser Ala Ile Ala Tyr Gly Gln Leu Thr Asn Gly
        35                  40                  45

Gly Ala Leu Asp Pro Ala Gly Leu Val Ala Ile Ala Ile Ala His Ala
    50                  55                  60

Leu Ala Leu Phe Val Gly Val Ser Val Ala Ala Asn Ile Ser Gly Gly
65                  70                  75                  80

His Leu Asn Pro Ala Val Thr Phe Gly Leu Ala Val Gly Gly His Ile
                85                  90                  95

Thr Ile Leu Thr Gly Leu Phe Tyr Trp Ile Ala Gln Leu Leu Gly Ala
            100                 105                 110

Ser Ile Ala Cys Leu Leu Leu Lys Phe Val Thr His Gly Lys Ala Ile
        115                 120                 125

Pro Thr His Gly Val Ala Gly Ile Ser Glu Leu Glu Gly Val Val Met
    130                 135                 140

Glu Ile Val Ile Thr Phe Ala Leu Val Tyr Thr Val Tyr Ala Thr Ala
145                 150                 155                 160

Ala Asp Pro Lys Lys Gly Ser Leu Gly Thr Ile Ala Pro Ile Ala Ile
                165                 170                 175

Gly Phe Ile Val Gly Ala Asn Ile Leu Ala Ala Gly Pro Phe Ser Gly
            180                 185                 190

Gly Ser Met Asn Pro Ala Arg Ser Phe Gly Pro Ala Val Ala Ala Gly
        195                 200                 205

Asn Phe Ala Gly Asn Trp Val Tyr Trp Val Gly Pro Leu Ile Gly Gly
    210                 215                 220

Gly Leu Ala Gly Leu Val Tyr Gly Asp Val Phe Ile Gly Ser Tyr Gln
225                 230                 235                 240

Pro Val Ala Asp Gln Asp Tyr Ala
                245

<210>  73
<211>  248
<212>  PRT
<213>  Zea mays

<400>  73

Met Val Lys Leu Ala Phe Gly Ser Phe Arg Asp Ser Leu Ser Ala Ala
1               5                   10                  15

Ser Leu Lys Ala Tyr Val Ala Glu Phe Ile Ala Thr Leu Leu Phe Val
            20                  25                  30

Phe Ala Gly Val Gly Ser Ala Ile Ala Tyr Ser Gln Leu Thr Lys Gly
        35                  40                  45

Gly Ala Leu Asp Pro Ala Gly Leu Val Ala Ile Ala Ile Ala His Ala
    50                  55                  60

Phe Ala Leu Phe Val Gly Val Ser Met Ala Ala Asn Ile Ser Gly Gly
65                  70                  75                  80

His Leu Asn Pro Ala Val Thr Phe Gly Leu Ala Val Gly Gly His Ile
                85                  90                  95

Thr Ile Leu Thr Gly Ile Leu Tyr Trp Val Ala Gln Leu Leu Gly Ala
            100                 105                 110

Ser Val Ala Cys Phe Leu Leu Gln Tyr Val Thr His Gly Gln Ala Ile
        115                 120                 125

Pro Thr His Gly Val Ser Gly Ile Ser Glu Ile Glu Gly Val Val Met
    130                 135                 140

Glu Ile Val Ile Thr Phe Ala Leu Val Tyr Thr Val Tyr Ala Thr Ala
145                 150                 155                 160

Ala Asp Pro Lys Lys Gly Ser Leu Gly Thr Ile Ala Pro Met Ala Ile
                165                 170                 175

Gly Phe Ile Val Gly Ala Asn Ile Leu Ala Ala Gly Pro Phe Ser Gly
            180                 185                 190

Gly Ser Met Asn Pro Ala Arg Ser Phe Gly Pro Ala Val Ala Ala Gly
        195                 200                 205

Asn Phe Ala Gly Asn Trp Val Tyr Trp Val Gly Pro Leu Val Gly Gly
    210                 215                 220

Gly Leu Ala Gly Leu Val Tyr Gly Asp Val Phe Ile Ala Ser Tyr Gln
225                 230                 235                 240

Pro Val Gly Gln Gln Glu Tyr Pro
                245

<210> 74
<211> 249
<212> PRT
<213> Hordeum vulgare

<400> 74

Met Val Lys Leu Ala Phe Gly Ser Phe Gly Asp Ser Phe Ser Ala Thr
1               5                   10                  15

Ser Ile Arg Ser Tyr Val Ala Glu Phe Ile Ala Thr Leu Leu Phe Val
            20                  25                  30

Phe Ala Gly Val Gly Ser Ala Ile Ser Tyr Gly Gln Leu Thr Gln Gly
        35                  40                  45

Gly Ala Leu Asp Pro Ala Gly Leu Val Ala Ile Ala Ile Ala His Ala
    50                  55                  60

Phe Ala Leu Phe Val Gly Val Ala Met Ala Ala Asn Ile Ser Gly Gly
65                  70                  75                  80

His Leu Asn Pro Ala Val Thr Phe Gly Leu Ala Val Gly Gly His Val
                85                  90                  95

Thr Ile Leu Thr Gly Leu Phe Tyr Trp Val Ala Gln Leu Leu Gly Ala
            100                 105                 110

Ser Val Ala Cys Leu Leu Leu Gln Phe Val Thr His Ala Gln Ala Met
        115                 120                 125

Pro Thr His Ala Val Ser Gly Ile Ser Glu Val Glu Gly Val Val Met
    130                 135                 140

Glu Ile Val Ile Thr Phe Ala Leu Val Tyr Thr Val Tyr Ala Thr Ala
145                 150                 155                 160

Ala Asp Pro Lys Lys Gly Ser Leu Gly Thr Ile Ala Pro Met Ala Ile
                165                 170                 175

Gly Phe Ile Val Gly Ala Asn Ile Leu Ala Ala Gly Pro Phe Ser Gly
            180                 185                 190

Gly Ser Met Asn Pro Ala Arg Ser Phe Gly Pro Ala Val Ala Ala Gly
        195                 200                 205

Asn Phe Ser Gly His Trp Val Tyr Trp Val Gly Pro Leu Ile Gly Gly
    210                 215                 220

Gly Leu Ala Gly Leu Val Tyr Gly Asp Val Phe Ile Ala Ser Tyr Gln
225                 230                 235                 240

Pro Val Gly His Gln Gln Glu Tyr Pro
                245

<210>  75
<211>  228
<212>  PRT
<213>  Antirrhinum majus

<400>  75

Phe Gly Ser Ile Gly Asp Ser Phe Ser Val Ala Ser Ile Lys Ala Tyr
1               5                   10                  15

Val Ala Glu Phe Ile Ala Thr Leu Leu Phe Val Phe Ala Gly Val Gly
            20                  25                  30

Ser Ala Ile Ala Tyr Asn Lys Leu Thr Ser Asp Ala Ala Leu Asp Pro
        35                  40                  45

Ala Gly Leu Val Ala Val Ala Val Ala His Ala Phe Ala Leu Phe Val
    50                  55                  60

Gly Val Ser Met Ala Ala Asn Val Ser Gly Gly His Leu Asn Pro Ala
65                  70                  75                  80

Val Thr Leu Gly Leu Ala Val Gly Gly Asn Ile Thr Ile Leu Thr Gly
                85                  90                  95

Leu Phe Tyr Trp Ile Ala Gln Cys Leu Gly Ser Thr Val Ala Cys Leu
            100                 105                 110

Leu Leu Lys Phe Val Thr Asn Gly Leu Ser Val Pro Thr His Gly Val
        115                 120                 125

Ala Ala Gly Met Asp Ala Ile Gln Gly Val Val Met Glu Ile Ile Ile
    130                 135                 140

Thr Phe Ala Leu Val Tyr Thr Val Tyr Ala Thr Ala Ala Asp Pro Lys
145                 150                 155                 160

Lys Gly Ser Leu Gly Val Ile Ala Pro Ile Ala Ile Gly Phe Ile Val
                165                 170                 175

Gly Ala Asn Ile Leu Ala Ala Gly Pro Phe Ser Gly Gly Ser Met Asn
            180                 185                 190

Pro Ala Arg Ser Phe Gly Pro Ala Val Ala Ser Gly Asp Phe Ser Gln
        195                 200                 205

Asn Trp Ile Tyr Trp Ala Gly Pro Leu Ile Gly Gly Ala Leu Ala Gly
    210                 215                 220

Phe Ile Tyr Gly
225

<210>  76
<211>  248
<212>  PRT
<213>  Zea mays

<400>  76

Met Val Lys Leu Ala Phe Gly Ser Phe Arg Asp Ser Leu Ser Ala Ala
1               5                   10                  15

Ser Leu Lys Ala Tyr Val Ala Glu Phe Ile Ala Thr Leu Leu Phe Val
            20                  25                  30

Phe Ala Gly Val Gly Ser Ala Ile Ala Tyr Ser Gln Leu Thr Lys Gly
        35                  40                  45

Gly Ala Leu Asp Pro Ala Gly Leu Val Ala Ile Ala Ile Ala His Ala
    50                  55                  60

Phe Ala Leu Phe Val Gly Val Ser Met Ala Ala Asn Ile Ser Gly Gly
65                  70                  75                  80

His Leu Asn Pro Ala Val Thr Phe Gly Leu Ala Val Gly Gly His Ile
                85                  90                  95

Thr Ile Leu Thr Gly Ile Leu Tyr Trp Val Ala Gln Leu Leu Gly Ala
            100                 105                 110

Ser Val Ala Cys Phe Leu Leu Gln Tyr Val Thr His Gly Gln Ala Ile
        115                 120                 125

Pro Thr His Gly Val Ser Gly Ile Ser Glu Ile Glu Gly Val Val Met
    130                 135                 140

Glu Ile Val Ile Thr Phe Ala Leu Val Tyr Thr Val Tyr Ala Thr Ala
145                 150                 155                 160

Ala Asp Pro Lys Lys Gly Ser Leu Gly Thr Ile Ala Pro Met Ala Ile
                165                 170                 175

Gly Phe Ile Val Gly Ala Asn Ile Leu Ala Ala Gly Pro Phe Ser Gly
            180                 185                 190

Gly Ser Met Asn Pro Ala Arg Ser Phe Gly Pro Ala Val Ala Ala Gly
        195                 200                 205

Asn Phe Ala Gly Asn Trp Val Tyr Trp Val Gly Pro Leu Val Gly Gly
    210                 215                 220

Gly Leu Ala Gly Pro Val Tyr Gly Asp Val Phe Ile Ala Ser Tyr Gln
225                 230                 235                 240

Pro Val Gly Gln Gln Glu Tyr Pro
                245

<210>  77
<211>  248
<212>  PRT
<213>  Helianthus annuus

<400>  77

Met Pro Gly Ile Ala Phe Gly Ser Phe Asp Asp Ser Phe Ser Ser Ser
1               5                   10                  15

Ser Ile Lys Ala Tyr Ile Ala Glu Phe Ile Ser Thr Leu Leu Phe Val
            20                  25                  30

Phe Ala Gly Val Gly Ser Ala Ile Ala Tyr Ala Lys Leu Thr Ala Asp
        35                  40                  45

Ala Ala Leu Asp Pro Pro Gly Leu Val Ala Val Ala Val Cys His Gly
    50                  55                  60

Phe Ala Leu Phe Val Ala Val Cys Ile Ala Ala Asn Ile Cys Gly Gly
65                  70                  75                  80

His Val Asn Pro Ala Val Thr Leu Gly Leu Ala Val Gly Gly Gln Ile
                85                  90                  95

Thr Phe Leu Thr Gly Leu Phe Tyr Trp Ile Ala Gln Leu Leu Gly Ser
            100                 105                 110

Thr Val Ala Cys Phe Leu Leu Ser Phe Val Thr Gly Gly Leu Ala Val
        115                 120                 125

Pro Thr His Gly Val Ala Glu Gly Val Gly Thr Ile Gln Gly Val Val
    130                 135                 140

Phe Glu Ile Ile Ile Thr Phe Ala Met Val Tyr Thr Val Tyr Ala Thr
145                 150                 155                 160

Ala Cys Asp Pro Lys Lys Gly Ala Leu Gly Thr Ile Ala Pro Ile Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Ala Val Ala Ser
        195                 200                 205

Phe Asp Phe Ser Gly His Trp Ile Tyr Trp Val Gly Pro Leu Val Gly
    210                 215                 220

Gly Gly Leu Ala Gly Leu Ile Tyr Pro Asn Val Phe Ile Ser Asn Glu
225                 230                 235                 240

His Ile Pro Leu Thr Asn Glu Tyr
                245

<210>  78
<211>  248
<212>  PRT
<213>  Helianthus annuus

<400>  78

Met Pro Gly Ile Ala Phe Gly Ser Phe Asp Asp Ser Phe Ser Ser Ser
1               5                   10                  15

Ser Ile Lys Ala Tyr Ile Ala Glu Phe Ile Ser Thr Leu Leu Phe Val
            20                  25                  30

Phe Ala Gly Val Gly Ser Ala Ile Ala Tyr Ala Lys Leu Thr Ala Asp
        35                  40                  45

Ala Ala Leu Asp Pro Ser Gly Leu Val Ala Ile Ala Val Cys His Gly
    50                  55                  60

Leu Ala Leu Phe Val Ala Val Ser Ile Ala Ala Asn Ile Ser Gly Gly
65                  70                  75                  80

His Val Asn Pro Ala Val Thr Phe Gly Leu Ala Val Gly Gly Gln Ile
                85                  90                  95

Thr Ile Leu Thr Gly Leu Phe Tyr Trp Ile Ala Gln Leu Leu Gly Ser
            100                 105                 110

Val Ala Ala Cys Phe Leu Leu Ser Phe Val Thr Gly Gly Leu Ala Val
        115                 120                 125

Pro Thr His Gly Val Ala Ala Gly Val Gly Val Leu Gln Gly Val Val
    130                 135                 140

Phe Glu Ile Ile Ile Thr Phe Ala Leu Val Tyr Thr Val Tyr Ala Thr
145                 150                 155                 160

Ala Cys Asp Pro Lys Lys Gly Ala Leu Gly Thr Ile Ser Pro Ile Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Thr Val Ala Ala
        195                 200                 205

Phe Asp Phe Ser Gly His Trp Phe Tyr Trp Val Gly Pro Leu Ile Gly
    210                 215                 220

Gly Gly Leu Ala Gly Ala Ile Tyr Pro Asn Val Phe Ile Ser Asn Glu
225                 230                 235                 240

His Ile Pro Leu Thr Asn Asp Tyr
                245

<210>  79
<211>  248
<212>  PRT
<213>  Vernicia fordii

<400>  79

Met Ala Arg Ile Ala Phe Gly Arg Phe Asp Asp Ser Phe Ser Leu Gly
1               5                   10                  15

Ser Phe Lys Ala Tyr Leu Ala Glu Phe Ile Ser Thr Leu Leu Tyr Val
            20                  25                  30

Phe Ala Gly Val Gly Ser Ala Ile Ala Tyr Asn Lys Leu Thr Ala Asn
        35                  40                  45

Ala Ala Leu Asp Pro Ala Gly Leu Val Ala Ile Ala Ile Cys His Gly
    50                  55                  60

Phe Ala Leu Phe Val Ala Val Ala Val Gly Ala Asn Ile Ser Gly Gly
65                  70                  75                  80

His Val Asn Pro Ala Val Ala Phe Gly Leu Ala Leu Gly Gly Gln Ile
                85                  90                  95

Thr Ile Leu Thr Gly Ile Phe Tyr Trp Ile Ala Gln Leu Leu Gly Ser
            100                 105                 110

Ile Val Ala Cys Phe Leu Leu Lys Leu Val Thr Gly Gly Leu Ala Ile
        115                 120                 125

Pro Thr His Ser Val Ala Gly Arg Val Gly Ala Ile Glu Gly Val Val
    130                 135                 140

Met Glu Ile Ile Ile Thr Phe Ala Leu Val Tyr Thr Val Tyr Ala Thr
145                 150                 155                 160

Ala Ala Asp Pro Lys Lys Gly Ser Leu Gly Thr Ile Ala Pro Ile Ala
                165                 170                 175

Ile Gly Phe Ile Val Gly Ala Asn Ile Leu Ala Ala Gly Pro Phe Ser
            180                 185                 190

Gly Gly Ser Met Tyr Pro Ala Arg Ser Phe Gly Pro Ala Val Val Ser
        195                 200                 205

Gly Asp Phe His Asp Asn Trp Ile Tyr Trp Val Gly Pro Leu Ile Gly
    210                 215                 220

Gly Gly Leu Ala Gly Leu Ile Tyr Gly Asn Leu Tyr Ile Ser Gly Asp
225                 230                 235                 240

His Thr Pro Leu Ser Asn Asp Phe
                245

<210>  80
<211>  248
<212>  PRT
<213>  Daucus carota

<400>  80

Met Val Lys Leu Ala Ile Gly Ser Val Gly Asp Ser Phe Ser Ala Val
1               5                   10                  15

Ser Leu Lys Ser Tyr Leu Ala Glu Phe Ile Ala Thr Leu Leu Phe Val
            20                  25                  30

Phe Ala Gly Val Gly Ser Ala Ile Ala Tyr Gly His Leu Thr Ala Asp
        35                  40                  45

Ala Ala Leu Asp Pro Ala Gly Leu Val Ala Ile Ala Ile Ala His Ala
    50                  55                  60

Phe Ala Leu Phe Val Gly Val Ser Met Ala Ala Asn Ile Ser Gly Gly
65                  70                  75                  80

His Leu Asn Pro Ala Val Thr Leu Gly Leu Ala Ile Gly Gly Asn Ile
                85                  90                  95

Thr Ile Ile Thr Gly Leu Phe Tyr Trp Ile Ala Gln Cys Leu Gly Ser
            100                 105                 110

Thr Val Ala Cys Phe Leu Leu Lys Phe Val Thr Ala Gly Lys Ala Ile
        115                 120                 125

Pro Thr His Gly Val Gly Ala Gly Leu Gly Ala Ala Glu Gly Val Val
    130                 135                 140

Phe Glu Ile Val Ile Thr Phe Ala Leu Val Tyr Thr Val Tyr Ala Thr
145                 150                 155                 160

Ala Ala Asp Pro Lys Lys Gly Ser Leu Gly Thr Ile Ala Pro Ile Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Ala Val Ala Ser
        195                 200                 205

Phe Asp Phe Ser Gly His Trp Ile Tyr Trp Val Gly Pro Leu Ile Gly
    210                 215                 220

Gly Gly Leu Ala Gly Leu Val Tyr Gly Asp Val Phe Ile Gly Ser Tyr
225                 230                 235                 240

Ala Pro Ile Ala Glu Asp Tyr Ala
                245

<210>  81
<211>  250
<212>  PRT
<213>  Antirrhinum majus

<400>  81

Met Val Lys Ile Ala Phe Gly Ser Ile Gly Asp Ser Phe Ser Val Ala
1               5                   10                  15

Ser Ile Lys Ala Tyr Val Ala Glu Phe Ile Ala Thr Leu Leu Phe Val
            20                  25                  30

Phe Ala Gly Val Gly Ser Ala Ile Ala Tyr Asn Lys Leu Thr Ser Asp
        35                  40                  45

Ala Ala Leu Asp Pro Ala Gly Leu Val Ala Val Ala Val Ala His Ala
    50                  55                  60

Phe Ala Leu Phe Val Gly Val Ser Met Ala Ala Asn Val Ser Gly Gly
65                  70                  75                  80

His Leu Asn Pro Ala Val Thr Leu Gly Leu Ala Val Gly Gly Asn Ile
                85                  90                  95

Thr Ile Leu Thr Gly Leu Phe Tyr Trp Ile Ala Gln Cys Leu Gly Ser
            100                 105                 110

Thr Val Ala Cys Leu Leu Leu Lys Phe Val Thr Asn Gly Leu Ser Val
        115                 120                 125

Pro Thr His Gly Val Ala Ala Gly Met Asp Ala Ile Gln Gly Val Val
    130                 135                 140

Met Glu Ile Ile Ile Thr Phe Ala Leu Val Tyr Thr Val Tyr Ala Thr
145                 150                 155                 160

Ala Ala Asp Pro Lys Lys Gly Ser Leu Gly Val Ile Ala Pro Ile Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Ala Val Ala Ser
        195                 200                 205

Gly Asp Phe Ser Gln Asn Trp Ile Tyr Trp Ala Gly Pro Leu Ile Gly
    210                 215                 220

Gly Ala Leu Ala Gly Phe Ile Tyr Gly Asp Val Phe Ile Thr Ala His
225                 230                 235                 240

Ala Pro Leu Pro Thr Ser Glu Asp Tyr Ala
                245                 250

<210>  82
<211>  250
<212>  PRT
<213>  Nicotiana tabacum

<400>  82

Met Val Arg Ile Ala Phe Gly Ser Ile Gly Asp Ser Phe Ser Val Gly
1               5                   10                  15

Ser Leu Lys Ala Tyr Val Ala Glu Phe Ile Ala Thr Leu Leu Phe Val
            20                  25                  30

Phe Ala Gly Val Gly Ser Ala Ile Ala Tyr Asn Lys Leu Thr Ala Asp
        35                  40                  45

Ala Ala Leu Asp Pro Ala Gly Leu Val Ala Val Ala Val Ala His Ala
    50                  55                  60

Phe Ala Leu Phe Val Gly Val Ser Ile Ala Ala Asn Ile Ser Gly Gly
65                  70                  75                  80

His Leu Asn Pro Ala Val Thr Leu Gly Leu Ala Val Gly Gly Asn Ile
                85                  90                  95

Thr Ile Leu Thr Gly Phe Phe Tyr Trp Ile Ala Gln Leu Leu Gly Ser
            100                 105                 110

Thr Val Ala Cys Leu Leu Leu Lys Tyr Val Thr Asn Gly Leu Ala Val
        115                 120                 125

Pro Thr His Gly Val Ala Ala Gly Leu Asn Gly Leu Gln Gly Val Val
    130                 135                 140

Met Glu Ile Ile Ile Thr Phe Ala Leu Val Tyr Thr Val Tyr Ala Thr
145                 150                 155                 160

Ala Ala Asp Pro Lys Lys Gly Ser Leu Gly Thr Ile Ala Pro Ile Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Ala Val Val Ala
        195                 200                 205

Gly Asp Phe Ser Gln Asn Trp Ile Tyr Trp Ala Gly Pro Leu Ile Gly
    210                 215                 220

Gly Gly Leu Ala Gly Phe Ile Tyr Gly Asp Val Phe Ile Gly Cys His
225                 230                 235                 240

Thr Pro Leu Pro Thr Ser Glu Asp Tyr Ala
                245                 250

<210> 83
<211> 250
<212> PRT
<213> Helianthus annuus

<400> 83

Met Val Lys Leu Ala Ile Gly Ser Ile Gly Asp Ser Leu Ser Ala Gly
1               5                   10                  15

Ser Ile Lys Ser Tyr Leu Ala Glu Phe Ile Ala Thr Leu Leu Phe Val
            20                  25                  30

Phe Ala Gly Val Gly Ser Ala Ile Ala Tyr Gly Lys Leu Thr Thr Asp
        35                  40                  45

Ala Ala Leu Asp Pro Ala Gly Leu Val Ala Ile Ala Ile Ala His Ala
    50                  55                  60

Phe Ala Leu Phe Val Gly Val Ser Met Ala Ala Asn Ile Ser Gly Gly
65                  70                  75                  80

His Leu Asn Pro Ala Val Thr Phe Gly Leu Ala Ile Gly Gly Asn Ile
                85                  90                  95

Thr Ile Ile Thr Gly Leu Phe Tyr Trp Ile Ala Gln Cys Leu Gly Ser
            100                 105                 110

Ile Val Ala Cys Phe Leu Leu Gln Phe Val Thr Gly Gly Leu Ala Val
        115                 120                 125

Pro Thr His Gly Val Ala Asp Gly Met Asn Gly Val Gln Gly Val Val
    130                 135                 140

Met Glu Ile Ile Ile Thr Phe Ala Leu Val Tyr Thr Val Tyr Ala Thr
145                 150                 155                 160

Ala Val Asp Pro Lys Lys Gly Ser Leu Gly Thr Ile Ala Pro Met Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Ala Val Val Ser
        195                 200                 205

Gly Asp Phe Ser Gln Asn Trp Ile Tyr Trp Val Gly Pro Leu Ile Gly
    210                 215                 220

Gly Gly Leu Ala Gly Phe Ile Phe Gly Asp Val Phe Ile Gly Ser Tyr
225                 230                 235                 240

Glu Thr Leu Pro Asp Ser Gly Asp Tyr Ala
                245                 250

<210>  84
<211>  250
<212>  PRT
<213>  Solanum tuberosum

<400>  84

Met Val Arg Ile Ala Phe Gly Ser Ile Gly Asp Ser Leu Ser Val Gly
1               5                   10                  15

Ser Leu Lys Ala Tyr Leu Ala Glu Phe Ile Ala Thr Leu Leu Phe Val
            20                  25                  30

Phe Ala Gly Val Gly Ser Ala Ile Ala Tyr Asn Lys Leu Thr Ser Asp
        35                  40                  45

Ala Ala Leu Asp Pro Ala Gly Leu Val Ala Ile Ala Val Ala His Ala
    50                  55                  60

Phe Ala Leu Phe Val Gly Val Ser Met Ala Ala Asn Ile Ser Gly Gly
65                  70                  75                  80

His Leu Asn Pro Ala Val Thr Leu Gly Leu Ala Val Gly Gly Asn Ile
                85                  90                  95

Thr Thr Leu Thr Gly Leu Phe Tyr Trp Val Ala Gln Leu Leu Gly Ser
            100                 105                 110

Thr Val Ala Cys Leu Leu Leu Lys Tyr Val Thr Asn Gly Leu Ala Val
        115                 120                 125

Pro Thr His Gly Val Ala Ala Gly Met Asn Gly Ala Glu Gly Val Val
    130                 135                 140

Met Glu Ile Val Ile Thr Phe Ala Leu Val Tyr Thr Val Tyr Ala Thr
145                 150                 155                 160

Ala Ala Asp Pro Lys Lys Gly Ser Leu Gly Thr Ile Ala Pro Ile Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Ala Val Val Ala
        195                 200                 205

Gly Asp Phe Ser Gln Asn Trp Ile Tyr Trp Val Gly Pro Leu Ile Gly
    210                 215                 220

Gly Gly Leu Ala Gly Phe Ile Tyr Gly Asp Val Phe Ile Gly Ser His
225                 230                 235                 240

Thr Pro Leu Pro Thr Ser Glu Asp Tyr Ala
                245                 250

<210>  85
<211>  250
<212>  PRT
<213>  Nicotiana tabacum

<400>  85

Met Val Leu Ile Ala Phe Gly Ser Ile Gly Asp Ser Phe Ser Val Gly
1               5                   10                  15

Ser Leu Lys Ala Tyr Val Ala Glu Phe Ile Ala Thr Leu Leu Phe Val
            20                  25                  30

Phe Ala Gly Val Gly Ser Ala Ile Ala Tyr Asn Lys Leu Thr Ala Asp
        35                  40                  45

Ala Ala Leu Asp Pro Ala Gly Leu Val Ala Val Ala Val Ala His Ala
    50                  55                  60

Phe Ala Leu Phe Val Gly Val Ser Ile Ala Ala Asn Ile Ser Gly Gly
65                  70                  75                  80

His Leu Asn Pro Ala Val Thr Leu Gly Leu Ala Val Gly Gly Asn Ile
                85                  90                  95

Thr Ile Leu Thr Gly Phe Phe Tyr Trp Ile Ala Gln Leu Leu Gly Ser
            100                 105                 110

Thr Val Ala Cys Leu Leu Leu Lys Tyr Val Thr Asn Gly Leu Ala Val
        115                 120                 125

Pro Thr His Gly Val Ala Ala Gly Leu Asn Gly Phe Gln Gly Val Val
    130                 135                 140

Met Glu Ile Ile Ile Thr Phe Ala Leu Val Tyr Thr Val Tyr Ala Thr
145                 150                 155                 160

Ala Ala Asp Pro Lys Lys Gly Ser Leu Gly Thr Ile Ala Pro Ile Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Ala Val Val Ala
        195                 200                 205

Gly Asp Phe Ser Gln Asn Trp Ile Tyr Trp Ala Gly Pro Leu Ile Gly
    210                 215                 220

Gly Gly Leu Ala Gly Phe Ile Tyr Gly Asp Val Phe Ile Gly Cys His
225                 230                 235                 240

Thr Pro Leu Pro Thr Ser Glu Asp Tyr Ala
                245                 250

<210>  86
<211>  250
<212>  PRT
<213>  Lycopersicon esculentum

<400>  86

Met Val Arg Ile Ala Phe Gly Ser Ile Gly Asp Ser Leu Ser Val Gly
1               5                   10                  15

Ser Leu Lys Ala Tyr Leu Ala Glu Phe Ile Ala Thr Leu Leu Phe Val
            20                  25                  30

Phe Ala Gly Val Gly Ser Ala Ile Ala Phe Asn Lys Leu Thr Ser Gly
        35                  40                  45

Ala Ala Leu Asp Pro Ala Gly Leu Val Ala Ile Ala Val Ala His Ala
    50                  55                  60

Phe Ala Leu Phe Val Gly Val Ser Met Ala Ala Asn Ile Ser Gly Gly
65                  70                  75                  80

His Leu Asn Pro Ala Val Thr Leu Gly Leu Ala Val Gly Gly Asn Ile
                85                  90                  95

Thr Ile Leu Thr Gly Leu Phe Tyr Trp Val Ala Gln Leu Leu Gly Ser
            100                 105                 110

Thr Val Ala Cys Leu Leu Leu Lys Tyr Val Thr Asn Gly Leu Ala Val
        115                 120                 125

Pro Thr His Gly Val Ala Ala Gly Met Ser Gly Ala Glu Gly Val Val
    130                 135                 140

Met Glu Ile Val Ile Thr Phe Ala Leu Val Tyr Thr Val Tyr Ala Thr
145                 150                 155                 160

Ala Ala Asp Pro Lys Lys Gly Ser Leu Gly Thr Ile Ala Pro Ile Ala
                165                 170                 175

Ile Gly Phe Ile Val Gly Ala Asn Ile Leu Ala Ala Gly Pro Phe Ser
            180                 185                 190

Gly Gly Ser Met Asn Pro Ala Arg Ser Phe Gly Pro Ala Val Val Ala
        195                 200                 205

Gly Asp Phe Ser Gln Asn Trp Ile Tyr Trp Val Gly Pro Leu Ile Gly
    210                 215                 220

Gly Gly Leu Ala Gly Phe Ile Tyr Gly Asp Val Phe Ile Gly Cys His
225                 230                 235                 240

Thr Pro Leu Pro Thr Ser Glu Asp Tyr Ala
                245                 250

<210>  87
<211>  234
<212>  PRT
<213>  Arabidopsis thaliana

<400>  87

Met Val Ser Pro Glu Asn Ala Asn Trp Ile Cys Asp Leu Ile Asp Ala
1               5                   10                  15

Asp Tyr Gly Ser Phe Thr Ile Gln Gly Pro Gly Phe Ser Trp Pro Val
            20                  25                  30

Gln Gln Pro Ile Gly Val Ser Ser Asn Ser Ser Ala Gly Val Asp Gly
        35                  40                  45

Ser Ala Gly Asn Ser Glu Ala Ser Lys Glu Pro Gly Ser Lys Lys Arg
    50                  55                  60

Gly Arg Cys Glu Ser Ser Ser Ala Thr Ser Ser Lys Ala Cys Arg Glu
65                  70                  75                  80

Lys Gln Arg Arg Asp Arg Leu Asn Asp Lys Phe Met Glu Leu Gly Ala
                85                  90                  95

Ile Leu Glu Pro Gly Asn Pro Pro Lys Thr Asp Lys Ala Ala Ile Leu
            100                 105                 110

Val Asp Ala Val Arg Met Val Thr Gln Leu Arg Gly Glu Ala Gln Lys
        115                 120                 125

Leu Lys Asp Ser Asn Ser Ser Leu Gln Asp Lys Ile Lys Glu Leu Lys
    130                 135                 140

Thr Glu Lys Asn Glu Leu Arg Asp Glu Lys Gln Arg Leu Lys Thr Glu
145                 150                 155                 160

Lys Glu Lys Leu Glu Gln Gln Leu Lys Ala Met Asn Ala Pro Gln Pro
                165                 170                 175

Ser Phe Phe Pro Ala Pro Pro Met Met Pro Thr Ala Phe Ala Ser Ala
            180                 185                 190

Gln Gly Gln Ala Pro Gly Asn Lys Met Val Pro Ile Ile Ser Tyr Pro
        195                 200                 205

Gly Val Ala Met Trp Gln Phe Met Pro Pro Ala Ser Val Asp Thr Ser
    210                 215                 220

Gln Asp His Val Leu Arg Pro Pro Val Ala
225                 230

<210>  88
<211>  234
<212>  PRT
<213>  Arabidopsis thaliana

<400>  88

Met Val Ser Pro Glu Asn Ala Asn Trp Ile Cys Asp Leu Ile Asp Ala
1               5                   10                  15

Asp Tyr Gly Ser Phe Thr Ile Gln Gly Pro Gly Phe Ser Trp Pro Val
            20                  25                  30

Gln Gln Pro Ile Gly Val Ser Ser Asn Ser Ser Ala Gly Val Asp Gly
        35                  40                  45

Ser Ala Gly Asn Ser Glu Ala Ser Lys Glu Pro Gly Ser Lys Lys Arg
    50                  55                  60

Gly Arg Cys Glu Ser Ser Ser Ala Thr Ser Ser Lys Ala Cys Arg Glu
65                  70                  75                  80

Lys Gln Arg Arg Asp Arg Leu Asn Asp Lys Phe Met Glu Leu Gly Ala
                85                  90                  95

Ile Leu Glu Pro Gly Asn Pro Pro Lys Thr Asp Lys Ala Ala Ile Leu
            100                 105                 110

Val Asp Ala Val Arg Met Val Thr Gln Leu Arg Gly Glu Ala Gln Lys
        115                 120                 125

Leu Lys Asp Ser Asn Ser Ser Leu Gln Asp Lys Ile Lys Glu Leu Lys
    130                 135                 140

Thr Glu Lys Asn Glu Leu Arg Asp Glu Lys Gln Arg Leu Lys Thr Glu
145                 150                 155                 160

Lys Glu Lys Leu Glu Gln Gln Leu Lys Ala Ile Asn Ala Pro Gln Pro
                165                 170                 175

Ser Phe Phe Pro Ala Pro Pro Met Met Pro Thr Ala Phe Ala Ser Ala
            180                 185                 190

Gln Gly Gln Ala Pro Gly Asn Lys Met Val Pro Ile Ile Ser Tyr Pro
        195                 200                 205

Gly Val Ala Met Trp Gln Phe Met Pro Pro Ala Ser Val Asp Thr Ser
    210                 215                 220

Gln Asp His Val Leu Arg Pro Pro Val Ala
225                 230

<210>  89
<211>  216
<212>  PRT
<213>  Oryza sativa

<400>  89

Cys Gly Ala Phe Pro Trp Asp Ala Ser Pro Ser Cys Ser Asn Pro Ser
1               5                   10                  15

Val Glu Val Ser Ser Tyr Val Asn Thr Thr Ser Tyr Val Leu Lys Glu
            20                  25                  30

Pro Gly Ser Asn Lys Arg Val Arg Ser Gly Ser Cys Gly Arg Pro Thr
        35                  40                  45

Ser Lys Ala Ser Arg Glu Lys Ile Arg Arg Asp Lys Met Asn Asp Arg
    50                  55                  60

Phe Leu Glu Leu Gly Thr Thr Leu Glu Pro Gly Lys Pro Val Lys Ser
65                  70                  75                  80

Asp Lys Ala Ala Ile Leu Ser Asp Ala Thr Arg Met Val Ile Gln Leu
                85                  90                  95

Arg Ala Glu Ala Lys Gln Leu Lys Asp Thr Asn Glu Ser Leu Glu Asp
            100                 105                 110

Lys Ile Lys Glu Leu Lys Ala Glu Lys Asp Glu Leu Arg Asp Glu Lys
        115                 120                 125

Gln Lys Leu Lys Val Glu Lys Glu Thr Leu Glu Gln Gln Val Lys Ile
    130                 135                 140

Leu Thr Ala Thr Pro Ala Tyr Met Pro His Pro Thr Leu Met Pro Ala
145                 150                 155                 160

Pro Tyr Pro Gln Ala Pro Leu Ala Pro Phe His His Ala Gln Gly Gln
                165                 170                 175

Ala Ala Gly Gln Lys Leu Met Met Pro Phe Val Gly Tyr Pro Gly Tyr
            180                 185                 190

Pro Met Trp Gln Phe Met Pro Pro Ser Glu Val Asp Thr Ser Lys Asp
        195                 200                 205

Ser Glu Ala Cys Pro Pro Val Ala
    210                 215

<210>  90
<211>  226
<212>  PRT
<213>  Arabidopsis thaliana

<400>  90

Met Val Ser Pro Glu Asn Thr Asn Trp Leu Ser Asp Tyr Pro Leu Ile
1               5                   10                  15

Glu Gly Ala Phe Ser Asp Gln Asn Pro Thr Phe Pro Trp Gln Ile Asp
            20                  25                  30

Gly Ser Ala Thr Val Ser Val Glu Val Asp Gly Phe Leu Cys Asp Ala
        35                  40                  45

Asp Val Ile Lys Glu Pro Ser Ser Arg Lys Arg Ile Lys Thr Glu Ser
    50                  55                  60

Cys Thr Gly Ser Asn Ser Lys Ala Cys Arg Glu Lys Gln Arg Arg Asp
65                  70                  75                  80

Arg Leu Asn Asp Lys Phe Thr Glu Leu Ser Ser Val Leu Glu Pro Gly
                85                  90                  95

Arg Thr Pro Lys Thr Asp Lys Val Ala Ile Ile Asn Asp Ala Ile Arg
            100                 105                 110

Met Val Asn Gln Ala Arg Asp Glu Ala Gln Lys Leu Lys Asp Leu Asn
        115                 120                 125

Ser Ser Leu Gln Glu Lys Ile Lys Glu Leu Lys Asp Glu Lys Asn Glu
    130                 135                 140

Leu Arg Asp Glu Lys Gln Lys Leu Lys Val Glu Lys Glu Arg Ile Asp
145                 150                 155                 160

Gln Gln Leu Lys Ala Ile Lys Thr Gln Pro Gln Pro Gln Pro Cys Phe
                165                 170                 175

Leu Pro Asn Pro Gln Thr Leu Ser Gln Ala Gln Ala Pro Gly Ser Lys
            180                 185                 190

Leu Val Pro Phe Thr Thr Tyr Pro Gly Phe Ala Met Trp Gln Phe Met
        195                 200                 205

Pro Pro Ala Ala Val Asp Thr Ser Gln Asp His Val Leu Arg Pro Pro
    210                 215                 220

Val Ala
225

<210>  91
<211>  256
<212>  PRT
<213>  Oryza sativa

<400>  91

Met Ala Ser Pro Glu Gly Ser Thr Trp Val Phe Asp Cys Pro Leu Met
1               5                   10                  15

Asp Asp Leu Ala Ala Ala Ala Gly Phe Asp Ala Ala Pro Ala Gly Gly
            20                  25                  30

Phe Tyr Trp Thr Thr Pro Ala Pro Pro Gln Ala Ala Leu Gln Pro Pro
        35                  40                  45

Pro Pro Gln Gln Gln Pro Val Ala Pro Ala Thr Ala Ala Pro Asn Ala
    50                  55                  60

Cys Ala Glu Ile Asn Gly Ser Val Asp Cys Glu His Gly Lys Glu Gln
65                  70                  75                  80

Pro Thr Asn Lys Arg Pro Arg Ser Glu Ser Gly Thr Arg Pro Ser Ser
                85                  90                  95

Lys Ala Cys Arg Glu Lys Val Arg Arg Asp Lys Leu Asn Glu Arg Phe
            100                 105                 110

Leu Glu Leu Gly Ala Val Leu Glu Pro Gly Lys Thr Pro Lys Met Asp
        115                 120                 125

Lys Ser Ser Ile Leu Asn Asp Ala Ile Arg Val Met Ala Glu Leu Arg
    130                 135                 140

Ser Glu Ala Gln Lys Leu Lys Glu Ser Asn Glu Ser Leu Gln Glu Lys
145                 150                 155                 160

Ile Lys Glu Leu Lys Ala Glu Lys Asn Glu Leu Arg Asp Glu Lys Gln
                165                 170                 175

Lys Leu Lys Ala Glu Lys Glu Ser Leu Glu Gln Gln Ile Lys Phe Leu
            180                 185                 190

Asn Ala Arg Pro Ser Phe Val Pro His Pro Pro Val Ile Pro Ala Ser
        195                 200                 205

Ala Phe Thr Ala Pro Gln Gly Gln Ala Ala Gly Gln Lys Leu Met Met
    210                 215                 220

Pro Val Ile Gly Tyr Pro Gly Phe Pro Met Trp Gln Phe Met Pro Pro
225                 230                 235                 240

Ser Asp Val Asp Thr Thr Asp Asp Thr Lys Ser Cys Pro Pro Val Ala
                245                 250                 255

<210>  92
<211>  253
<212>  PRT
<213>  Oryza sativa

<400>  92

Met Ser Gly Thr Pro Ala Asp Gly Gly Gly Gly Gly Gly Gly Gly Gly
1               5                   10                  15

Gly Gly Ser Gly Asp Asp Trp Phe Leu Asp Cys Gly Ile Leu Glu Asp
            20                  25                  30

Leu Pro Ala Ala Ala Cys Gly Ala Phe Pro Trp Asp Ala Ser Pro Ser
        35                  40                  45

Cys Ser Asn Pro Ser Val Glu Val Ser Ser Tyr Val Asn Thr Thr Ser
    50                  55                  60

Tyr Val Leu Lys Glu Pro Gly Ser Asn Lys Arg Val Arg Ser Gly Ser
65                  70                  75                  80

Cys Gly Arg Pro Thr Ser Lys Ala Ser Arg Glu Lys Ile Arg Arg Asp
                85                  90                  95

Lys Met Asn Asp Arg Phe Leu Glu Leu Gly Thr Thr Leu Glu Pro Gly
            100                 105                 110

Lys Pro Val Lys Ser Asp Lys Ala Ala Ile Leu Ser Asp Ala Thr Arg
        115                 120                 125

Met Val Ile Gln Leu Arg Ala Glu Ala Lys Gln Leu Lys Asp Thr Asn
    130                 135                 140

Glu Ser Leu Glu Asp Lys Ile Lys Glu Leu Lys Ala Glu Lys Asp Glu
145                 150                 155                 160

Leu Arg Asp Glu Lys Gln Lys Leu Lys Val Glu Lys Glu Thr Leu Glu
                165                 170                 175

Gln Gln Val Lys Ile Leu Thr Ala Thr Pro Ala Tyr Met Pro His Pro
            180                 185                 190

Thr Leu Met Pro Ala Pro Tyr Pro Gln Ala Pro Leu Ala Pro Phe His
        195                 200                 205

His Ala Gln Gly Gln Ala Ala Gly Gln Lys Leu Met Met Pro Phe Val
    210                 215                 220

Gly Tyr Pro Gly Tyr Pro Met Trp Gln Phe Met Pro Pro Ser Glu Val
225                 230                 235                 240

Asp Thr Ser Lys Asp Ser Glu Ala Cys Pro Pro Val Ala
                245                 250